(19) Europäisches Patentamt
European Patent Office
Office européen des brevets

(11) EP 2 455 380 A1

(12) **EUROPEAN PATENT APPLICATION**
published in accordance with Art. 153(4) EPC

(43) Date of publication:
**23.05.2012 Bulletin 2012/21**

(21) Application number: **10799814.8**

(22) Date of filing: **12.07.2010**

(51) Int Cl.:
***C07D 471/04*** *(2006.01)*
***A61K 31/437*** *(2006.01)*
***A61K 31/55*** *(2006.01)*
***A61P 25/16*** *(2006.01)*
***A61P 25/28*** *(2006.01)*
***A61P 43/00*** *(2006.01)*
***C07D 487/04*** *(2006.01)*

(86) International application number:
**PCT/JP2010/061774**

(87) International publication number:
**WO 2011/007756 (20.01.2011 Gazette 2011/03)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **13.07.2009 JP 2009165134**

(71) Applicant: **Takeda Pharmaceutical Company Limited**
**Osaka-shi, Osaka 541-0045 (JP)**

(72) Inventors:
- **KOIKE, Tatsuki**
**Osaka-shi**
**Osaka 532-8686 (JP)**
- **HOASHI, Yasutaka**
**Osaka-shi**
**Osaka 532-8686 (JP)**
- **TOMATA, Yoshihide**
**Osaka-shi**
**Osaka 532-8686 (JP)**

(74) Representative: **Jones, Nicholas Andrew et al**
**Withers & Rogers LLP**
**4 More London Riverside**
**GB-London SE1 2AU (GB)**

(54) **HETEROCYCLIC COMPOUND AND USE THEREOF**

(57) The present invention provides a heterocycle derivative having a superior amyloid β production inhibitory activity and use thereof.

The present invention relates to a compound represented by the formula (I):

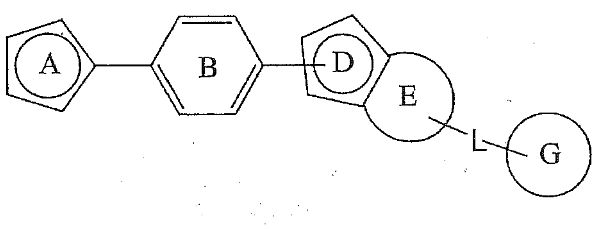

(I)

wherein each symbol is as defined in the present specification, or a salt thereof.

EP 2 455 380 A1

## Description

[Technical Field]

The present invention relates to a heterocyclic compound having a superior amyloid β production inhibitory activity, and useful as an agent for the prophylaxis or treatment of mild cognitive impairment, Alzheimer's disease and the like.

(Background of the Invention)

The major symptoms of dementia are Alzheimer's disease and mild cognitive impairment, and the patient number is drastically increasing with the advent of aging society. The sole therapeutic drugs therefor are symptomatic improvement drugs such as acetylcholinesterase inhibitors and the like, and the development of a drug capable of halting or delaying the progression of pathology, or a drug having a prophylactic effect has been desired.
The etiology of Alzheimer's disease is considered to be senile plaque formed by accumulation of a peptide consisting of about 40 amino acids, which is called amyloid β (hereinafter sometimes to be simply referred to as Aβ), or nerve cell death. Aβ is a peptide produced by processing a single transmembrane protein amyloid precursor (hereinafter sometimes to be simply referred to as APP), which is a precursor protein, with a degrading enzyme called secretase, and the main molecule species are Aβ40 consisting of 40 amino acids and Aβ42 consisting of 42 amino acids. Of these, Aβ42 aggregates easily and is considered to play a key role in senile plaque formation or nerve cell death (non-patent document 1). On the other hand, secretase, which is an excision enzyme, is known to include β-secretase for cutting out amino terminal and γ-secretase for cutting out carboxy terminal. γ-Secretase is constituted with presenilin (PS) and 3 kinds of cofactor proteins (nicastrin: NCT, APH-1, PEN-2) etc. (non-patent document 2). A radical treatment drug for Alzheimer's disease, which is based on inhibition of these secretases and suppression of production or secretion of Aβ, has been investigated (non-patent document 1). In the meantime, since γ-secretase is involved not only in the processing of APP but also functions such as activation of Notch receptor playing an important role in cell differentiation by intramembranous cleavage, and the like, the development of a drug capable of specifically inhibiting Aβ production alone without influencing other than APP processing has been desired (non-patent document 3).
Patent document 1 describes, as an amyloid β production inhibitor, a compound represented by the following formula:
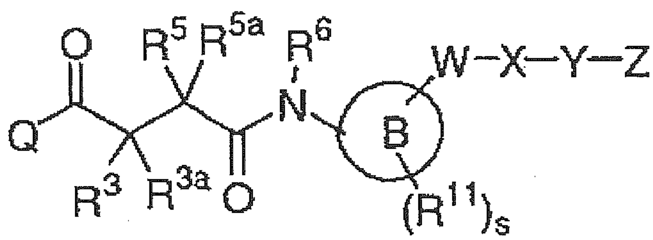

wherein each symbol is as defined in patent document 1.
Patent document 2 describes, as a compound having an amyloid β level regulating action (use: neurodegenerative disease), a compound represented by the following formula:
$$(A)\text{-}L_A\text{-}(B)\text{-}L_B\text{-}(C)\text{-}L_C\text{-}(D)$$

wherein each symbol is as defined in patent document 2.
Patent document 3 describes, as a cinnamide compound (use: neurodegenerative diseases caused by amyloid β, such as Alzheimer's disease, Down's disease and the like), a compound represented by the following formula:
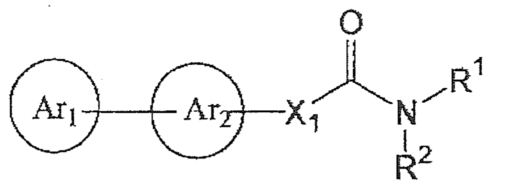

wherein each symbol is as defined in patent document 3.
Patent document 4 describes, as a polycyclic cinnamide compound (use: neurodegenerative diseases caused by amyloid β, such as Alzheimer's disease, Down's disease and the like), a compound represented by the following formula:
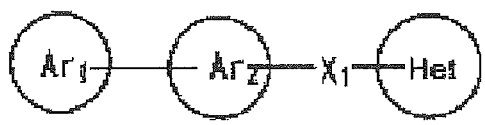

wherein each symbol is as defined in patent document 4.
Patent document 5 describes, as an imidazolyl-phenyl-vinyl-heterocycle derivative (use: Alzheimer's disease), a compound represented by the following formula:
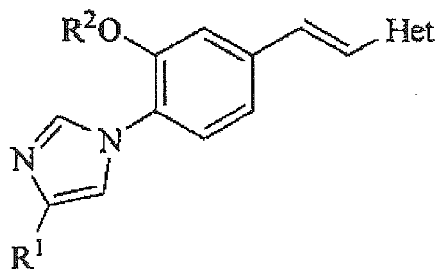

wherein each symbol is as defined in patent document 5.
Patent document 6 describes, as a γ-secretase modulator, a compound represented by the following formula:
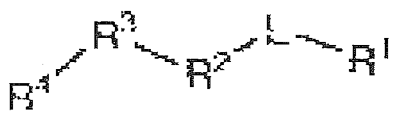

wherein each symbol is as defined in patent document 6.
Patent document 7 describes, as various heterocyclic compounds, a tachykinin receptor antagonist represented by the following formula:
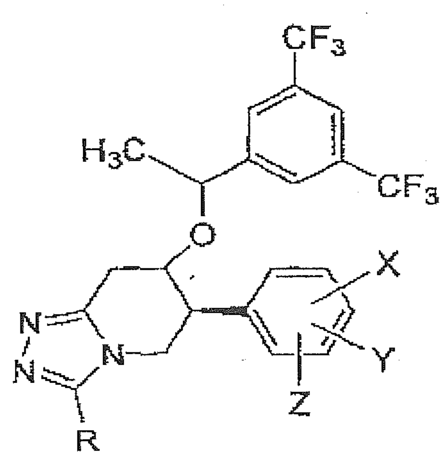

Ia

wherein each symbol is as defined in patent document 7.

Patent document 8 describes an mGluR5 regulator represented by the following formula:

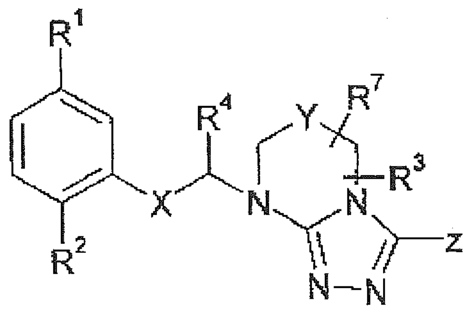

wherein each symbol is as defined in patent document 8.

Patent document 9 describes an 11β-hydroxysteroid dehydrogenase type 1 inhibitor represented by the following formula:

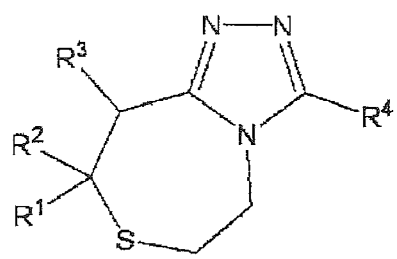

wherein each symbol is as defined in patent document 9.

[Document List]

[patent document]

patent document 1: WO2001/60826

patent document 2: W02004/110350
patent document 3: WO2005/115990
patent document 4: WO2007/102580
patent document 5: WO2008/097538
patent document 6: WO2009/073777
patent document 7: WO2007/081897
patent document 8: WO2007/130824
patent document 9: WO2008/078725

[non-patent document]

non-patent document 1: Annual Reports in Medicinal Chemistry, 2007, vol. 42, p.27-47
non-patent document 2: Naunyn-Schmiedeberg's Arch. Pharmacol., 2008, vol. 377, p.295-300
non-patent document 3: Neurotherapeutics (2008), vol. 5, p. 391-398
non-patent document 4: Expert Opinion on Therapeutic Patents, 2008, vol. 18, p.693-793

[Summary Of The Invention]

[Problems to be Solved by the Invention]

The development of a compound having a superior amyloid β production inhibitory activity, useful as an agent for the prophylaxis or treatment of mild cognitive impairment, Alzheimer's disease and the like, and having superior properties in terms of efficacy, low toxicity, stability, pharmacokinetics and the like has been desired.
The present invention aims to provide a heterocyclic compound having a chemical structure different from that of known compounds (including the aforementioned compounds) and having an amyloid β production inhibitory activity, and a prophylactic drug or a therapeutic drug for diseases such as mild cognitive impairment, Alzheimer's disease and the like, which contains the heterocyclic compound.

[Means of Solving the Problems]

The present inventors have conducted intensive studies in an attempt to solve the aforementioned problems and found that a compound represented by the following formula (I) or a salt thereof has a superior amyloid β production inhibitory activity and conducted further studies, which resulted in the completion of the present invention.
Accordingly, the present invention provides the following.

[1] A compound represented by the formula (I):

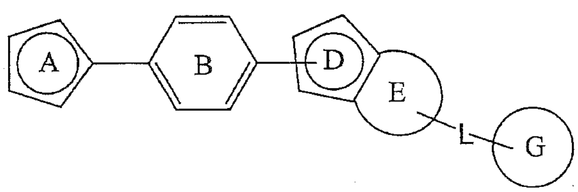

(I)

wherein

ring A is an imidazole ring, an oxazole ring or a triazole ring, each of which optionally has substituent(s),
ring B is a benzene ring, a pyridine ring or a pyrimidine ring, each of which optionally has substituent(s),
a group represented by a partial structural formula in the formula (I)

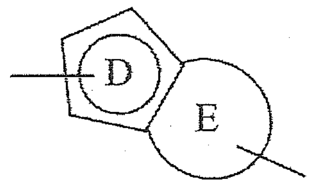

is a group represented by

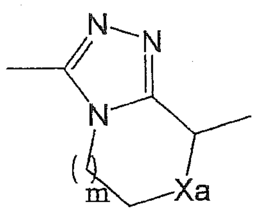

or

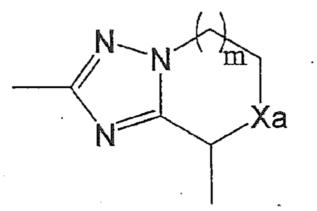

, each of which optionally has substituent(s),

> wherein Xa is $-CH_2-$, -NH-, -O-, -S-, -SO- or $-SO_2-$ and m is 0, 1 or 2,
> L is a bond, -O- or $-O-Y^1-$ wherein $Y^1$ is a $C_{1-6}$ alkylene group optionally having substituent(s), and
> ring G is an aromatic hydrocarbon ring or an aromatic heterocycle, each of which optionally has substituent(s),

or a salt thereof;

> [2] the compound of the above-mentioned [1], wherein ring G is a benzene ring or a benzimidazole ring, each of which optionally has substituent(s), or a salt thereof;
> [3] the compound of the above-mentioned [1], wherein ring A is an imidazole ring, an oxazole ring or a triazole ring, each of which is optionally substituted by 1 to 3 $C_{1-6}$ alkyl groups, ring B is a benzene ring or a pyridine ring, each of which is optionally substituted by 1 to 3 substituents selected from a halogen atom, a cyano group and a $C_{1-6}$ alkoxy group,
> Xa is $-CH_2-$,
> m is 1 or 2,
> L is a bond, -O- or $-O-Y^{1'}-$ wherein $Y^{1'}$ is a $C_{1-6}$ alkylene group, and
> ring G is a benzene ring or benzimidazole ring optionally substituted by 1 to 3 substituents selected from a halogen atom, a $C_{1-6}$ alkyl group optionally substituted by a halogen atom, a $C_{1-6}$ alkoxy group optionally substituted by a halogen atom, a $C_{2-6}$ alkenyl group, a $C_{1-6}$ alkylthio group, a $C_{1-6}$ alkylsulfonyl group and a morpholinyl group,
> or a salt thereof;
> [4] the compound of the above-mentioned [3], wherein ring A is an imidazole ring, an oxazole ring or a triazole ring, each of which is substituted by 1 to 3 $C_{1-6}$ alkyl groups,
> ring B is a benzene ring or a pyridine ring, each of which is substituted by 1 to 3 substituents selected from a halogen atom, a cyano group and a $C_{1-6}$ alkoxy group, and ring G is a benzene ring or a benzimidazole ring, each of which is optionally substituted by 1 to 3 substituents selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group,
> or a salt thereof;
> [5] the compound of the above-mentioned [4], wherein ring B is (1) a benzene ring substituted by 1 to 3 substituents selected from a halogen atom, a cyano group and a $C_{1-6}$ alkoxy group, or (2) a pyridine ring substituted by 1 to 3 $C_{1-6}$ alkoxy groups, a group represented by a partial structural formula in the formula (I)

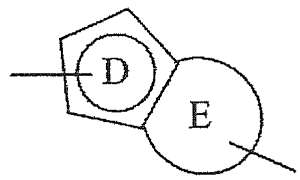

is a group represented by

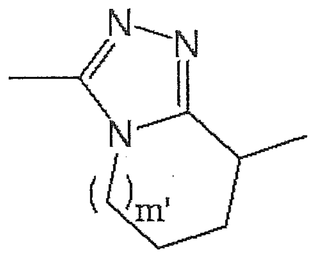

wherein m' is 1 or 2, or

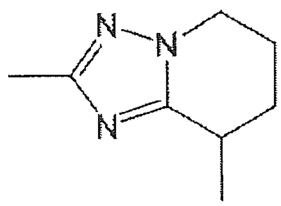

,

and

ring G is

(1)a benzene ring optionally substituted by 1 to 3 substituents selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group, or

(2) a benzimidazole ring substituted by 1 to 3 $C_{1-6}$ alkyl groups substituted by a halogen atom,

or a salt thereof;

[6] the compound of the above-mentioned [1], wherein ring A is an oxazole ring substituted by 1 or 2 $C_{1-6}$ alkyl groups,
ring B is a benzene ring substituted by 1 to 3 $C_{1-6}$ alkoxy groups,
Xa is $-CH_2-$,
m is 1 or 2,
L is a bond or -O-, and
ring G is a benzene ring substituted by 1 to 3 substituents selected from a halogen atom and a $C_{1-6}$ alkyl group substituted by a halogen atom,
or a salt thereof;
[7] the compound of the above-mentioned [6], wherein a group represented by a partial structural formula in the formula (I)

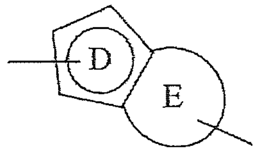

is a group represented by

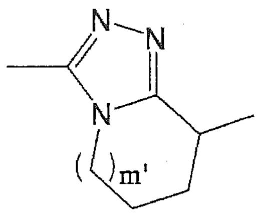

wherein m' is 1 or 2, or

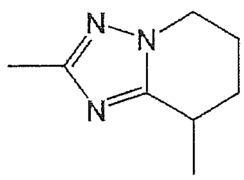

or a salt thereof;

[8] the compound of the above-mentioned [1], wherein ring A is an oxazole ring substituted by 1 or 2 $C_{1-6}$ alkyl groups, ring B is a benzene ring substituted by 1 to 3 $C_{1-6}$ alkoxy groups, a group represented by a partial structural formula in the formula (I)

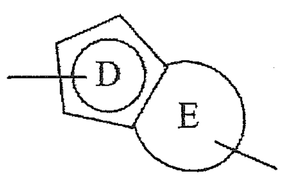

is a group represented by

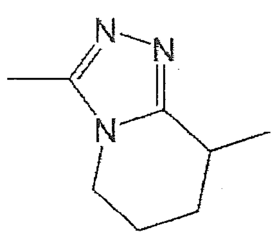

,

L is a bond, and
ring G is a benzene ring substituted by 1 to 3 substituents selected from a halogen atom and a $C_{1-6}$ alkyl group substituted by a halogen atom,

or a salt thereof;

[9] 8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine or a salt thereof;
[10] 8-[4-chloro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine or a salt thereof;
[11] a prodrug of the compound of the above-mentioned [1] or a salt thereof;
[12] a medicament containing the compound of the above-mentioned [1] or a salt thereof or a prodrug thereof;
[13] the medicament of the above-mentioned [12], which is a prophylactic or therapeutic drug for mild cognitive impairment or Alzheimer's disease;
[14] a method of inhibiting amyloid β production, comprising administering an effective amount of the compound of the above-mentioned [1] or a salt thereof or a prodrug thereof to a mammal;
[15] a method of preventing or treating mild cognitive impairment or Alzheimer's disease, comprising administering an effective amount of the compound of the above-mentioned [1] or a salt thereof or a prodrug thereof to a mammal;
[16] use of the compound of the above-mentioned [1] or a salt thereof or a prodrug thereof for the production of a drug for suppressing amyloid β production;
[17] use of the compound of the above-mentioned [1] or a salt thereof or a prodrug thereof for the production of a prophylactic or therapeutic drug for mild cognitive impairment or Alzheimer's disease;
[18] the compound of the above-mentioned [1] or a salt thereof or a prodrug thereof for the suppression of amyloid β production;
[19] the compound of the above-mentioned [1] or a salt thereof or a prodrug thereof for the prophylaxis or treatment of mild cognitive impairment or Alzheimer's disease;

and the like.

[Effect of the Invention]

Since a compound represented by the formula (I) (hereinafter sometimes to be referred to as compound (I)) or a salt thereof, or a prodrug thereof has a superior amyloid β production inhibitory activity, it is useful as a safe prophylactic or therapeutic drug for any diseases possibly relating to the abnormality of amyloid β production, such as mild cognitive impairment, Alzheimer's disease and the like.

(Detailed Description of the Invention)

The present invention is explained in detail in the following.
Examples of the "halogen atom" used in the present specification include fluorine, chlorine, bromine and iodine.
Examples of the "aromatic hydrocarbon ring" of the "aromatic hydrocarbon ring optionally having substituent (s)" used in the present specification include $C_6$-$_{14}$ aromatic hydrocarbon ring (preferably $C_6$-$_{12}$ aromatic hydrocarbon ring) such as benzene, naphthalene, anthracene, phenanthrene, acenaphthylene and the like and the like.
Examples of the "aromatic heterocycle" of the "aromatic heterocycle optionally having substituent(s)" used in the present specification include 5- or 6-membered monocyclic aromatic heterocycles such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine and the like; 8- to 16-membered (preferably, 8- to 12-membered) condensed aromatic heterocycles such as benzofuran, isobenzofuran, pyrazolothiophene, benzo[b]thiophene, benzo[c]thiophene, indole, isoindole, 1H-indazole, benzimidazole, benzoxazole, 1,2-benzisoxazole, benzothiazole, 1,2-benzisothiazole, 1H-benzotriazole, quinoline, isoquinoline, cinnoline, quinazoline, quinoxaline, phthalazine, naphthyridine, purine, pteridinee, carbazole, α-carboline, β-carboline, γ-carboline, acridine, phenoxathiine, phenothiazine, phenazine, phenoxathiine, thianthrene, phenanthridine, phenanthrolin, indolizine, pyrrolopyridine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, 1,2,4-triazolo[4,3-a]pyridine, 1,2,4-triazolo[4,3-b]pyridazine, thienopyrazine and the like (preferably, a heterocycle obtained by condensation of 1 or 2 (preferably, 1) from the aforementioned 5- or 6-membered monocyclic aromatic heterocycles and 1 or 2

(preferably, 1) benzene rings, or a heterocycle obtained by condensation of the same or different, 2 or 3 (preferably, 2) heterocycles from the aforementioned 5-or 6-membered monocyclic aromatic heterocycles) and the like.

Ring G is an aromatic hydrocarbon ring or an aromatic heterocycle, each of which optionally has substituent(s). In the formula (I), as the "aromatic hydrocarbon ring" of the "aromatic hydrocarbon ring optionally having substituent(s)" for ring G, a $C_6$-$_{14}$ aromatic hydrocarbon ring is preferable, a $C_6$-$_{12}$ aromatic hydrocarbon ring is more preferable, and benzene is particularly preferable.

In the formula (I), as the "aromatic heterocycle" of the "aromatic heterocycle optionally having substituent(s)" for ring G, a 5- or 6-membered monocyclic aromatic heterocycle (e.g., a 5-membered ring containing, besides carbon atom, 1 - 4 hetero atoms selected from nitrogen atom, oxygen atom and sulfur atom, such as thiophene, furan, oxazole, isoxazole, thiazole, isothiazole, thiadiazole, imidazole, pyrazole, triazole, tetrazole and the like, a 6-membered ring containing 1 - 3 nitrogen atoms besides carbon atom, such as pyridine, pyrimidine, triazine, pyridazine, pyrazine and the like), or a condensed aromatic heterocycle obtained by condensation of one of the aforementioned 5- or 6-membered monocyclic aromatic heterocycles and one benzene ring is preferable. Specifically, pyrazole, pyridine, pyrimidine, imidazole, triazole, thiazole, thiophene, 1H-indazole, imidazo[1,2-a]pyridine, benzimidazole or benzoxazole is preferable, and benzimidazole is more preferable.

Ring G is preferably an aromatic hydrocarbon ring optionally having substituent(s), more preferably a benzene ring optionally having substituent(s).

Other preferable embodiments of ring G are a benzene ring optionally having substituent(s) and a benzimidazole ring optionally having substituent(s). More preferred are a benzene ring optionally having substituent(s) and a substituted benzimidazole ring, and more preferred is a substituted benzene ring.

Examples of the "substituent" of the "aromatic hydrocarbon ring optionally having substituent(s)" and "aromatic heterocycle optionally having substituent(s)" include

(1) a halogen atom (e.g., fluorine, chlorine, bromine, iodine),
(2) a $C_{1-6}$ alkyl group (e.g., methyl, ethyl, propyl, isopropyl, butyl, isobutyl, sec-butyl, tert-butyl, pentyl, hexyl) optionally substituted by a halogen atom,
(3) a $C_3$-$_6$ cycloalkyl group (e.g., cyclopropyl, cyclobutyl, cyclopentyl, cyclohexyl),
(4) a $C_2$-$_6$ alkynyl group (e.g., ethynyl, 1-propynyl, propargyl),
(5) a $C_2$-$_6$ alkenyl group (e.g., vinyl, allyl, isopropenyl, butenyl, isobutenyl),
(6) a $C_7$-$_{12}$ aralkyl group (e.g., benzyl, α-methylbenzyl, phenethyl),
(7) a $C_{6-10}$ aryl group (e.g., phenyl, naphthyl, preferably phenyl group),
(8) a $C_{1-6}$ alkoxy group (e.g., methoxy, ethoxy, propoxy, isopropoxy, butoxy, isobutoxy, sec-butoxy, tert-butoxy) optionally substituted by a halogen atom,
(9) a $C_6$-$_{10}$ aryloxy group (e.g., phenoxy),
(10) a formyl group or $C_{1-6}$ alkyl-carbonyl group (e.g., acetyl, propionyl, butyryl, isobutyryl),
(11) a $C_{6-10}$ aryl-carbonyl group (e.g., benzoyl, naphthoyl),
(12) a formyloxy group or $C_{1-6}$ alkyl-carbonyloxy group (e.g., acetyloxy, propionyloxy, butyryloxy, isobutyryloxy),
(13) a $C_6$-$_{10}$ aryl-carbonyloxy group (e.g., benzoyloxy, naphthoyloxy),
(14) a carboxyl group,
(15) a $C_{1-6}$ alkoxy-carbonyl group (e.g., methoxycarbonyl, ethoxycarbonyl, propoxycarbonyl, isopropoxycarbonyl, butoxycarbonyl, isobutoxycarbonyl, tert-butoxycarbonyl),
(16) a $C_7$-$_{12}$ aralkyloxy-carbonyl group (e.g., benzyloxycarbonyl),
(17) a carbamoyl group,
(18) a mono-, di- or tri-halogeno-$C_{1-6}$ alkyl group (e.g., chloromethyl, dichloromethyl, trifluoromethyl, 2,2,2-trifluoroethyl),
(19) an oxo group,
(20) an amidino group,
(21) an imino group,
(22) an amino group,
(23) a mono-$C_{1-6}$ alkylamino group (e.g., methylamino, ethylamino, propylamino, isopropylamino, butylamino),
(24) a di-$C_{1-6}$, alkylamino group (e.g., dimethylamino, diethylamino, dipropylamino, diisopropylamino, dibutylamino, N-ethyl-N-methylamino),
(25) a 3- to 8-membered nitrogen-containing heterocyclic group optionally having substituent(s) and optionally containing, besides a carbon atom and one nitrogen atom, 1 - 3 hetero atoms selected from nitrogen atom, oxygen atom and sulfur atom (e.g., a 3- to 8-membered nitrogen-containing heterocyclic group optionally containing, besides a carbon atom and one nitrogen atom, 1 - 3 hetero atoms selected from nitrogen atom, oxygen atom and sulfur atom and optionally having 1 - 5 substituents selected from halogen atom, nitro group, cyano group, hydroxyl group, optionally halogenated $C_{1-6}$ alkyl group (e.g., methyl, ethyl, propyl, isopropyl, butyl, isobutyl, sec-butyl, tert-butyl,

pentyl, hexyl), optionally halogenated $C_{1-6}$ alkoxy group (e.g., methoxy, ethoxy, propoxy, isopropoxy, butoxy, isobutoxy, sec-butoxy, tert-butoxy), amino group, mono-$C_{1-6}$ alkylamino group (e.g., methylamino, ethylamino, propylamino, isopropylamino, butylamino), di-$C_{1-6}$ alkylamino group (e.g., dimethylamino, diethylamino, dipropylamino, diisopropylamino, dibutylamino, N-ethyl-N-methylamino), carboxyl group, $C_{1-6}$ alkyl-carbonyl group (e.g., acetyl, propionyl, butyryl, isobutyryl), $C_{1-6}$ alkoxy-carbonyl group (e.g., methoxycarbonyl, ethoxycarbonyl, propoxycarbonyl, isopropoxycarbonyl, butoxycarbonyl, isobutoxycarbonyl, tert-butoxycarbonyl), carbamoyl group, mono-$C_{1-6}$ alkylcarbamoyl group (e.g., methylcarbamoyl, ethylcarbamoyl, propylcarbamoyl, isopropylcarbamoyl, butylcarbamoyl, isobutylcarbamoyl, sec-butyl, pentylcarbamoyl, hexylcarbamoyl), di-$C_{1-6}$ alkyl-carbamoyl group (e.g., dimethylcarbamoyl, diethylcarbamoyl, dipropylcarbamoyl), $C_{6-10}$ aryl-carbamoyl group (e.g., phenylcarbamoyl, naphthylcarbamoyl), $C_{6-10}$ aryl group (e.g., phenyl, naphthyl), $C_{6-10}$ aryloxy group (e.g., phenoxy), optionally halogenated $C_{1-6}$ alkyl-carbonylamino group (e.g., acetylamino, propionylamino, butyrylamino, isobutyrylamino), oxo group and the like; for example, aziridinyl, azetidinyl, pyrrolidinyl, pyridyl, pyrrolinyl, pyrrolyl, imidazolyl, pyrazolyl, imidazolidinyl, piperidyl, oxadiazolyl, isoxazolyl, morpholinyl, dihydropyridyl, tetrahydropyridyl, piperazinyl, N-methylpiperazinyl, N-ethylpiperazinyl),

(26) a $C_{1-3}$ alkylenedioxy group (e.g., methylenedioxy, ethylenedioxy),
(27) a hydroxyl group,
(28) a nitro group,
(29) a cyano group,
(30) a mercapto group,
(31) a sulfo group,
(32) a sulfino group,
(33) a phosphono group,
(34) a sulfamoyl group,
(35) a mono-$C_{1-6}$ alkylsulfamoyl group (e.g., N-methylsulfamoyl, N-ethylsulfamoyl, N-propylsulfamoyl, N-isopropylsulfamoyl, N-butylsulfamoyl),
(36) a di-$C_{1-6}$ alkylsulfamoyl group (e.g., N,N-dimethylsulfamoyl, N,N-diethylsulfamoyl, N,N-dipropylsulfamoyl, N, N-dibutylsulfamoyl),
(37) a $C_{1-6}$ alkylthio group (e.g., methylthio, ethylthio, propylthio, isopropylthio, butylthio, sec-butylthio, tert-butylthio),
(38) a $C_{6-10}$ arylthio group (e.g., phenylthio, naphthylthio),
(39) a $C_{1-6}$ alkylsulfinyl group (e.g., methylsulfinyl, ethylsulfinyl, propylsulfinyl, butylsulfinyl),
(40) a $C_{6-10}$ arylsulfinyl group (e.g., phenylsulfinyl, naphthylsulfinyl),
(41) a $C_{1-6}$ alkylsulfonyl group (e.g., methylsulfonyl, ethylsulfonyl, propylsulfonyl, butylsulfonyl),
(42) a $C_{6-10}$ arylsulfonyl group (e.g., phenylsulfonyl, naphthylsulfonyl)

(in the present specification, the above-mentioned substituents are collectively referred to as substituent group (a)) and the like.

The "aromatic hydrocarbon ring" and "aromatic heterocycle" of the "aromatic hydrocarbon ring optionally having substituent(s)" and "aromatic heterocycle optionally having substituent(s)" for ring G optionally have 1 - 5, preferably 1 - 3, more preferably 1 or 2.of the aforementioned substituents at substitutable position(s) on the ring. Especially, the "aromatic hydrocarbon ring" of the "aromatic hydrocarbon ring optionally having substituent(s)" particularly preferably has 1 - 3 (preferably 1 or 2, more preferably 2) of the aforementioned substituents at substitutable position(s) on the ring. The "aromatic heterocycle" of the "aromatic heterocycle optionally having substituent(s)" particularly preferably has one of the aforementioned substituents at a substitutable position on the ring. When the number of the substituents is two or more, respective substituents may be the same or different. In addition, these substituents are optionally substituted by 1 to 3 substituents from substituent group (a) at substitutable position (s).

As the substituent of the "aromatic hydrocarbon ring" of the "aromatic hydrocarbon ring optionally having substituent(s)" for ring G,

(1) a halogen atom (e.g., fluorine, chlorine, bromine),
(2) a $C_{1-6}$ alkyl group (e.g., trifluoromethyl, isopropyl) optionally substituted by a halogen atom,
(3) a $C_{1-6}$ alkoxy group (e.g., trifluoromethoxy, methoxy) optionally substituted by a halogen atom,
(4) a $C_{2-6}$ alkenyl group (e.g., 1-methylethenyl),
(5) a $C_{1-6}$ alkylthio group (e.g., methylthio),
(6) a $C_{1-6}$ alkylsulfonyl group (e.g., methylsulfonyl),
(7) a morpholinyl group

and the like are preferable.
Among these,

(1) a halogen atom (e.g., fluorine, chlorine, bromine),
(2) a $C_{1-6}$ alkyl group (e.g., trifluoromethyl) substituted by a halogen atom,
(3) a $C_{1-6}$ alkoxy group (e.g., trifluoromethoxy) substituted by a halogen atom,
(4) a $C_{2-6}$ alkenyl group (e.g., 1-methylethenyl),
(5) a $C_{1-6}$ alkylthio group (e.g., methylthio),
(6) a $C_{1-6}$ alkylsulfonyl group (e.g., methylsulfonyl),
(7) a morpholinyl group

and the like are preferable.
Among these,

(1) a halogen atom (e.g., fluorine, chlorine, bromine, preferably, fluorine, chlorine),
(2) a $C_{1-6}$ alkyl group (e.g., trifluoromethyl) substituted by a halogen atom,

and the like are more preferable.
As the substituent of the "aromatic heterocycle" of the "aromatic heterocycle optionally having substituent(s)" for ring G,

(1) a halogen atom,
(2) a $C_{1-6}$ alkyl group optionally substituted by a halogen atom (e.g., trifluoromethyl),
(3) a $C_{1-6}$ alkoxy group optionally substituted by a halogen atom,
(4) a $C_{2}$-$_{6}$ alkenyl group,
(5) a $C_{1-6}$ alkylthio group,
(6) a $C_{1-6}$ alkylsulfonyl group,
(7) a morpholinyl group

and the like are preferable.
Among these,

(1) a halogen atom,
(2) a $C_{1-6}$ alkyl group substituted by a halogen atom (e.g., trifluoromethyl),
(3) a $C_{1-6}$ alkoxy group substituted by a halogen atom,
(4) a $C_{2-6}$ alkenyl group,
(5) a $C_{1-6}$ alkylthio group,
(6) a $C_{1-6}$ alkylsulfonyl group,
(7) a morpholinyl group

and the like are preferable.
Among these, a $C_{1-6}$ alkyl group substituted by a halogen atom (e.g., trifluoromethyl) and the like are more preferable.
Ring A is an imidazole ring, an oxazole ring or a triazole ring, each of which optionally has substituent(s).
As ring A, a substituted imidazole ring, a substituted oxazole ring and a substituted triazole ring are preferable, and substituted oxazole ring is more preferable.
Examples of the "substituent" of the "imidazole ring optionally having substituent(s)", "oxazole ring optionally having substituent(s)" and "triazole ring optionally having substituent(s)" used in the present specification include those exemplified as the above-mentioned substituent group (a).
The "imidazole ring", "oxazole ring" and "triazole ring" of the "imidazole ring optionally having substituent(s)", "oxazole ring optionally having substituent(s)" and "triazole ring optionally having substituent(s)" optionally have 1 - 5, preferably 1 - 3, more preferably 1 or 2, still more preferably 1, from the aforementioned substituents at substitutable position(s) on the ring. When the number of the substituents is two or more, respective substituents may be the same or different. In addition, these substituents are optionally substituted by 1 to 3 substituents from substituent group (a) at substitutable position(s).
As the substituent of the "imidazole ring optionally having substituent(s)", "oxazole ring optionally having substituent(s)" and "triazole ring optionally having substituent(s)" for ring A, a $C_{1-6}$ alkyl group (e.g., methyl) and the like are preferable.
Ring B is a benzene ring, a pyridine ring or a pyrimidine ring, each of which optionally has substituent(s).
As ring B, a benzene ring optionally having substituent(s) and a pyridine ring optionally having substituent(s) are preferable, a substituted benzene ring and a substituted pyridine ring are more preferable, and a substituted benzene ring is further preferable.
Examples of the "substituent" of the "benzene ring optionally having substituent(s)", "pyridine ring optionally

having substituent(s)" and "pyrimidine ring optionally having substituent(s)" used in the present specification include those exemplified as the above-mentioned substituent group (a).
The "benzene ring", "pyridine ring" and "pyrimidine ring" of the "benzene ring optionally having substituent(s)", "pyridine ring optionally having substituent(s)" and "pyrimidine ring optionally having substituent(s)" optionally have 1 - 5, preferably 1 - 3, more preferably 1, from the aforementioned substituents at substitutable position(s) on the ring. When the number of the substituents is two or more, respective substituents may be the same or different. In addition, these substituents are optionally substituted by 1 to 3 substituents from substituent group (a) at substitutable position (s).
As the substituent of the "benzene ring optionally having substituent(s)", "pyridine ring optionally having substituent(s)" and "pyrimidine ring optionally having substituent(s)" for ring B,

(1) a halogen atom (e.g., fluorine)
(2) a cyano group
(3) a $C_{1-6}$ alkoxy group (e.g., methoxy)

and the like are preferable.
Among these, a $C_{1-6}$ alkoxy group (e.g., methoxy) and the like are preferable.
In the formula (I), a group represented by a partial structural formula constituted by ring D and ring E
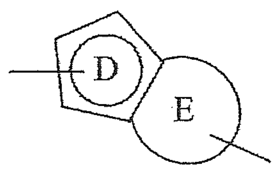

is a group represented by
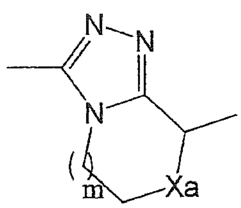

or

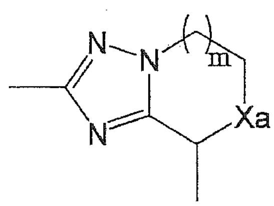

wherein Xa is $-CH_2-$, -NH-, -O-, -S-, -SO-, or $-SO_2-$ and m is 0, 1, or 2, each of which optionally has substituent(s).
Examples of the partial structural formula include
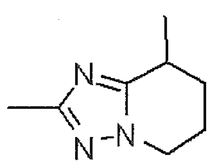

,

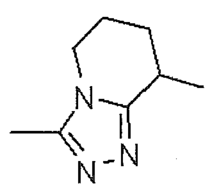

,

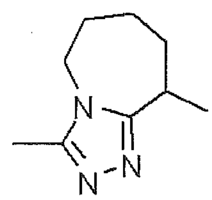

,

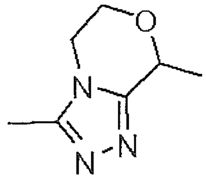

and

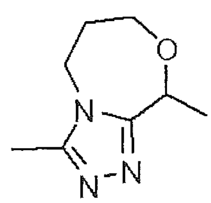

Here, as Xa, $-CH_2-$ is preferable. As m, 1 and 2 is preferable, and 1 is more preferable.

As the partial structural formula, a group represented by

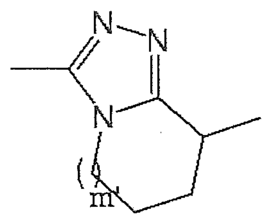

wherein m' is 1 or 2, or

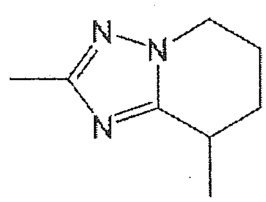

is preferable, and a group represented by

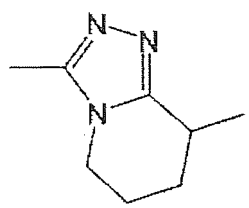

is more preferable.

L is a bond, -O- or -O-$Y^1$- wherein $Y^1$ is a $C_{1-6}$ alkylene group optionally having substituent(s).
As L, a bond, -O- and -O-$Y^{1'}$- wherein $Y^{1'}$ is a $C_{1-6}$ alkylene group are preferable, a bond and -O- are more preferable, and a bond is more preferable.

Examples of the "$C_{1-6}$ alkylene group" of the "$C_{1-6}$ alkylene group optionally having substituent(s)" used in the present specification include methylene, ethylene, propylene, butylene, pentylene, hexylene and the like. Examples of the "substituent" of the "$C_{1-6}$ alkylene group optionally having substituent(s)" include

(1) a halogen atom (e.g., fluorine, chlorine, bromine, iodine),
(2) a $C_{1-6}$ alkyl group (e.g., methyl, ethyl, propyl, isopropyl, butyl, isobutyl, sec-butyl, tert-butyl, pentyl, hexyl),
(3) a $C_{3-6}$ cycloalkyl group (e.g., cyclopropyl, cyclobutyl, cyclopentyl, cyclohexyl),
(4) a $C_{2-6}$ alkynyl group (e.g., ethynyl, 1-propynyl, propargyl),
(5) a $C_{2-6}$ alkenyl group (e.g., vinyl, allyl, isopropenyl, butenyl, isobutenyl),
(6) a $C_{7-12}$ aralkyl group (e.g., benzyl, α-methylbenzyl, phenethyl),
(7) a $C_{6-10}$ aryl group (e.g., phenyl, naphthyl, preferably phenyl),
(8) a $C_{1-6}$ alkoxy group (e.g., methoxy, ethoxy, propoxy, isopropoxy, butoxy, isobutoxy, sec-butoxy, tert-butoxy),
(9) a $C_{6-10}$ aryloxy group (e.g., phenoxy),
(10) a formyl group or $C_{1-6}$ alkyl-carbonyl group (e.g., acetyl, propionyl, butyryl, isobutyryl),
(11) a $C_{6-10}$ aryl-carbonyl group (e.g., benzoyl, naphthoyl),
(12) a formyloxy group or $C_{1-6}$ alkyl-carbonyloxy group (e.g., acetyloxy, propionyloxy, butyryloxy, isobutyryloxy),
(13) a $C_{6-10}$ aryl-carbonyloxy group (e.g., benzoyloxy, naphthoyloxy),
(14) a carboxyl group,
(15) a $C_{1-6}$ alkoxy-carbonyl group (e.g., methoxycarbonyl, ethoxycarbonyl, propoxycarbonyl, isopropoxycarbonyl,

butoxycarbonyl, isobutoxycarbonyl, tert-butoxycarbonyl),
(16) a $C_{7-12}$ aralkyloxy-carbonyl group (e.g., benzyloxycarbonyl),
(17) a carbamoyl group,
(18) a mono-, di- or tri-halogeno-$C_{1-6}$ alkyl group (e.g., chloromethyl, dichloromethyl, trifluoromethyl, 2,2,2-trifluoroethyl),
(19) an oxo group,
(20) an amidino group,
(21) an imino group,
(22) an amino group,
(23) a mono-$C_{1-6}$ alkylamino group (e.g., methylamino, ethylamino, propylamino, isopropylamino, butylamino),
(24) a di-$C_{1-6}$ alkylamino group (e.g., dimethylamino, diethylamino, dipropylamino, diisopropylamino, dibutylamino, N-ethyl-N-methylamino),
(25) a 3- to 8-membered nitrogen-containing heterocyclic group optionally having substituent(s) and optionally containing, besides a carbon atom and one nitrogen atom, 1 - 3 hetero atoms selected from nitrogen atom, oxygen atom and sulfur atom [e.g., a 3- to 8-membered nitrogen-containing heterocyclic group optionally containing, besides a carbon atom and one nitrogen atom, 1 - 3 hetero atoms selected from nitrogen atom, oxygen atom and sulfur atom and optionally having 1 - 5 substituents selected from halogen atom, nitro group, cyano group, hydroxyl group, optionally halogenated $C_{1-6}$ alkyl group (e.g., methyl, ethyl, propyl, isopropyl, butyl, isobutyl, sec-butyl, tert-butyl, pentyl, hexyl), optionally halogenated $C_{1-6}$ alkoxy group (e.g., methoxy, ethoxy, propoxy, isopropoxy, butoxy, isobutoxy, sec-butoxy, tert-butoxy), amino group, mono-$C_{1-6}$ alkylamino group (e.g., methylamino, ethylamino, propylamino, isopropylamino, butylamino), di-$C_{1-6}$ alkylamino group (e.g., dimethylamino, diethylamino, dipropylamino, diisopropylamino, dibutylamino, N-ethyl-N-methylamino), carboxyl group, $C_{1-6}$ alkyl-carbonyl group (e.g., acetyl, propionyl, butyryl, isobutyryl), $C_{1-6}$ alkoxy-carbonyl group (e.g., methoxycarbonyl, ethoxycarbonyl, propoxycarbonyl, isopropoxycarbonyl, butoxycarbonyl, isobutoxycarbonyl, tert-butoxycarbonyl), carbamoyl group, mono-$C_{1-6}$ alkyl-carbamoyl group (e.g., methylcarbamoyl, ethylcarbamoyl, propylcarbamoyl, isopropylcarbamoyl, butylcarbamoyl, isobutylcarbamoyl, sec-butyl, pentylcarbamoyl, hexylcarbamoyl), di-$C_{1-6}$ alkyl-carbamoyl group (e.g., dimethylcarbamoyl, diethylcarbamoyl, dipropylcarbamoyl), $C_{6-10}$ aryl-carbamoyl group (e.g., phenylcarbamoyl, naphthylcarbamoyl), $C_{6-10}$ aryl group (e.g., phenyl, naphthyl), $C_{6-10}$ aryloxy group (e.g., phenoxy), optionally halogenated $C_{1-6}$ alkyl-carbonylamino group (e.g., acetylamino, propionylamino, butyrylamino, isobutyrylamino), oxo group and the like; for example, aziridinyl, azetidinyl, pyrrolidinyl, pyridyl, pyrrolinyl, pyrrolyl, imidazolyl, pyrazolyl, imidazolidinyl, piperidyl, oxadiazolyl, isoxazolyl, morpholinyl, dihydropyridyl, tetrahydropyridyl, piperazinyl, N-methylpiperazinyl, N-ethylpiperazinyl],
(26) a $C_{1-3}$ alkylenedioxy group (e.g., methylenedioxy, ethylenedioxy),
(27) a hydroxyl group,
(28) a nitro group,
(29) a cyano group,
(30) a mercapto group,
(31) a sulfo group,
(32) a sulfino group,
(33) a phosphono group,
(34) a sulfamoyl group,
(35) a mono-$C_{1-6}$ alkylsulfamoyl group (e.g., N-methylsulfamoyl, N-ethylsulfamoyl, N-propylsulfamoyl, N-isopropylsulfamoyl, N-butylsulfamoyl),
(36) a di-$C_{1-6}$ alkylsulfamoyl group (e.g., N,N-dimethylsulfamoyl, N,N-diethylsulfamoyl, N,N-dipropylsulfamoyl, N,N-dibutylsulfamoyl),
(37) a $C_{1-6}$ alkylthio group (e.g., methylthio, ethylthio, propylthio, isopropylthio, butylthio, sec-butylthio, tert-butylthio),
(38) a $C_{6-10}$ arylthio group (e.g., phenylthio, naphthylthio),
(39) a $C_{1-6}$ alkylsulfinyl group (e.g., methylsulfinyl, ethylsulfinyl, propylsulfinyl, butylsulfinyl),
(40) a $C_{6-10}$ arylsulfinyl group (e.g., phenylsulfinyl, naphthylsulfinyl),
(41) a $C_{1-6}$ alkylsulfonyl group (e.g., methylsulfonyl, ethylsulfonyl, propylsulfonyl, butylsulfonyl),
(42) a $C_{6-10}$ arylsulfonyl group (e.g., phenylsulfonyl, naphthylsulfonyl)

(in the present specification, the above-mentioned substituents are collectively referred to as substituent group (b)) and the like.
The "$C_{1-6}$ alkylene group" of the "$C_{1-6}$ alkylene group optionally having substituent(s)" optionally has 1 - 5, preferably 1 - 3, from the aforementioned substituents at substitutable position(s) on the alkylene chain. When the number of the substituents is two or more, respective substituents may be the same or different. In addition, these substituents are optionally substituted by 1 to 3 substituents from substituent group (b) at substitutable position(s).

As the "$C_{1-6}$ alkylene group optionally having substituent(s)" for $Y^1$, a $C_{1-6}$ alkylene group is preferable, and methylene is more preferable.

Examples of the "substituent" that the above-mentioned partial structural formula optionally has include those exemplified as the above-mentioned substituent group (a), each of which optionally has 1 - 5, preferably 1 - 3, from the aforementioned substituents at substitutable position(s) on the ring (e.g., ring E). When the number of the substituents is two or more, respective substituents may be the same or different. In addition, these substituents are optionally substituted by 1 to 3 substituents from substituent group (a) at substitutable position(s).

As compound (I) or a salt thereof, the following compounds (Ia) - (Ih) are preferably used.

[Compound (Ia)]

In compounds represented by the formula (I):

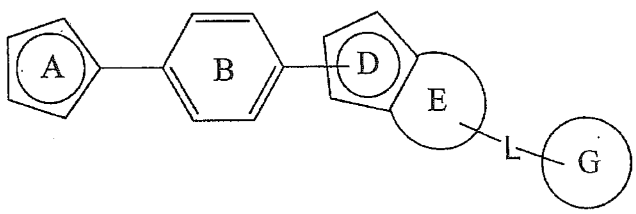

(I)

wherein each symbol is as defined above,
a compound wherein
ring A is an imidazole ring, an oxazole ring, or a triazole ring, each of which has a substituent (e.g., methyl);
ring B is a benzene ring or a pyridine ring, each of which has a substituent (e.g., methoxy, fluorine atom, cyano);
a group represented by a partial structural formula

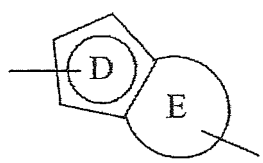

is a group represented by

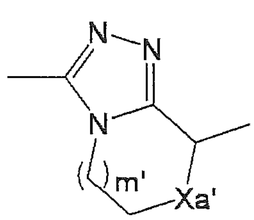

or

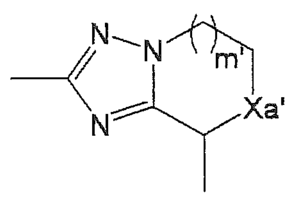

wherein Xa' is -O- or $-CH_2-$ (preferably Xa' is $-CH_2-$), and m' is 1 or 2 (preferably m' is 1), preferably,

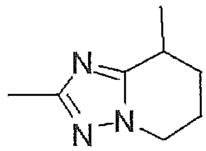 , 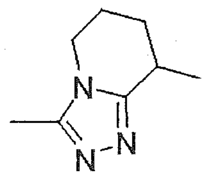 or 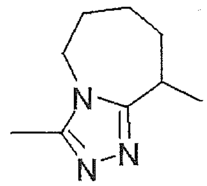

L is a bond, -O- or -O-Y1'- wherein Y1' is a $C_{1-6}$ alkylene group (e.g., methylene) (preferably L is a bond, -O-$CH_2$-, or -O-); and
ring G is a benzene ring optionally substituted by (1) a halogen atom (e.g., fluorine, chlorine, bromine), (2) a $C_{1-6}$ alkyl group optionally substituted by a halogen atom (e.g., trifluoromethyl, isopropyl), (3) a $C_{1-6}$ alkoxy group optionally substituted by a halogen atom (e.g., trifluoromethoxy, methoxy), (4) a $C_2$-$_6$ alkenyl group (e.g., 1-methylethenyl), (5) a $C_{1-6}$ alkylthio group (e.g., methylthio), (6) a $C_{1-6}$ alkylsulfonyl group (e.g., methylsulfonyl) or (7) a morpholinyl group, or a benzimidazole ring optionally substituted by a $C_{1-6}$ alkyl group optionally substituted by a halogen atom (e.g., trifluoromethyl);
is preferable.

[Compound (Ib)]

Compound (I) wherein
ring A is an imidazole ring, an oxazole ring or a triazole ring, each of which optionally has substituent(s),
ring B is a benzene ring, a pyridine ring or a pyrimidine ring, each of which optionally has substituent(s),
a group represented by a partial structural formula in the formula (I)
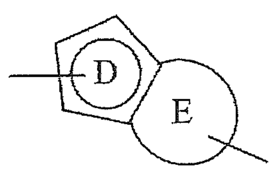

is a group represented by
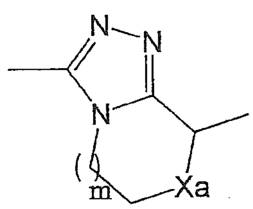 or 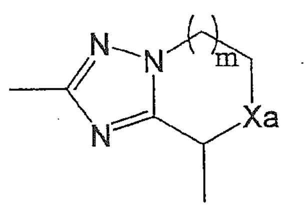

wherein Xa is -$CH_2$-, -NH-, -O-, -S-, -SO- or -$SO_2$- and m is 0, 1 or 2, each of which optionally has substituent(s),
L is a bond, -O- or -O-$Y^1$- wherein $Y^1$ is a $C_{1-6}$ alkylene group optionally having substituent(s), and
ring G is a benzene ring or a benzimidazole ring, each of which optionally has substituent(s),
or a salt thereof.

[Compound (Ic)]

Compound (I) wherein
ring A is an imidazole ring, an oxazole ring or a triazole ring, each of which is optionally substituted by 1 to 3 $C_{1-6}$ alkyl groups,
ring B is a benzene ring or a pyridine ring, each of which is optionally substituted by 1 to 3 substituents selected from a

halogen atom, a cyano group and a $C_{1-6}$ alkoxy group,
a group represented by a partial structural formula in the formula (I)

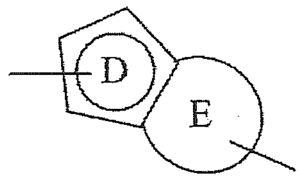

is a group represented by

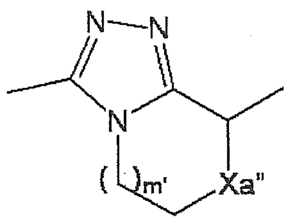

or

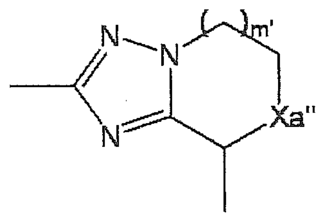

wherein Xa" is $-CH_2-$ and m' is 1 or 2, each of which optionally has substituent(s),
L is a bond, -O- or -O-$Y^{1'}$- wherein $Y^{1'}$ is a $C_{1-6}$ alkylene group, and
ring G is a benzene ring or a benzimidazole ring, each of which is optionally substituted by 1 to 3 substituents selected from a halogen atom, a $C_{1-6}$ alkyl group optionally substituted by a halogen atom, a $C_{1-6}$ alkoxy group optionally substituted by a halogen atom, a $C_{2-6}$ alkenyl group, a $C_{1-6}$ alkylthio group, a $C_{1-6}$ alkylsulfonyl group and a morpholinyl group,
or a salt thereof.

[Compound (Id)]

Compound (I) wherein
ring A is an imidazole ring, an oxazole ring or a triazole ring, each of which is substituted by 1 - 3 (preferably 1) $C_{1-6}$ alkyl group"
ring B is a benzene ring or a pyridine ring, each of which is substituted by 1 - 3 (preferably 1) substituent selected from a halogen atom, a cyano group and a $C_{1-6}$ alkoxy group,
a group represented by a partial structural formula in the formula (I)

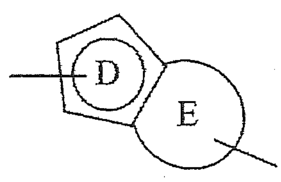

is a group represented by

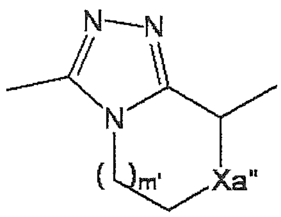 or 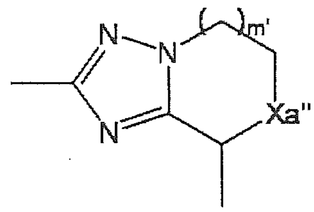

wherein Xa" is $-CH_2-$, and m' is 1 or 2, each of which optionally has substituent(s),
L is a bond, -O- or -O-$Y^{1'}$ - wherein $Y^{1'}$ is a $C_{1-6}$ alkylene group, and
ring G is

(1) a benzene ring optionally substituted by 1 - 3 (preferably 1 or 2) substituents selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group, or
(2) a benzimidazole ring optionally substituted by 1 - 3 (preferably 1) substituent selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group,

or a salt thereof.

[Compound (Ie)]

Compound (I) wherein
ring A is an imidazole ring, an oxazole ring or a triazole ring, each of which is substituted by 1 - 3 (preferably 1) $C_{1-6}$ alkyl group,
ring B is

(1) a benzene ring substituted by 1 - 3 (preferably 1) substituent selected from a halogen atom, a cyano group and a $C_{1-6}$ alkoxy group, or
(2) a pyridine ring substituted by 1 - 3 (preferably 1) $C_{1-6}$ alkoxy group,

a group represented by a partial structural formula in the formula (I)
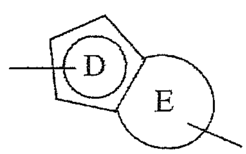

is a group represented by
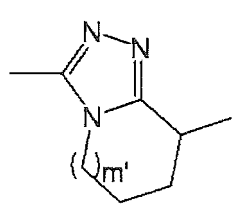

wherein m' is 1 or 2, or
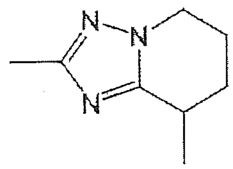

,

L is a bond, -O- or $-O-Y^{1'}-$ wherein $Y^1$ is a $C_{1-6}$ alkylene group, and ring G is

(1) a benzene ring optionally substituted by 1 - 3 (preferably 1 or 2) substituents selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group, or
(2) a benzimidazole ring substituted by 1 - 3 (preferably 1) $C_{1-6}$ alkyl group substituted by a halogen atom,

or a salt thereof.

[Compound (If)]

Compound (I) wherein
ring A is an oxazole ring substituted by 1 or 2 (preferably 1) $C_{1-6}$ alkyl group,
ring B is a benzene ring substituted by 1 - 3 (preferably 1) $C_{1-6}$ alkoxy group,
a group represented by a partial structural formula in the formula (I)
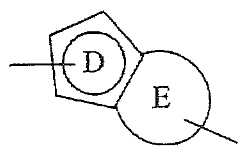

is a group represented by
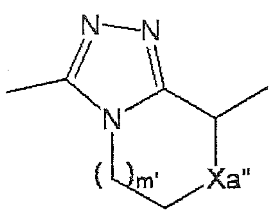

or

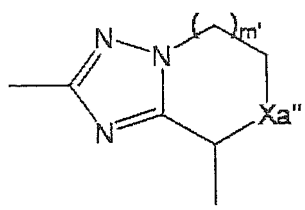

wherein Xa" is $-CH_2-$, and m' is 1 or 2, each of which optionally has substituent(s),
L is a bond or -O-, and
ring G is a benzene ring substituted by 1 - 3 (preferably 1 or 2) substituents selected from a halogen atom and a $C_{1-6}$ alkyl group substituted by a halogen atom,
or a salt thereof.

[Compound (Ig)]

Compound (I) wherein
ring A is an oxazole ring substituted by 1 or 2 (preferably 1) $C_{1-6}$ alkyl group,
ring B is a benzene ring substituted by 1 - 3 (preferably 1) $C_{1-6}$ alkoxy group,
a group represented by a partial structural formula in the formula (I)

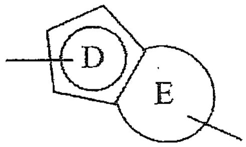

is a group represented by

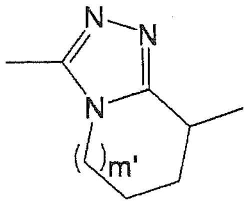

wherein m' is 1 or 2, or

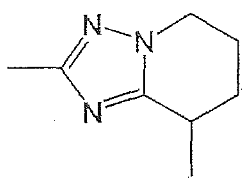

,

L is a bond or -O-, and
ring G is a benzene ring substituted by 1 - 3 (preferably 1 or 2) substituents selected from a halogen atom and a $C_{1-6}$ alkyl group substituted by a halogen atom,
or a salt thereof.

[Compound (Ih)]

Compound (I) wherein
ring A is an oxazole ring substituted by 1 or 2 (preferably 1) $C_{1-6}$ alkyl group,
ring B is a benzene ring substituted by 1 - 3 (preferably 1) $C_{1-6}$ alkoxy group,
a group represented by a partial structural formula in the formula (I)

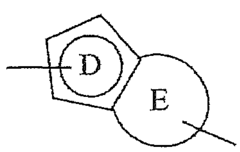

is a group represented by

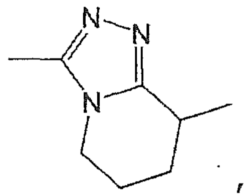

,

L is a bond, and
ring G is a benzene ring substituted by 1 - 3 (preferably 2) substituents selected from a halogen atom and a $C_{1-6}$ alkyl group substituted by a halogen atom,
or a salt thereof.

As compound (I) or a salt thereof, the compounds described in the Example are more preferable.
Among them,
8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine (compound described in Example 28) or a salt thereof, and
8-[4-chloro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine (compound described in Example 34) or a salt thereof
are particularly preferable.

When compound (I) is a salt, examples of such salt include metal salts, ammonium salt, salts with organic base, salts with inorganic acid, salts with organic acid, salts with basic or acidic amino acid and the like. Preferable examples of metal salts include alkali metal salts such as sodium salt, potassium salt and the like; alkaline earth metal salts such as calcium salt, magnesium salt, barium salt and the like; aluminum salt and the like. Preferable examples of salts with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of salts with inorganic acid include salts with hydrochloric acid, hydrobromic acid, nitric acid, sulfuric acid, phosphoric acid and the like. Preferable examples of salts with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of salts with basic amino acid include salts with arginine, lysine, ornithine and the like, and preferable examples of salts with acidic amino acid include salts with aspartic acid, glutamic acid and the like.
Of these, pharmaceutically acceptable salts are preferable. When a compound has an acidic functional group, preferable examples thereof include inorganic salts such as alkali metal salts (e.g., sodium salt, potassium salt etc.), alkaline earth metal salts (e.g., calcium salt, magnesium salt etc.) and the like, ammonium salts and the like. When the compound has an basic functional group, preferable examples thereof include salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic and the like.
In the following, compound (I) and a salt thereof are also generically referred to as the compound of the present invention.

The compound of the present invention and starting compounds thereof can be produced according to a method known per se, for example, a method shown in the following schemes, and the like. In the following, the "room temperature" generally shows 10 to 30°C, and each symbol in the chemical structures described in the schemes is as defined above unless otherwise specified. The compound in the formula also includes the form of a salt, and examples of such salt include those similar to the salts of the compound of the present invention and the like. In addition, while the compound obtained in each step can be used for the next reaction as a reaction mixture or a crude product, it can also be isolated from a reaction mixture according to a conventional method, or can be easily purified by a separation means such as recrystallization, distillation, chromatography and the like. When the compound in the formula is commercially available, a commercially available product can be directly used. In addition, when each ring in the formula (I) has a substituent, the corresponding precursor is considered to also have a similar substituent.
When the starting compound has amino, carboxy, hydroxy or heterocyclic group, these groups may be protected by a protecting group generally used in the peptide chemistry and the like. In this case, the object compound can be obtained by removing the protecting group as necessary after the reaction. Introduction and removal of these protecting groups can be performed by a method known per se, for example, the method described in "Protective Groups in Organic Synthesis, 3rd Ed." (Theodora W. Greene, Peter G. M. Wuts, Wiley-Interscience, 1999) and the like. In the formula, $P^1$ to $P^4$ are each a protecting group of a nitrogen atom in amine or amide, a protecting group of a hydroxy group, or a hydrogen atom, and those known per se can be used. For example, as $P^1$ - $P^4$, preferred are tert-butylcarbamate group, benzylcarbamate group, benzyl group, methyl group, ethyl group and the like. In addition, $P^1$ - $P^4$ per se may be the substituents of the compound of the present invention and, for example, tert-butylcarbamate group, benzylcarbamate

group, benzyl group, methyl group, ethyl group and the like can be mentioned.
As the "leaving group" for $LG^1$ - $LG^6$, for example, a halogen atom (e.g., a chlorine atom, a bromine atom, an iodine atom etc.), $C_{1-6}$ alkylsulfonyloxy (e.g., methanesulfonyloxy, ethanesulfonyloxy, trifluoromethanesulfonyloxy etc.), $C_{6-10}$ arylsulfonyloxy (e.g., benzenesulfonyloxy, p-toluenesulfonyloxy etc.), $C_{1-6}$ alkylsulfonyl (e.g., methanesulfonyl, ethanesulfonyl etc.) and the like are used.
In addition, $LG^1$ - $LG^6$ also include a substituent that can be converted to a leaving group, and can be converted to a leaving group in a desired step by a reaction known per se.
For example, when $LG^1$ - $LG^6$ are methylthio groups, they can be converted to methanesulfonyl groups by oxidation reaction.

Each step described below can be performed without solvent, or by dissolving or suspending in an appropriate solvent, where two or more kinds of solvents may be used by mixing them at an appropriate ratio. Of those recited as examples of the solvent to be used in the production method of the compound of the present invention, the following solvents are specifically used.

alcohols:

methanol, ethanol, 1-propanol, 2-propanol, tert-butyl alcohol, 2-methoxyethanol etc.

ethers:

diethyl ether, diisopropyl ether, diphenylether, tetrahydrofuran, 1,4-dioxane, 1,2-dimethoxyethane, etc.

aromatic hydrocarbons:

benzene, chlorobenzene, toluene, xylene, etc.

saturated hydrocarbons:

cyclohexane, hexane, etc.

amides:

N,N-dimethylformamide, N,N-dimethylacetamide, hexamethylphosphoric triamide, etc.

halogenated hydrocarbons:

dichloromethane, chloroform, carbon tetrachloride, 1,2-dichloroethane, etc.

nitriles:

acetonitrile, propionitrile, etc.

sulfoxides:

dimethyl sulfoxide, etc.

aromatic organic bases:

pyridine, lutidine, etc.

acid anhydrides:

acetic anhydride, etc.

organic acids:

formic acid, acetic acid, propionic acid, trifluoroacetic acid, methanesulfonic acid, etc.

inorganic acids:

hydrochloric acid, sulfuric acid, etc.

esters:

methyl acetate, ethyl acetate, butyl acetate, etc.

ketones:

acetone, methylethyl ketone, etc.

Of those recited as examples of the base or deoxidizer to be used in the production method of the compound of the present invention, the following bases and deoxidizers are specifically used.
inorganic bases:

sodium hydroxide, potassium hydroxide, magnesium hydroxide, etc.

basic salts:

sodium carbonate, potassium carbonate, cesium carbonate, calcium carbonate, sodium hydrogen carbonate, etc.

organic bases:

triethylamine, diisopropylethylamine, tributylamine, cyclohexyldimethylamine, pyridine, lutidine, 4-dimethylaminopyridine, N,N-dimethylaniline, N-methylpiperidine, N-methylpyrrolidine, N-methylmorpholine, 1,5-diazabicyclo[4.3.0]-5-nonene, 1,4-diazabicyclo[2.2.2]octane, 1,8-diazabicyclo[5.4.0]-7-undecene, imidazole, etc.

metal alkoxides:

sodium methoxide, sodium ethoxide, potassium tert-butoxide, etc.

alkali metal hydrides:

sodium hydride, potassium hydride, etc.

metal amides:

sodium amide, lithium diisopropylamide, lithium hexamethyldisilazide, etc.

organic lithiums:

methyl lithium, n-butyl lithium, sec-butyl lithium, tert-butyl lithium, etc.

Of those recited as examples of the acid or acidic catalyst to be used in the production method of the compound of the present invention, the following acid and acidic catalyst are specifically used.
inorganic acids:

hydrochloric acid, sulfuric acid, nitric acid, hydrobromic acid, phosphoric acid, etc.

organic acids:

acetic acid, trifluoroacetic acid, oxalic acid, phthalic acid, fumaric acid, tartaric acid, maleic acid, citric acid, succinic acid, methanesulfonic acid, p-toluenesulfonic acid, 10-camphorsulfonic acid, etc.

Lewis acids:

boron trifluoride ether complex, zinc iodide, anhydrous aluminum chloride, anhydrous zinc chloride, anhydrous iron chloride, etc.

Production method A of the compound of the present invention

(reaction 01)

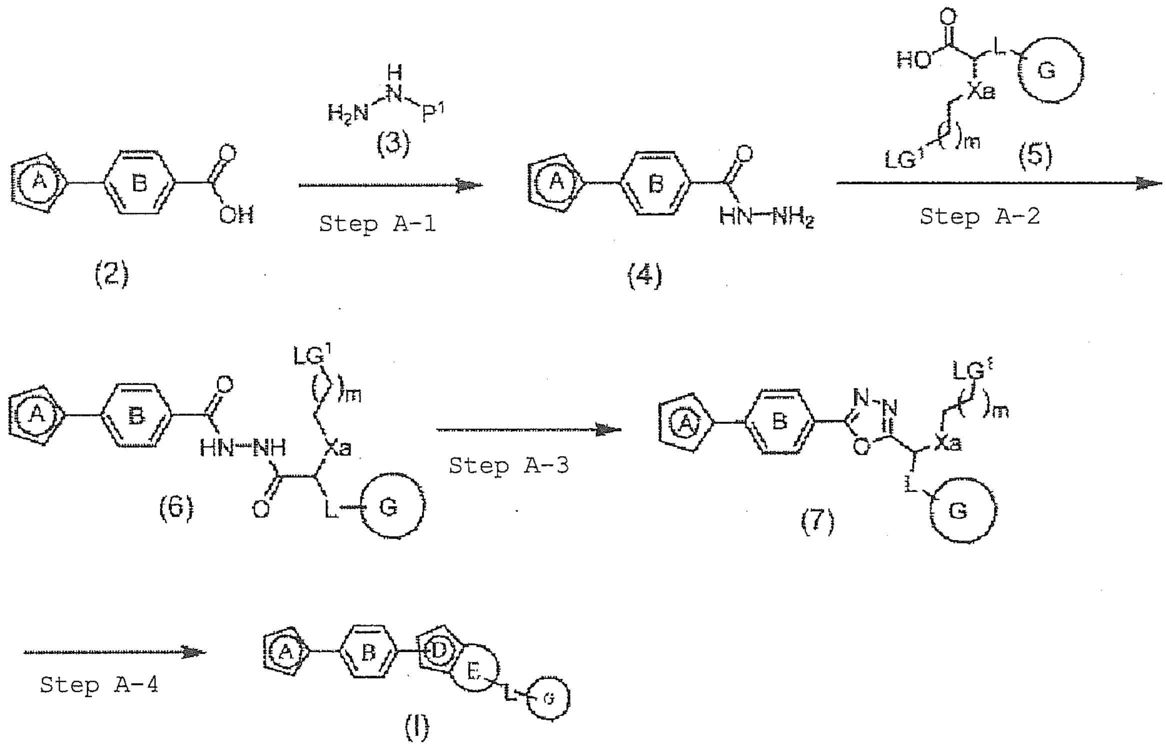


wherein each symbol is as defined above.
The compound of the present invention can be produced by performing a series of reaction steps of step A-1 to step A-4.

(step A-1)

Compound (4) can be produced by reacting carboxylic acid or a salt thereof or a reactive derivative thereof with compound (3), followed by removal of the protecting group $P^1$. When $P^1$ is a hydrogen atom, removal of the protecting group can be omitted. Examples of a reactive derivative of the carboxylic acid include acid halides such as acid chloride, acid bromide and the like, acid amides with pyrazole, imidazole, benzotriazole and the like, acid anhydrides such as acetic anhydride, propanoic anhydride, butanoic anhydride and the like, acid azides, active esters such as diethoxy phosphate ester, diphenoxy phosphate ester, p-nitrophenyl ester, 2,4-dinitrophenyl ester, cyanomethyl ester, pentachlorophenyl ester, ester with N-hydroxysuccinimide, ester with N-hydroxyphthalimide, ester with 1-hydroxybenzotriazole, ester with 6-chloro-1-hydroxybenzotriazole, ester with 1-hydroxy-1H-2-pyridone and the like, active thioesters such as 2-pyridyl thioester, 2-benzothiazolyl thioester and the like, and the like. Instead of using reactive derivative, carboxylic acid or a salt thereof may be directly reacted with compound (3) in the presence of a suitable condensing agent. Examples of the condensing agent include N,N'-disubstituted carbodiimides such as N,N'-dicyclohexylcarbodiimide, 1-ethyl-3-(3-dimethylaminopropyl)carbodiimide (WSC) hydrochloride and the like, azolides such as N,N'-carbonyldiimidazole and the like, dehydrating agents such as N-ethoxycarbonyl-2-ethoxy-1,2-dihydroquinoline, phosphorus oxychloride, alkoxyacetylene and the like, 2-halogenopyridinium salts such as 2-chloromethylpyridinium iodide, 2-fluoro-1-methylpyridinium iodide and the like, phosphoryl cyanides such as diethylphosphoryl cyanide and the like, 2-(7-azabenzotriazol-1-yl)-1,1,3,3-hexafluorophosphate (HATU), O-(7-azabenzotriazol-1-yl)-N,N,N',N'-tetramethyluronium tetrafluoroborate (TATU) and the like. When these condensing agents are used, the reaction is considered to proceed via a reactive derivative of carboxylic acid. The amount of carboxylic acid or a salt thereof or a reactive derivative thereof to be used is generally about 0.2 - 5.0 mol, preferably about 0.5 - 2.0 mol, per 1 mol of compound (3). This reaction is advantageously performed using a solvent inert to the reaction. Such solvent is not particularly limited as long as the reaction proceeds and, for example, a solvent such as ethers, aromatic hydrocarbons, saturated hydrocarbons, amides, halogenated hydrocarbons, nitriles, sulfoxides, aromatic organic bases and the like or a mixed solvent thereof and the like are

preferable. When an acidic substance is released by the reaction, the reaction can be performed in the presence of a deoxidizing agent to remove the substance from the reaction system. As such deoxidizing agent, basic salts, organic bases and the like are used. For example, basic salts, organic bases and the like can also be used to promote the reaction. While the reaction time varies depending on the reagent and solvent to be used, it is generally 10 min - 72 hr, preferably 30 min - 24 hr. The reaction temperature is generally 0 - 100˚C, preferably 0 - 70˚C.

(step A-2)

Compound (6) can be produced by reacting compound (4) with carboxylic acid (5) or a salt thereof or a reactive derivative thereof. The reaction may be performed in the same manner as in step A-1.

(step A-3)

Compound (7) can be produced by subjecting compound (6) to an intramolecular cyclization reaction. The reaction can be performed according to a production method of an oxadiazole ring known per se, or a method analogous thereto and, for example, a method using a dehydrating agent can be used. Examples of the dehydrating agent include diphosphorus pentoxide, phosphorus oxychloride, phosphorus pentachloride, phosgene, N,N'-dicyclohexylcarbodiimide, alumina, polyphosphoric acid, acetic anhydride, acetyl chloride, sodium dioxide, thionyl chloride, methanesulfonyl chloride, p-toluenesulfonyl chloride, trifluoroacetic anhydride or complexes of triphenylphosphine and halogenated hydrocarbons such as carbon tetrachloride, carbon tetrabromide and the like, and the like. The amount of the dehydrating agent to be used is not less than about 1.0 - 100 mol, per 1 mol of compound (6). This reaction is advantageously performed without solvent or using a solvent inert to the reaction. Such solvent is not particularly limited as long as the reaction proceeds and, for example, a solvent such as ethers, halogenated hydrocarbons, esters, ketones and the like or a mixed solvent thereof and the like are preferable. While the reaction time varies depending on the reagent and solvent to be used, it is generally 10 min - 30 hr, preferably 1 hr - 10 hr. The reaction temperature is generally 0 - 150˚C, preferably 0 - 100˚C.

(step A-4)

The compound of the present invention can be produced by subjecting compound (7) to an intramolecular cyclization reaction in the presence of a nitrogen source. Examples of the nitrogen source include ammonia, ammonia salts such as ammonia acetate, amides such as formamide. The amount of the nitrogen source to be used is not less than about 1.0 - 100 mol, per 1 mol of compound (7). This reaction is advantageously performed using a solvent inert to the reaction. Such solvent is not particularly limited as long as the reaction proceeds and, for example, a solvent such as ethers, aromatic hydrocarbons, saturated hydrocarbons, amides, halogenated hydrocarbons, sulfoxides, organic acids, inorganic acids, water and the like, or a mixed solvent thereof and the like are preferable. While the reaction time varies depending on the reagent and solvent to be used, it is generally 10 min - 72 hr, preferably 30 min - 24 hr. The reaction temperature is generally 0 - 250˚C, preferably 20 - 150˚C.

The compound of the present invention can be also produced by converting the leaving group $LG^1$ of compound (7) to an amino group, which is then subjected to an intramolecular cyclization reaction. The conversion method of the leaving group $LG^1$ to amino group can be performed according to a method known per se, or a method analogous thereto and, for example, a method which comprises substituting the leaving group $LG^1$ with phthalimide and deprotecting the phthalic acid, a method which comprises substituting the leaving group $LG^1$ with an azide group and reducing the azide group, and the like can be used. The intramolecular cyclization reaction is advantageously performed using a solvent inert to the reaction. Such solvent is not particularly limited as long as the reaction proceeds and, for example, a solvent such as ethers, aromatic hydrocarbons, saturated hydrocarbons, amides, halogenated hydrocarbons, sulfoxides, organic acids, inorganic acids, water and the like, or a mixed solvent thereof and the like are preferable. While the reaction time varies depending on the reagent and solvent to be used, it is generally 10 min - 72 hr, preferably 30 min - 24 hr. The reaction temperature is generally 0 - 250˚C, preferably 20 - 150˚C.

The conversion reaction of leaving group $LG^1$ to an amino group, an azido group, and a phthalimido group exemplified in step A-4 can also be applied to compound (5) and compound (6). In this case, the obtained compound is subjected to step A-2 - step A-3, and subjected to the intramolecular cyclization reaction exemplified in step A-4, whereby the compound of the present invention can be produced.

Compounds (2), (3), (4), (5), (6) and (7) may be commercially available products, or can also be produced according to a method known per se or a method analogous thereto. Compound (5) can also be produced according to the method described in Tetrahedron Letters, vol. 44, page 365 (2003), Tetrahedron, vol. 58, page 7663 (2002) and the like, or a method analogous thereto.

Production method B of the compound of the present invention

(reaction 02)

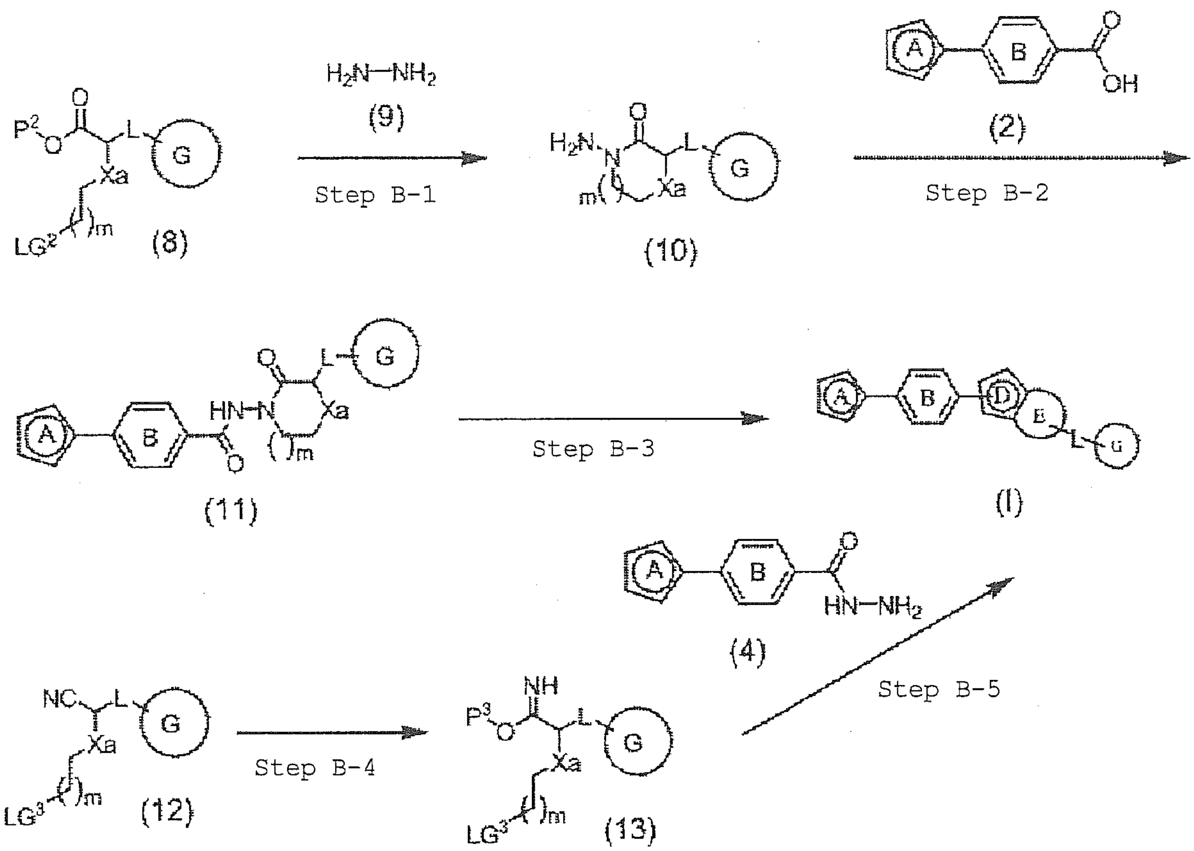


wherein each symbol is as defined above.
The compound of the present invention can be produced by performing a series of reaction steps of step B-1 to step B-3, or step B-4 to step B-5.

(step B-1)

Compound (10) can be produced by a condensation reaction of compound (8) and compound (9). Examples of compound (9) include hydrazine, hydrazinemonohydrate, hydrazine hydrochloride, hydrazine sulfate, hydrazine acetate, hydrazine carbonate and the like. Compound (9) is used in not less than about 0.8 mol per 1 mol of compound (8) and can also be used as a solvent. This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 100 hr, preferably 1 hr - 30 hr. The reaction temperature is generally -20 - 200˚C, preferably 0 - 100˚C.

(step B-2)

Compound (11) can be produced by reacting compound (10) and carboxylic acid (2), a salt thereof or a reactive derivative thereof. The reaction can be performed by a method similar to step A-1.

(step B-3)

The compound of the present invention can be produced by subjecting compound (11) to an intramolecular cyclization reaction in the presence of a nitrogen atom source. The reaction can be performed by a method similar to step A-4. In addition, to promote the reaction, compound (11) can also be subjected to a reaction with a dehydrating agent prior to the intramolecular cyclization reaction. The reaction with a dehydrating agent can be performed by a method similar to step A-3.

(step B-4)

Compound (13) can be produced by adding alcohols represented by $P^3OH$ to compound (12). Alcohols are used in not less than about 0.8 mol per 1 mol of compound (12) and can also be used as a solvent. In addition, to promote the reaction, the reaction can also be performed in the presence of an acid or base. Examples of the acid include acid chlorides such as acetyl chloride and the like, inorganic acids, organic acids, acid anhydrides, Lewis acid and the like. The amount of the acid to be used is about 0.01 - 100 mol, preferably about 0.1 - 20 mol, per 1 mol of compound (12). Examples of the base include inorganic bases, basic salts, organic bases, metal alkoxides, alkali metal hydrides, metal amides, organic lithiums and the like. The amount of the base to be used is about 0.01 - 100 mol, preferably about 0.1 - 20 mol, per 1 mol of compound (12). This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, sulfoxides, esters, ketones, aromatic organic bases, water and the like or a mixed solvent thereof and the like are preferable. While the reaction time varies depending on the reagent and solvent to be used, it is generally 10 min - 50 hr, preferably 30 min - 20 hr. The reaction temperature is generally 0 - 100˚C, preferably 0 - 50˚C.

(step B-5)

The compound of the present invention can be produced by subjecting compound (13) and compound (4) to a condensation reaction in the presence of a base. The amount of compound (13) to be used is about 0.2 - 5 mol, preferably about 0.5 - 2 mol, per 1 mol of compound (4). Examples of the base include inorganic bases, basic salts, organic bases, metal alkoxides, alkali metal hydrides and the like. The amount of the base to be used is about 0.01 - 100 mol, preferably about 0.1 - 20 mol, per 1 mol of compound (4). This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, sulfoxides, esters, ketones, aromatic organic bases, water and the like or a mixed solvent thereof and the like are preferable. While the reaction time varies depending on the reagent and solvent to be used, it is generally 10 min - 50 hr, preferably 30 min - 20 hr. The reaction temperature is generally -20 - 200˚C, preferably 0 - 100˚C.

Compounds (8), (9), (10), (11), (12) and (13) may be commercially available products, or can also be produced according to a method known per se or a method analogous thereto. In addition, compound (8) and compound (12) can be produced by a production method of compound (5) or a method analogous thereto, and known substituent conversion reaction, condensation reaction, oxidation reaction, reduction reaction and the like, conducted individually or by a combination of two or more thereof. These reactions can be carried out, for example, according to the method described in Shin Jikken Kagaku Koza (Courses in Experimental Chemistry), vols. 14 and 15 (edited by the Chemical Society of Japan); ORGANIC FUNCTIONAL GROUP PREPARATIONS, 2nd edition, Academic Press (ACADEMIC PRESS, INC.), 1989; Comprehensive Organic Transformations (VCH Publishers Inc.), 1989, and the like, or a method analogous thereto.

Production method of compound (2)

(reaction 03)

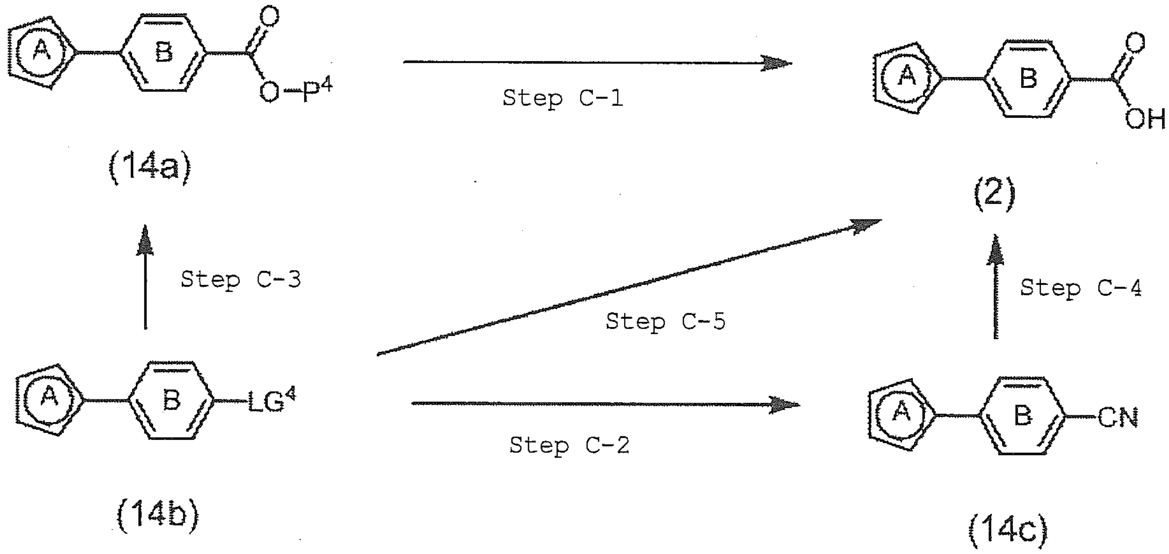


(14a) (2) (14b) (14c)

wherein each symbol is as defined above.

Compound (2) can be produced from compound (14a) according to step C-1, from compound (14c) according to step C-4, or from compound (14b) according to step C-5. Compound (14a) can be produced from compound (14b) according to step C-3, and compound (14c) can be produced from compound (14b) according to step C-2.

(step C-1)

Compound (2) can be produced by removing a protecting group of compound (14a). Removal of a protecting group can be performed according to a method known per se, for example, the method described in Wiley-Interscience Inc., 1999, "Protective Groups in Organic Synthesis, 3rd Ed." (Theodora W. Greene, Peter G. M. Wuts) and the like.

(step C-4)

Compound (2) can be produced by subjecting compound (14c) to hydrolysis. The reaction can also be performed in the presence of an acid or a base to promote the reaction. Examples of the acid include acid chlorides such as acetyl chloride and the like, inorganic acids, organic acids, Lewis acids and the like. Examples of the base include inorganic bases, basic salts, organic bases, metal alkoxides and the like. The amount of the acid to be used is about 0.01 - 100 mol, preferably about 0.1 - 20 mol, per 1 mol of compound (14c). The amount of the base to be used is about 0.01 - 100 mol, preferably about 0.1 - 20 mol, per 1 mol of compound (14c). This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, sulfoxides, esters, ketones, aromatic organic bases, water and the like or a mixed solvent thereof and the like are preferable. While the reaction time varies depending on the reagent and solvent to be used, it is generally 10 min - 50 hr, preferably 30 min - 20 hr. The reaction temperature is generally 0 - 200°C, preferably 0 - 140°C.

(step C-5)

Compound (2) can be produced by reacting compound (14b) with carbon dioxide in the presence of a base. The amount of the carbon dioxide to be used is not less than about 0.8 mol, per 1 mol of compound (14b), and the reaction can also be performed in a carbon dioxide stream. Dry ice can also be used as a carbon dioxide source. Examples of the base include alkali metal hydrides, metal amides, organic lithiums and the like. The amount of the base to be used is about 0.8 - 2 mol, preferably about 1.0 - 1.5 mol, per 1 mol of compound (14b). This reaction is advantageously performed using a solvent inert to the reaction. Such solvent is not particularly limited as long as the reaction proceeds and, for example, a solvent such as ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 100 hr, preferably 30 min - 24 hr. The reaction temperature is generally -100 - 100°C, preferably -78 - 50°C.

Compound (2) can also be produced by subjecting compound (14b) to a reaction with carbon monoxide in the presence of a metal catalyst and water or alcohols. The reaction may be performed by using not less than about 0.8 mol of carbon monoxide relative to 1 mol of compound (14b) in a carbon monoxide stream. Water or alcohols are used in not less than about 0.8 mol per 1 mol of compound (14b) and can also be used as a solvent. As the metal catalyst, a palladium compound [e.g., palladium acetate(II), tetrakis(triphenylphosphine)palladium(0), bis(triphenylphosphine) palladium(II) chloride, dichlorobis(triethylphosphine)palladium(0), tris(dibenzylideneacetone)dipalladium(0), complex of palladium acetate(II) and 1,1'-bis(diphenylphosphino)ferrocene, complex of palladium acetate(II) and 1,3-bis(diphenylphosphino)propane etc.] is preferable. The reaction is generally performed in the presence of a base. Examples of the base include inorganic bases, organic bases, basic salts and the like. The amount of the metal catalyst to be used is about 0.000001 - 5.0 mol, preferably about 0.0001 - 1.0 mol, per 1 mol of compound (14b). In addition, the amount of the base to be used is about 1.0 - 20 mol, preferably about 1.0 - 5.0 mol, per 1 mol of compound (14b). This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, esters, water and the like or a mixed solvent thereof and the like are preferable. While the reaction time varies depending on the reagent and solvent to be used, it is generally 1 min - 200 hr, preferably 5 min - 100 hr. The reaction temperature is -10 - 200˚C, preferably 0 - 100˚C. In addition, microwave may be irradiated to promote the reaction.

(step C-3)

Compound (14a) can be produced by reacting compound (14b) and alkyl chlorocarbonates in the presence of a base. Examples of the alkyl chlorocarbonates include methyl chlorocarbonate, ethyl chlorocarbonate and the like. The amount of the alkyl chlorocarbonates to be used is about 0.8 - 10 mol, preferably about 1.0 - 2 mol, per 1 mol of compound (14b). Examples of the base include alkali metal hydrides, metal amides, organic lithiums and the like can be mentioned. The amount of the base to be used is about 0.8 - 2 mol, preferably about 1.0 - 1.5 mol, per 1 mol of compound (14b). This reaction is advantageously performed using a solvent inert to the reaction. Such solvent is not particularly limited as long as the reaction proceeds and, for example, a solvent such as ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 100 hr, preferably 30 min - 24 hr. The reaction temperature is generally -100 - 100˚C, preferably -78 - 50˚C.
Compound (14a) can also be produced by subjecting compound (14b) to a reaction with carbon monoxide. The reaction can be performed by a method similar to step C-5.

(step C-2)

Compound (14c) can be produced by reacting compound (14b) and cyanide in the presence of a metal catalyst. Examples of the cyanide include sodium cyanide, potassium cyanide, zinc cyanide, potassium hexacyanoferrate (II) and the like. The amount of the cyanide to be used is about 0.8 - 10 mol, preferably about 1.0 - 5 mol, per 1 mol of compound (14b). As the metal catalyst, a metal complex having various ligands is used. Examples thereof include a palladium compound [e.g., palladium acetate(II), tetrakis(triphenylphosphine)palladium(0), bis(triphenylphosphine)palladium(II) chloride, dichlorobis(triethylphosphine)palladium(0), tris(dibenzylideneacetone)dipalladium(0), complex of palladium acetate(II) and 1,1'-bis(diphenylphosphino)ferrocene, complex of tris(dibenzylideneacetone)dipalladium(0) and 2-(di-tert-butylphosphino)biphenyl etc.], a nickel compound [e.g., tetrakis(triphenylphosphine)nickel (0), bis(triethylphosphine)nickel(II) chloride, bis(triphenylphosphine)nickel(II) chloride etc.], a copper compound [e.g., copper oxide, copper iodide(I), copper sulfate, copper chloride(II) etc.] and the like. The amount of the metal catalyst to be used is about 0.0001 - 5 mol, preferably about 0.001 - 1 mol, per 1 mol of compound (14b). This reaction is preferably performed in the presence of a base. Examples of the base include inorganic bases, organic bases, metal alkoxides, alkali metal hydrides, metal amides and the like. The amount of the base to be used is about 1.0 - 20 mol, preferably about 1.0 - 5.0 mol, per 1 mol of compound (14b). In addition, zinc may be used as an additive in this reaction. The amount of the zinc to be used is about 0.0001 - 5 mol, preferably about 0.001 - 1 mol, per 1 mol of compound (14b). When a metal catalyst unstable to oxygen is used in this reaction, for example, the reaction is preferably performed in an inactive gas stream such as argon gas, nitrogen gas and the like. This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, nitriles, sulfoxides, esters, water and the like or a mixed solvent thereof and the like are preferable. While the reaction time varies depending on the reagent and solvent to be used, it is generally 10 min - 100 hr, preferably 30 min - 50 hr. The reaction temperature is -10 - 250˚C, preferably 50 - 150˚C. In addition, microwave may be irradiated to promote the reaction.
Compounds (14a), (14b), and (14c) may be commercially available products, or can also be produced according

to a method known per se or a method analogous thereto.

Production method etc. of compound (14) and, for example, compounds (14d), (14e) and (14f) and the like from compound (14)

Compound (14d) is a compound (14) wherein ring A is an oxazole ring optionally having substituent(s):

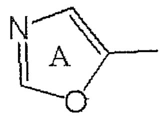
,

compound (14e) is a compound (14) wherein ring A is a triazole ring optionally having substituent(s):

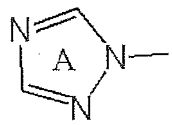
,

compound (14f) is a compound (14) wherein ring A is an imidazole ring optionally having substituent(s):

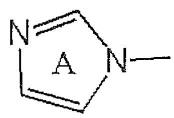
.

(reaction 04)

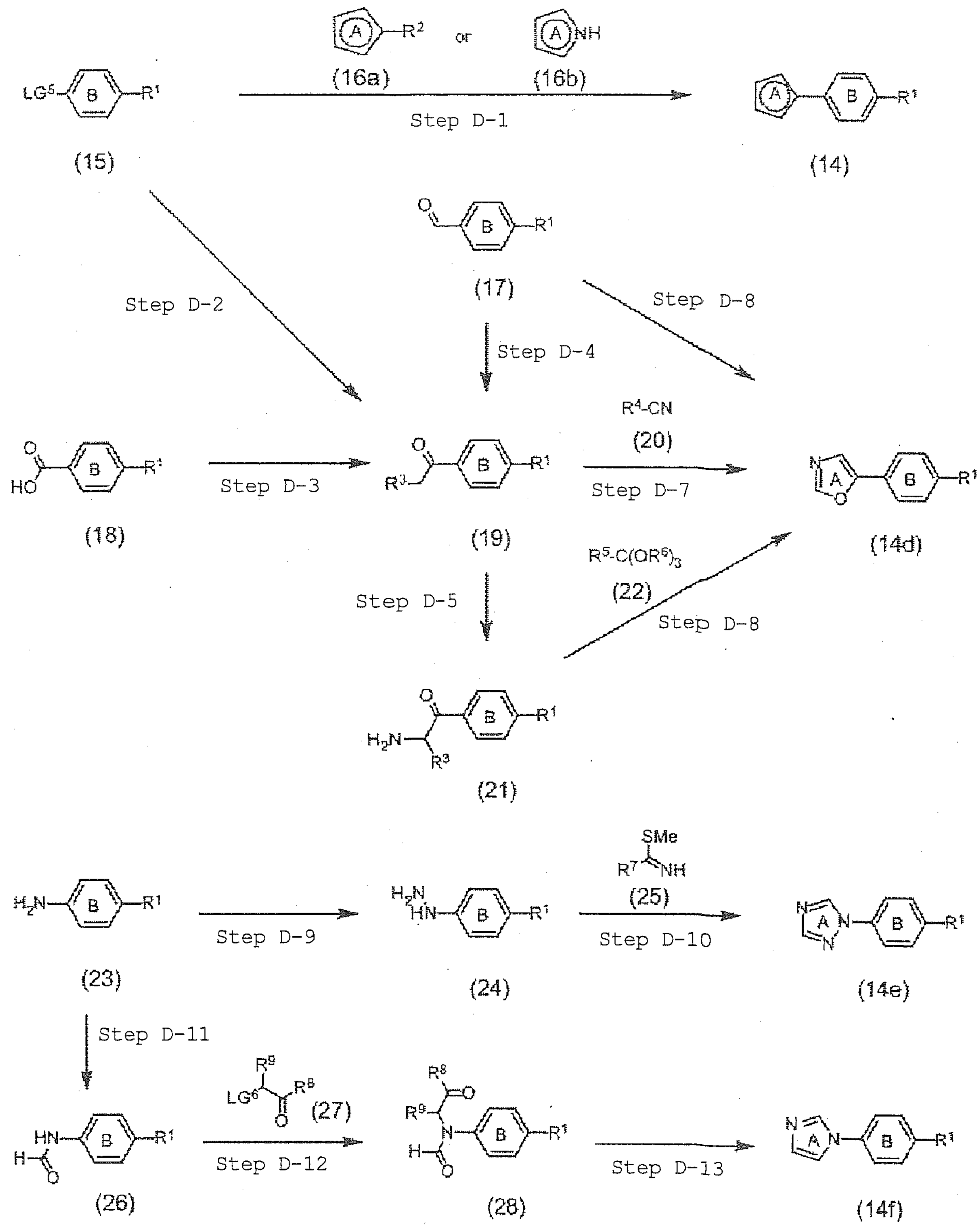


wherein -$R^1$ is a substituent such as -Coop$^4$, -$LG^4$, -CN and the like; $R^2$ is a boron atom moiety of organic boronic acid or organic boronic acid ester, a tri-$C_{1-6}$ alkylstanyl group, a hydrogen atom and the like; $R^3$ - $R^9$ are $C_{1-6}$ alkyl group or hydrogen atom, each of which optionally has substituent(s); other symbols are as defined above.

(step D-1)

Compound (14) can be produced by condensation of compound (15) and compound (16a). In the formula, $R^2$

is a boron atom moiety of organic boronic acid or organic boronic acid ester, a tri-$C_{1-6}$ alkylstanyl group, a hydrogen atom and the like. As the organic boronic acid or organic boronic acid ester, dihydroxyboranyl group, 4,4,5,5-tetramethyl-1,3,2-dioxaborolan-2-yl group and the like are preferable, and as the tri-$C_{1-6}$ alkylstanyl group, tributylstanyl group and the like are preferable. The condensation reaction is performed by reacting compound (15) and compound (16a) in the presence of a metal catalyst. As the metal catalyst, a palladium compound [e.g., palladium acetate(II), tetrakis(triphenylphosphine)palladium(0), bis(triphenylphosphine)palladium(II) chloride, dichlorobis(triethylphosphine)palladium(0), tris(dibenzylideneacetone)dipalladium(0), complex of palladium acetate(II) and 1,1'-bis(diphenylphosphino)ferrocene etc.] is preferable. The reaction is generally performed in the presence of a base. Examples of the base include inorganic bases, basic salts and the like can be mentioned. The amount of the compound (16a) to be used is about 0.1 - 10 mol, preferably about 0.8 - 2.0 mol, per 1 mol of compound (15). The amount of the metal catalyst to be used is about 0.000001 - 5.0 mol, preferably about 0.0001 - 1.0 mol, per 1 mol of compound (15). In addition, he amount of the base to be used is about 1.0 - 20 mol, preferably about 1.0 - 5.0 mol, per 1 mol of compound (15). When a metal catalyst unstable to oxygen is used in these reactions, for example, the reaction is preferably performed in an inactive gas stream such as argon gas, nitrogen gas and the like. This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, esters, water and the like or a mixed solvent thereof and the like are preferable. While the reaction time varies depending on the reagent and solvent to be used, it is generally 1 min - 200 hr, preferably 5 min - 100 hr. The reaction temperature is -10 - 250°C, preferably 0 - 150°C. In addition, microwave may be irradiated to promote the reaction.

Compound (14) can also be produced by condensing compound (15) and compound (16b) in the presence of a metal catalyst. As the metal catalyst, a metal complex having various ligands is used. Examples thereof include a palladium compound [e.g., palladium acetate(II), tetrakis(triphenylphosphine)palladium(0), bis(triphenylphosphine)palladium(II) chloride, dichlorobis(triethylphosphine)palladium(0), tris(dibenzylideneacetone)dipalladium(0), complex of palladium acetate(II) and 1,1'-bis(diphenylphosphino)ferrocene, complex of tris(dibenzylideneacetone)dipalladium(0) and 2-dicyclohexylphosphino-2'-(N,N-dimethylamino)biphenyl(DavePhos), or 2-dicyclohexylphosphino-2',4',6'-triisopropylbiphenyl(XPhos) etc.], nickel compound [e.g., tetrakis(triphenylphosphine)nickel(0), bis(triethylphosphine)nickel (II) chloride, bis(triphenylphosphine)nickel(II) chloride etc.], rhodium compound [e.g., chlorotris(triphenylphosphine)rhodium (III) etc.], cobalt compound, copper compound [e.g., copper oxide, copper iodide(I), copper sulfate, copper chloride (II) etc.], platinum compound and the like. Among these, a palladium compound or a copper compound is preferable. The amount of compound (16b) to be used is about 0.8 - 10 mol, preferably about 1.0 - 3.0 mol, per 1 mol of compound (15). The amount of the metal catalyst to be used is about 0.0001 - 5 mol, preferably about 0.001 - 1 mol, per 1 mol of compound (15). This reaction is preferably performed in the presence of a base. Examples of the base include inorganic bases, organic bases, metal alkoxides, alkali metal hydrides, metal amides and the like can be mentioned. The amount of the base to be used is about 1.0 - 20 mol, preferably about 1.0 - 5.0 mol, per 1 mol of compound (15). When a metal catalyst unstable to oxygen is used in this reaction, for example, the reaction is preferably performed in an inactive gas stream such as argon gas, nitrogen gas and the like. This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, nitriles, sulfoxides, esters, water and the like or a mixed solvent thereof and the like are preferable. While the reaction time varies depending on the reagent and solvent to be used, it is generally 10 min - 100 hr, preferably 30 min - 50 hr. The reaction temperature is -10 - 250°C, preferably 50 - 150°C. In addition, microwave may be irradiated to promote the reaction.

Compound (14) can also be produced by condensation of compound (15) and compound (16b). The amount of compound (16b) to be used is about 1.0 - 20 mol, preferably about 1.0 - 5 mol, per 1 mol of compound (15). In addition, to promote the reaction, the reaction can also be performed in the presence of a base. Examples of the base include inorganic bases, basic salts, organic bases, metal alkoxides, alkali metal hydrides, metal amides, organic lithiums and the like. The amount of the base to be used is about 1.0 - 20 mol, preferably about 1.0 - 3.0 mol, per 1 mol of compound (15). This reaction is advantageously performed using a solvent inert to the reaction. Such solvent is not particularly limited as long as the reaction proceeds and, for example, a solvent such as alcohols, ethers, aromatic hydrocarbons, saturated hydrocarbons, amides, halogenated hydrocarbons, nitriles, sulfoxides, esters, ketones, aromatic organic bases, water and the like or a mixed solvent thereof and the like are preferable. While the reaction time varies depending on the reagent and solvent to be used, it is generally 10 min - 100 hr, preferably 30 min - 50 hr. The reaction temperature is generally 0 - 250°C, preferably 0 - 200°C. In addition, microwave may be irradiated to promote the reaction.

(step D-6)

Compound (14d) can be produced by subjecting compound (17) to a condensation reaction with 1-[(isocyanomethyl)sulfonyl]-4-methylbenzene in the presence of a base. Examples of the base include inorganic bases, basic salts, organic bases, metal alkoxides and the like. The amount of the base to be used is about 0.8 - 20 mol, preferably

about 1.0 - 5.0 mol, per 1 mol of compound (17). The amount of 1-[(isocyanomethyl)sulfonyl]-4-methylbenzen to be used is about 0.8 - 20 mol, preferably about 1.0 - 5.0 mol, per 1 mol of compound (17). This reaction is advantageously performed using a solvent inert to the reaction. Such solvent is not particularly limited as long as the reaction proceeds and for example, a solvent such as alcohols, ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 50 hr, preferably 1 hr - 24 hr. The reaction temperature is generally -20 - 200˚C, preferably 0 - 100˚C.

(step D-7)

Compound (14d) can also be produced by subjecting compound (19) and compound (20) to a condensation reaction in the presence of an oxidant and an acid. Examples of the oxidant include organic peracids such as perbenzoic acid, m-chloroperbenzoic acid (MCPBA), peracetic acid and the like, perchlorates such as lithium perchlorate, silver perchlorate, tetrabutyl ammonium perchlorate and the like, periodic acids such as iodobenzenediacetate, sodium periodate, Dess-Martin periodinane, o-iodooxybenzoic acid (IBX) and the like, manganese acids such as manganese dioxide, potassium permanganate and the like, leads such as tetraacetic acid lead and the like, chromates such as pyridinium chlorochromate, pyridinium dichlorochromate and the like, inorganic nitrogen compounds such as acyl nitrate, dinitrogen tetroxide and the like, halogen compounds such as halogen, N-bromosuccinimide (NBS), N-chlorosuccinimide (NCS) and the like, sulfuryl chloride, chloramine-T, oxygen, hydrogen peroxide and the like. The amount of the oxidant to be used is about 0.8 - 20 mol, preferably about 1.0 - 5.0 mol, per 1 mol of compound (19). Examples of the acid include inorganic acids, organic acids, Lewis acid and the like. The amount of the acid to be used is about 0.8 - 20 mol, preferably about 1.0 - 10 mol, per 1 mol of compound (19). Examples of compound (20) include $C_{1-6}$ alkylnitriles such as acetonitrile, propionitrile and the like, and the like. Compound (20) is used in not less than about 0.8 mol per 1 mol of compound (19) and can also be used as a solvent. The solvent is not particularly limited as long as the reaction proceeds and for example, a solvent such as ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 100 hr, preferably 30 min - 48 hr. The reaction temperature is generally -20 - 200˚C, preferably -10 - 100˚C.

(step D-8)

Compound (14d) can also be produced by subjecting compound (21) and compound (22) to a condensation reaction in the presence of an acid. Examples of the acid include inorganic acids, organic acids, Lewis acid and the like. The amount of the acid to be used is about 0.001 - 10 mol, preferably about 0.1 - 2.0 mol, per 1 mol of compound (21). Examples of the compound (22) include orthoacid esters such as trimethyl orthoacetate, triethyl orthopropionate, trimethyl orthoformate and the like, and the like. Compound (22) is used in not less than about 0.8 mol per 1 mol of compound (21) and can also be used as a solvent. This reaction is advantageously performed using a solvent inert to the reaction. Such solvent is not particularly limited as long as the reaction proceeds and for example, a solvent such as alcohols, ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 50 hr, preferably 1 hr - 24 hr. The reaction temperature is generally -20 - 200˚C, preferably 0 - 100˚C.

Compound (14d) can also be produced according to a method known per se, for example, the method described in Bioorganic & Medicinal Chemistry Letters, vol. 13, page 2059 (2003) and the like, or a method analogous thereto.

Compound (19) can be produced from compound (15) according to step D-2, from compound (17) according to step D-4, or from compound (18) according to step D-3. Compound (21) can be produced from compound (19) according to step D-5.

(step D-2)

Examples of the step D-2 include a method comprising subjecting compound (15), tributyl(ethoxyvinyl)tin and the like to a reaction similar to the method of producing compound (14) from compound (15) and compound (16a) and the like.

(step D-4)

As step D-4, for example, a method which comprises adding a Grignard reagent represented by $R^3CH_2MgBr$ and the like to an aldehyde group, which is then subjected to an oxidation reaction, and the like can be used.

(step D-3, step D-5)

As step D-3, for example, a method which comprises converting a carboxyl group to a Weinreb amide, which is then subjected to a reaction with a Grignard reagent represented by $R^3CH_2MgBr$ etc., and the like can be used. Examples of the step D-5 include a method which comprises reacting ketone with a halogenating agent to give α-haloketone, and subjecting same to a reaction with an aminating agent and the like. These reaction can be performed according to, for example, a method described in Shin Jikken Kagaku Koza (Courses in Experimental Chemistry), vols. 14, 15 (The Chemical Society of Japan ed.); ORGANIC FUNCTIONAL GROUP PREPARATIONS, 2nd edition, Academic Press (ACADEMIC PRESS, INC.), 1989; Comprehensive Organic Transformations (VCH Publishers Inc.), 1989 and the like.

(step D-10)

Compound (14e) can be produced by condensing compound (24) and compound (25) and subjecting the compound to a condensation reaction with orthoacid esters. Examples of compound (25) include alkyl imidothioates such as methyl ethanimidothioate hydroiodide, methyl propanimidothioate hydrochloride and the like, and compound (25) can be produced according to a method known per se, for example, the method described in Indian Journal of Chemistry, Section B: Organic Chemistry Including Medicinal Chemistry, vol. 21, page 272 (1982) and the like, or a method analogous thereto. The amount of compound (25) to be used is about 0.8 - 10 mol, preferably about 1.0 - 5 mol, per 1 mol of compound (24). Examples of the orthoacid esters include trimethyl orthoacetate, triethyl orthopropionate, trimethyl orthoformate and the like. The orthoacid esters are used in not less than about 0.8 mol per 1 mol of compound (24) and can also be used as a solvent. While a solvent for the condensation reaction with compound (24) and compound (25) is not particularly limited as long as the reaction proceeds, for example, a solvent such as alcohols, ethers, aromatic hydrocarbons, saturated hydrocarbons, amides, halogenated hydrocarbons, nitriles, sulfoxides and the like, or a mixed solvent thereof and the like are preferable. While the reaction time varies depending on the reagent and solvent to be used, it is generally 5 min - 100 hr, preferably 10 min - 24 hr. The reaction temperature is generally -20 - 200˚C, preferably -10 - 100˚C. While a solvent for the condensation reaction with orthoacid ester is not particularly limited as long as the reaction proceeds, for example, a solvent such as alcohols, ethers, aromatic hydrocarbons, saturated hydrocarbons, amides, halogenated hydrocarbons, nitriles, sulfoxides, aromatic organic bases, organic bases and the like, or a mixed solvent thereof and the like are preferable. While the reaction time varies depending on the reagent and solvent to be used, it is generally 10 min - 200 hr, preferably 10 min - 48 hr. The reaction temperature is generally -20 - 200˚C, preferably -10 - 150˚C.

(step D-9)

Compound (24) can be produced by reacting compound (23) with nitrous acids in the presence of an acid, and subjecting the compound to a reduction reaction. Examples of the acid include inorganic acids, organic acids, Lewis acid and the like. The acid is used in not less than about 0.01 mol per 1 mol of compound (23) and can also be used as a solvent. Examples of the nitrous acids include nitrite salts such as sodium nitrite, potassium nitrite and the like, nitrous acid esters such as isoamyl nitrite and the like, and the like. The amount of the nitrous acids to be used is about 0.8 - 10 mol, preferably about 1.0 - 5 mol, per 1 mol of compound (23). Examples of the reducing agent include reducing agents such as tin chloride and the like, and the like. The amount of the reducing agent to be used is about 0.8 - 20 mol, preferably about 1.0 - 10 mol, per 1 mol of compound (23). The solvent in the reaction with nitrite is not particularly limited as long as the reaction proceeds and for example, a solvent such as inorganic acids, organic acids, alcohols, ethers, amides, nitriles, sulfoxides and the like or a mixed solvent thereof and the like are preferable. While the reaction time varies depending on the reagent and solvent to be used, it is generally 5 min - 100 hr, preferably 10 min - 24 hr. The reaction temperature is generally -30 - 100˚C, preferably -20 - 80˚C. The solvent in the reduction reaction is not particularly limited as long as the reaction proceeds and for example, a solvent such as inorganic acids, organic acids, alcohols, ethers, amides, nitriles, sulfoxides and the like or a mixed solvent thereof and the like are preferable. While the reaction time varies depending on the reagent and solvent to be used, it is generally 5 min - 100 hr, preferably 10 min - 24 hr. The reaction temperature is generally -30 - 100˚C, preferably -20 - 80˚C.

Compound (14f) can also be produced by performing a series of reaction steps of from compound (23) to step D-11, step D-12, and step D-13.

(step D-11)

Compound (26) can be produced by reacting compound (23) with a formylating agent. Examples of the formylating agent include formic acid esters such as N,N-dimethylformamide, N-formylpiperidine, N-formylmorpholine, ethyl

formate and the like, and the like. The amount of the formylating agent to be used is about 1.0 - 100 mol, preferably about 1.0 - 30 mol, per 1 mol of compound (23). This reaction is advantageously performed using a solvent inert to the reaction. Such solvent is not particularly limited as long as the reaction proceeds and for example, a solvent such as ethers, halogenated hydrocarbons, aromatic hydrocarbons, saturated hydrocarbons and the like or a mixed solvent thereof and the like are preferable. While the reaction time varies depending on the reagent and solvent to be used, it is generally 30 min - 50 hr, preferably 30 min - 24 hr. The reaction temperature is generally 0 - 200˚C, preferably 0 - 150˚C.

(step D-12)

Compound (28) can be produced by reacting compound (26) with an alkylating agent (27) in the presence of a base. Examples of the base include inorganic bases, basic salts, organic bases, metal amides and the like. The amount of the base to be used is about 1.0 - 5.0 mol, preferably about 1.0 - 2.0 mol, per 1 mol of compound (26). For example, sodium iodide, potassium iodide and the like can be preferably added to promote the reaction. This reaction is advantageously performed using a solvent inert to the reaction. Such solvent is not particularly limited as long as the reaction proceeds and for example, a solvent such as ethers, aromatic hydrocarbons, saturated hydrocarbons, amides, halogenated hydrocarbons, nitriles, sulfoxides and the like or a mixed solvent thereof and the like are preferable. While the reaction time varies depending on the reagent and solvent to be used, it is generally 10 min - 100 hr, preferably 30 min - 24 hr. The reaction temperature is generally -20 - 200˚C, preferably -10 - 150˚C.

(step D-13)

Compound (14f) can be produced by a heat treatment of compound (28) in the presence of ammonium acetate in a solvent of acetic acid. The amount of the ammonium acetate to be used is about 3.0 - 50 mol, preferably about 5.0 - 30 mol, per 1 mol of compound (28). The reaction time is generally 10 min - 100 hr, preferably 30 min - 24 hr. The reaction temperature is generally 0 - 100˚C, preferably 50 - 100˚C.

Compound (14) can also be produced according to a method known per se, for example, the method described in European journal of organic chemistry, vol. 13, p. 2970 (2006), Synthetic communications, vol. 36, page 2927 (2006), Journal of organic chemistry, vol. 44, page 4160 (1979), Journal of the chemical society, page 4251 (1954), WO 2008/77649 and the like, or a method analogous thereto.

Compounds (14), (14d), (14e) and (14f) can be further converted to a desired compound by a known substituent conversion reaction, condensation reaction, oxidation reaction, reduction reaction and the like, conducted individually or by a combination of two or more thereof. These reactions can be carried out, for example, according to the method described in Shin Jikken Kagaku Koza (Courses in Experimental Chemistry), vols. 14 and 15 (edited by the Chemical Society of Japan); ORGANIC FUNCTIONAL GROUP PREPARATIONS, 2nd edition, Academic Press (ACADEMIC PRESS, INC.), 1989; Comprehensive Organic Transformations (VCH Publishers Inc.), 1989, and the like or a method analogous thereto. For example, when ring A has one or two halogen atoms, a case can be mentioned that one or two of the halogen atom(s) can be converted to a $C_{1-6}$ alkyloxy group by reacting with a $C_{1-6}$ alkyloxide according to a method known per se, or a method analogous thereto.

Compounds (15), (16a), (16b), (17), (18), (19), (20), (21), (22), (23), (24), (25), (26), (27) and (28) may be commercially available products, or can also be produced according to a method known per se or a method analogous thereto.

Production method C of the compound of the present invention

(reaction 05)

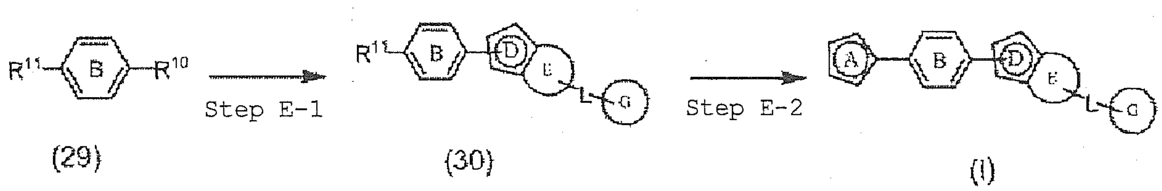


(29) (30) (I)

wherein $R^{10}$ is a substituent such as halogen, carboxyl group, ester group, cyano group, hydrazide and the like; $R^{11}$ is a substituent such as halogen, carboxyl group, aldehyde group, amino group, $C_{1-6}$ alkylcarbonyl group and the like; and other symbols are as defined above.

The compound of the present invention can also be produced by performing a series of reaction steps of from compound (29) to step E-1 and step E-2.

(step E-1)

This reaction step can be performed by the reaction steps shown in step A-1 to A-4, step B-2 to B-3, step B-5, and step C-1 to C-5 and the like, conducted individually or by a combination of two or more thereof.

(step E-2)

This reaction step can be performed by the reaction steps shown in step D-1 to D-13 and the like, conducted individually or by a combination of two or more thereof.

Compounds (29) and (30) may be commercially available products, or can also be produced by a method known per se or a method analogous thereto.

The compound of the present invention can be produced as any one configuration isomer or stereoisomer, or a mixture thereof. These isomers can be obtained as single products according to synthesis method, separation method (e.g., concentration, solvent extraction, column chromatography, recrystallization etc.), optical resolution method (e.g., fractional recrystallization, chiral column method, diastereomer method etc.) and the like known per se. They can also be converted to a desired isomer by heating, an acid catalyst, a transition metal complex, a metal catalyst, a radical catalyst, photoirradiation, a strong base catalyst and the like according to the method described in Shin Jikken Kagaku Koza (New Experimental Chemistry Course), vol. 14, pp. 251-253 (edited by the Chemical Society of Japan), Jikken Kagaku Kouza, 4th Ed. vol. 19, pp.273-274 (edited by the Chemical Society of Japan) and the like or a method analogous thereto.

Among the aforementioned compounds (2) - (30), those having a configurational isomer can be isolated and purified by, for example, a conventional separation means such as extraction, recrystallization, distillation, chromatography and the like, when isomerization occurs, whereby a pure compound can be produced. In addition, isomerization of double bond may be promoted by heating, acid catalyst, transition metal complex, metal catalyst, radical species catalyst, photoirradiation or strong base catalyst and the like according to the method described in Shin Jikken Kagaku Koza (New Experimental Chemistry Course), vol. 14, pp. 251-253 (edited by the Chemical Society of Japan), Jikken Kagaku Koza (Courses in Experimental Chemistry), 4th Ed., vol. 19, pp. 273-274 (edited by the Chemical Society of Japan) and the like or a method analogous thereto, whereby a corresponding pure isomer can be obtained. While the compound of the present invention has a stereoisomer depending on the kind of the substituent, not only the isomer itself but also a mixture thereof are encompassed in the present invention. In the above-mentioned reaction steps, where desired, the compound of the present invention can be produced by a known hydrolysis reaction, deprotection reaction, acylation reaction, alkylation reaction, hydrogenation reaction, oxidation reaction, reduction reaction, carbon chain extension reaction or substituent exchange reaction, condensation reaction and the like, conducted individually or by a combination of two or more thereof. These reactions can be carried out, for example, according to the method described in Shin Jikken Kagaku Koza (New Experimental Chemistry Course), vols. 14 and 15 (edited by the Chemical Society of Japan); ORGANIC FUNCTIONAL GROUP PREPARATIONS, 2nd edition, Academic Press (ACADEMIC PRESS, INC.), 1989; Comprehensive Organic Transformations (VCH Publishers Inc.), 1989, and the like.
The compound of the present invention can be isolated and purified by a known means, for example, phase transfer, concentration, solvent extraction, fractional distillation, liquid conversion, crystallization, recrystallization, chromatography and the like.

When the compound of the present invention is obtained as a free compound, it can be converted into a desired salt by a method known per se or a modification thereof; conversely, when compound (I) is obtained as a salt, it can be converted into a free form or another desired salt by a method known per se or a modification thereof.
When the compound of the present invention has an isomer such as optical isomer, stereoisomer, positional isomer, rotational isomer and the like, any one isomer and a mixture thereof are also encompassed in the compound of the present invention. For example, when an optical isomer is present in the compound of the present invention, an optical isomer resolved from a racemate is also encompassed in the compound of the present invention. These isomers can be obtained as single products by synthesis method and separation method (e.g., concentration, solvent extraction, column chromatography, recrystallization etc.), optical resolution method (e.g., fractional recrystallization, chiral column method, diastereomer method etc.) and the like known per se.
The compound of the present invention may be a crystal, and both single crystal form and a crystalline mixture are encompassed in the compound of the present invention. The crystal can be produced by crystallization by a crystallization method known per se. The compound of the present invention may be a pharmaceutically acceptable cocrystal or cocrystal salt. Here, the cocrystal and cocrystal salt mean crystalline substances consisting of two or more kinds of distinctive solids at room temperature, each having different physical properties (e.g., structure, melting point, melting

heat, hygroscopicity, dissolution property and stability etc.). The cocrystal and cocrystal salt can be produced by a cocrystallization method known per se.
The compound of the present invention may be a solvate (e.g., hydrate etc.) or a non-solvate (e.g., non-hydrate etc.), all of which are also encompassed in the compound of the present invention.
A compound labeled with an isotope (e.g., $^{3}H$, $^{14}C$, $^{35}S$, $^{125}I$ etc.) etc. and a deuterium converter are also encompassed in the compound of the present invention.
A prodrug of the compound of the present invention means a compound which is converted to the compound of the present invention with a reaction due to an enzyme, gastric acid, etc. under the physiological condition in the living body, that is, a compound which is converted to the compound of the present invention by oxidation, reduction, hydrolysis, etc. according to an enzyme; and a compound which is converted to the compound of the present invention by hydrolysis etc. due to gastric acid, etc.
A prodrug of the compound of the present invention may be a compound obtained by subjecting an amino group in the compound of the present invention to an acylation, alkylation or phosphorylation (e.g., a compound obtained by subjecting an amino group in the compound of the present invention to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxyl group in the compound of the present invention to an acylation, alkylation, phosphorylation or boration (e.g., a compound obtained by subjecting a hydroxyl group in the compound of the present invention to an acetylation, palmitoylation, propanoylation, pivaloylation, succinylation, fumarylation, alanylation or dimethylaminomethylcarbonylation, etc.); a compound obtained by subjecting a carboxy group in the compound of the present invention to an esterification or amidation (e.g., a compound obtained by subjecting a carboxy group in the compound of the present invention to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, etc.) and the like. Any of these compounds can be produced from the compound of the present invention by a method known per se.
A prodrug of the compound of the present invention may also be one which is converted into the compound of the present invention under a physiological condition, such as those described in IYAKUHIN no KAIHATSU (Development of Pharmaceuticals), Vol. 7 (Design of Molecules), p. 163-198 (HIROKAWA SHOTEN).
The compound of the present invention or a prodrug thereof has a superior amyloid β production inhibitory activity, shows low toxicity (e.g., acute toxicity, chronic toxicity, genetic toxicity, reproductive toxicity, cardiotoxicity, drug interaction, carcinogenicity etc.) and shows superior stability and disposition (absorbability, distribution, metabolism, excretion etc.), and therefore, is useful as a pharmaceutical product. Since the compound of the present invention or a prodrug thereof has an action to inhibit amyloid β production in a mammal (e.g., mouse, rat, hamster, rabbit, cat, dog, bovine, sheep, monkey, human etc.), it can be used as a prophylactic or therapeutic drug for diseases possibly related to amyloid β production. Examples of the "diseases possibly related to amyloid β production" include neurodegenerative diseases (e.g., senile dementia, Alzheimer's disease, Parkinson's disease etc.), memory disorders (e.g., senile dementia, mild cognitive impairment (MCI), amnesia etc.), ischemic central nervous disorders (e.g., cerebral amyloid angiopathy (CAA) etc.), Down's disease and the like.
The compound of the present invention or a prodrug thereof is preferably useful as an amyloid β production inhibitor, or a prophylactic or therapeutic drug for mild cognitive impairment or Alzheimer's disease.
A medicament containing the compound of the present invention or a prodrug thereof (hereinafter to be referred to as the "medicament of the present invention") is obtained as, for example, tablet (including sugar-coated tablet, film-coated tablet, sublingual tablet, orally disintegrating tablet, buccal tablet and the like), pill, powder, granule, capsule (including soft capsule, microcapsule), troche, syrup, liquid, emulsion, suspension, controlled-release preparation (e.g., immediate-release preparation, sustained-release preparation, sustained-release microcapsule), aerosol, films (e.g., orally disintegrable films, oral cavity mucosa patch film), injection (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparations, pulmonary preparation (inhalant), eye drop and the like by using the compound of the present invention or a prodrug thereof alone or along with a pharmacologically acceptable carrier according to a method known per se as a production method of pharmaceutical preparations (e.g., the method described in the Japanese Pharmacopoeia etc.). It can be safely administered orally or parenterally (e.g., intravenous, intramuscular, subcutaneous, intraorgan, intranasal, intradermal, instillation, intracerebral, rectal, vaginal, intraperitoneal, intratumor, tumor proximal administration, administration to a lesion and the like).
The content of the compound of the present invention or a prodrug thereof in the medicament of the present invention is about 0.01 - 100 wt% of the whole medicament. While the dose of the medicament of the present invention varies depending on the subject of administration, administration route, disease, symptom and the like, it is, for example, about 0.001 - about 100 mg/kg body weight, preferably about 0.005 - about 50 mg/kg body weight, more preferably about 0.01

- about 2 mg/kg body weight as the amount of the compound of the present invention or a prodrug thereof, which is the active ingredient, for the treatment of, for example, Alzheimer's disease by oral administration to an adult patient. This amount is desirably administered in about 1 to 3 portions a day according to the symptom.

Examples of the pharmacologically acceptable carrier include various organic or inorganic carrier substances conventionally used as preparation materials. For example, excipient, lubricant, binder and disintegrant for solid preparations, or solvent, solubilizing agent, suspending agent, isotonicity agent, buffer and soothing agent for liquid preparations and the like, are exemplified. Where necessary, conventional preservative, antioxidizing agent, colorant, sweetening agent, adsorbent, wetting agent and the like can be used appropriately in an appropriate amount

Examples of the excipient include lactose, sucrose, D-mannitol, starch, cornstarch, crystalline cellulose, light anhydrous silicic acid and the like. Examples of the lubricant include magnesium stearate, calcium stearate, talc, colloidal silica and the like. Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose and the like. Examples of the disintegrant include starch, carboxymethylcellulose, calcium carboxymethylcellulose, croscarmellose sodium, sodium carboxymethyl starch, L-hydroxypropylcellulose and the like. Examples of the solvent include water for injection, alcohol, propylene glycol, macrogol, sesame oil, corn oil, olive oil and the like. Examples of the solubilizing agents include polyethylene glycol, propylene glycol, D-mannitol, benzyl benzoate, ethanol, trisaminomethane, cholesterol, triethanolamine, sodium carbonate, sodium citrate and the like. Examples of the suspending agent include surfactants such as stearyltriethanolamine, sodium lauryl sulfate, lauryl aminopropionate, lecithin, benzalkonium chloride, benzethonium chloride, glyceryl monostearate etc.; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose etc., and the like. Examples of the isotonicity agent include glucose, D-sorbitol, sodium chloride, glycerol, D-mannitol and the like. Examples of the buffer include buffer such as phosphate, acetate, carbonate, citrate etc., and the like. Examples of the soothing agent include benzyl alcohol and the like. Examples of the preservative include p-hydroxybenzoates, chlorobutanol, benzyl alcohol, phenethyl alcohol, dehydroacetic acid, sorbic acid and the like. Examples of the antioxidizing agent include sulfite, ascorbic acid, α-tocopherol and the like.

When the compound of the present invention or a prodrug thereof is applied to each of the above-mentioned diseases, it can be used in appropriate combination with a medicament or a treatment method generally employed for the disease.

In the following, a combined use of the compound of the present invention or a prodrug thereof with a concomitant drug is referred to as "the combination agent of the present invention".

Examples of such concomitant drug include acetylcholinesterase inhibitors (e.g., donepezil, rivastigmine, galanthamine etc.), inhibitors of amyloid β protein production, secretion, accumulation, coagulation and/or deposition, β-secretase inhibitors, amyloid β protein coagulation inhibitors, amyloid β vaccine, amyloid β antibody, amyloid β degrading enzyme etc., brain function activation drugs (e.g., idebenone, memantine, vinpocetine etc.), therapeutic drugs for abnormal behavior, wandering and the like which are developed with the progression of dementia (e.g., sedative, antianxiety agent etc.), drugs for suppression of progression of Alzheimer's disease (Alzhemed etc.), apoptosis inhibitors, neuronal differentiation regeneration promoters, anti-parkinsonian drugs (e.g., L-DOPA, deprenyl, carbidopa+levodopa, pergolide, ropinirole, cabergoline, pramipexole, entacapone, lazabemide etc.), therapeutic agents for amyotrophic lateral sclerosis (e.g., riluzole etc., neurotrophic factor etc.), antidepressants (e.g., fluoxetine, sertraline, paroxetine, venlafaxine, nefazodone, reboxetine, mirtazapine, imipramine hydrochloride, duloxetine, escitalopram, mifepristone, doxepin etc.), antianxiety drugs (e.g., alprazolam, bromazepam, chlordiazepoxide, diazepam, etizolam, flutoprazepam, lorazepam etc.), antiepileptic drugs (e.g., lamotrigine etc.), sleep inducing agents (e.g., GABA system sleep inducing agents such as brotizolam, estazolam, flurazepam, nitrazepam, triazolam, flunitrazepam, lormetazepam, rilmazafone, quazepam, zopiclone, eszopiclone, zolpidem, zaleplon, indiplon, gabaxadol etc.; non-GABA system sleep inducing agents such as eplivaserin, pruvanserin, diphenhydramine, trazodone, doxepin etc., ramelteon etc.), therapeutic agents for narcolepsy, therapeutic agents for schizophrenia (e.g., olanzapine, risperidone, quetiapine, iloperidone, etc.), anti-obesity drugs, non-steroidal anti-inflammatory drugs (e.g., indomethacin, ibuprofen, acetylsalicylic acid, diclofenac, naproxen, piroxicam etc.), COX-2 inhibitors (e.g., celecoxib, rofecoxib etc.), cerebral circulation and metabolism improvement drugs (e.g., nicergoline, ibudilast, ifenprodil etc.), disease-modified anti-rheumatic drugs (DMARDs), anti-cytokine drugs (TNF inhibitor, MAP kinase inhibitor etc.), steroid drugs (e.g., dexamethasone, hexestrol, cortisone acetate etc.), therapeutic agents for incontinence·frequent urination (e.g., flavoxate hydrochloride, oxybutynin hydrochloride, propiverine hydrochloride etc.), therapeutic drugs for osteoporosis, hypolipidemic agents (e.g., simvastatin, fluvastatin, pravastatin, atorvastatin, etc.), antihypertensive agents (e.g., captopril, delapril, enalapril, nifedipine, nicardipine, amlodipine, alprenolol, propranolol, metoprolol, losartan, valsartan, candesartan, etc.), therapeutic agents for diabetes (e.g., pioglitazone, rosiglitazone, metformin, glibenclamide, nateglinide, voglibose, etc.), antiplatelet agents (e.g., ticlopidine, heparin, urokinase, alteplase, tisokinase, nasaruplase, cilostazol, etc.), antioxidizing agents (e.g., linolenic acid, ascorbic acid, icosapentaenoic acid, docosahexaenoic acid, tocopherol, etc.), vitamins (e.g., tocopherol, ascorbic acid, etc.), sex hormones (e.g., estrogen, estrone, estradiol, etc.), anticonvulsants (e.g., carbamazepine, valproic acid, clonazepam, vigabatrin,

lamotrigine, gabapentin, etc.) and the like.

By combining the compound of the present invention or a prodrug thereof and a concomitant drug, a superior effect such as

(1) the dose can be reduced as compared to single administration of the compound of the present invention or a prodrug thereof, or a concomitant drug,
(2) the concomitant drug can be selected according to the condition of patients (mild case, severe case and the like),
(3) the period of treatment can be set longer by selecting a concomitant drug having different action mechanism from the compound of the present invention or a prodrug thereof,
(4) a sustained treatment effect can be designed by selecting a concomitant drug having different action mechanism from the compound of the present invention or a prodrug thereof,
(5) a synergistic effect can be afforded by a combined use of the compound of the present invention or a prodrug thereof, and a concomitant drug, and the like, can be achieved.

The combination agent of the present invention has low toxicity, and for example, the compound of the present invention or a prodrug thereof, and/or the above-mentioned concomitant drug can be mixed, according to a method known per se, with a pharmacologically acceptable carrier to give pharmaceutical compositions, such as tablets (including sugar-coated tablet, film-coated tablet), powders, granules, capsules, solutions, emulsions, suspensions, injections, suppositories, sustained release preparations (e.g., sublingual tablet, microcapsule etc.), plasters, orally disintegrating tablets, orally disintegrating films and the like, which can be safely administered orally or parenterally (e.g., subcutaneous, topical, rectal, intravenous administrations etc.).
Examples of the pharmacologically acceptable carriers usable for the production of the combination agent of the present invention include various organic or inorganic carrier substances conventionally used as preparation materials can be mentioned. For example, excipient, lubricant, binder and disintegrant for solid preparations, or solvent, solubilizing agent, suspending agent, isotonicity agent, buffer and soothing agent for liquid preparations and the like, are exemplified. Where necessary, conventional preservative, antioxidizing agent, colorant, sweetening agent, adsorbent, wetting agent and the like can be used appropriately in appropriate amount.

When using the combination agent of the present invention, the administration time of the compound of the present invention or a prodrug thereof, and the concomitant drug is not restricted, and the compound of the present invention or a prodrug thereof or a pharmaceutical composition thereof and the concomitant drug or a pharmaceutical composition thereof can be administered to an administration subject simultaneously, or may be administered at different times. The dosage of the concomitant drug may be determined according to the dose clinically used, and can be appropriately selected depending on an administration subject, administration route, disease, combination and the like.

The administration mode of the combination agent of the present invention is not particularly restricted, and it is sufficient that the compound of the present invention and the concomitant drug are combined in administration. Examples of such administration mode include the following:

(1) administration of a single preparation obtained by simultaneously processing the compound of the present invention or a prodrug thereof and the concomitant drug, (2) simultaneous administration of two kinds of preparations of the compound of the present invention or a prodrug thereof and the concomitant drug, which have been separately produced, by the same administration route, (3) administration of two kinds of preparations of the compound of the present invention or a prodrug thereof and the concomitant drug, which have been separately produced, by the same administration route in a staggered manner, (4) simultaneous administration of two kinds of preparations of the compound of the present invention or a prodrug thereof and the concomitant drug, which have been separately produced, by different administration routes, (5) administration of two kinds of preparations of the compound of the present invention or a prodrug thereof and the concomitant drug, which have been separately produced, by different administration routes in a staggered manner (e.g., administration in the order of the compound of the present invention or a prodrug thereof and the concomitant drug, or in the reverse order) and the like.

The compounding ratio of the compound of the present invention or a prodrug thereof to the concomitant drug in the combination agent of the present invention can be appropriately selected depending on an administration subject, administration route, diseases and the like.
For example, the content of the compound of the present invention or a prodrug thereof in the combination agent of the present invention varies depending on the form of a preparation, and usually about 0.01 to 100 wt%, preferably about 0.1 to 50 wt%, further preferably about 0.5 to 20 wt%, based on the whole preparation.
While the content of the concomitant drug in the combination agent of the present invention varies depending on the form of a preparation, it is usually about 0.01 to 100 wt%, preferably about 0.1 to 50 wt%, further preferably about 0.5 to 20 wt%, based on the whole preparation.

While the content of the additives such as carrier and the like in the combination agent of the present invention varies depending on the form of a preparation, it is generally about 1 to 99.99 wt%, preferably about 10 to 90 wt%, based on the whole preparation.
Similar contents can be employed for individual preparations of the compound of the present invention or a prodrug thereof and the concomitant drug.

[Examples]

While the present invention is explained in more detail in the following by referring to Reference Examples, Examples, Formulation Examples and Experimental Examples. However, the examples are mere examples and do not limit the present invention, and may be modified without departing from the scope of the invention. In the explanation of the following production methods, the compounds to be the starting materials and the reaction resultant products may form salts that do not prevent the reactions.
The symbols in the Examples mean the following.

| | |
|---|---|
| NMR | : nuclear magnetic resonance spectrum |
| s | : singlet |
| d | : doublet |
| t | : triplet |
| q | : quartet |
| quin | : quintet |
| dd | : double doublet |
| td | : triple doublet |
| dt | : double triplet |
| m | : multiplet |
| br | : broad |
| brs | : broad singlet |
| J | : coupling constant |
| THF | : tetrahydrofuran |
| MeOH | : methanol |
| DMF | : N,N-dimethylformamide |
| DMSO | : dimethyl sulfoxide |
| LC/MS | : liquid chromatography-mass spectrometry spectrum |
| ESI | : electrospray method |
| APCI | : atmospheric chemical ionization |
| $[M+H]^+$ : | molecular ion peak |
| TFA | : trifluoroacetic acid |
| M | : mol concentration |
| N | : normal concentration |
| WSC | : N-(3-dimethylaminopropyl)-N'- ethylcarbodiimidehydrochloride |
| HOBt | : 1-hydroxybenzotriazole monohydrate |
| DEPC | : diethyl cyanophosphate |
| IPE | : diisopropyl ether |
| Pd/C | : 10% palladium carbon (containing water (50%)) |
| CDI | : N,N-carbonyldiimidazole |
| HATU | : 2-(7-azabenzotriazol-1-yl)-1,1,3,3- hexafluorophosphate |
| DMA | : dimethylacetamide |
| DME | : 1,2-dimethoxyethane |
| EtOH | : ethanol |
| DMPU | : N,N-dimethylpropyleneurea |
| $Pd_2(dba)_3$ | : tris(dibenzylideneacetone)dipalladium |
| $Pd(PPh_3)_4$ | : tetrakistriphenylphosphinepalladium |
| HPLC | : high performance liquid chromatography |
| UV | : ultraviolet ray |
| quant. | : quantitatively |

Elution in the column chromatography in Examples and Reference Examples was conducted under observation by TLC (thin layer chromatography). In the TLC observation, $60F_{254}$ manufactured by Merck or NH manufactured by

Fuji Silysia Chemical Ltd. was used as a TLC plate, the solvent used in column chromatography as an elution solvent was used as an eluent, and UV detector was employed as a detection method. As silica gel for column, Kieselgel 60 (70 to 230 mesh), Kieselgel 60 (230 to 400 mesh) manufactured by the same Merck or Purif-Pack by MORITEX was used. As basic silica gel for column (NH silica gel), basic silica NH-DM1020 (100 to 200 mesh) manufactured by Fuji Silysia Chemical Ltd. or Purif-Pack by MORITEX was used. $^1$H NMR spectrum was measured using tetramethylsilane as an internal or external standard and Bruker AVANCE-300 ($^1$H resonance frequency: 300 MHz) or Varian VNMRS-400 ($^1$H resonance frequency: 400 MHz) spectrometer, and the chemical shift is shown in 5 value and the coupling constant is shown in Hz. In mixed solvents, the numerical values shown in the parentheses are mixing volume ratios of respective solvents. In addition, %of solution means a number in g in 100 mL of a solution. The room temperature generally means a temperature from about 10˚C to 30˚C. The unit of sample concentration (c) in optical rotation ($[\alpha]_D$) is g/100 mL.

MS (mass spectrum) was measured by LC/MS. As the ionization method, ESI method, or APCI method was used. The data indicates those found. Generally, a molecular ion peak is observed. In the case of a compound having a tert-butoxycarbonyl group (-Boc), a peak after elimination of a tert-butoxycarbonyl group or tert-butyl group may be observed as a fragment ion. In the case of a compound having a hydroxyl group (-OH), a peak after elimination of $H_2O$ may be observed as a fragment ion. In the case of a salt, a molecular ion peak or fragment ion peak of free form is generally observed.

LC/MS in Examples and Reference Examples was measured under conditions of the following 1. - 6.

1.

measurement device: Waters LC/MS system
column: CAPCELLPAK C18, S-3 μm, 1.5×3.5 mm (Shiseido Co., Ltd.)
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=90/10), 2.00 min (SOLUTION A/SOLUTION B=5/95), 2.75 min (SOLUTION A/SOLUTION B=5/95), 2.76 min (SOLUTION A/SOLUTION B=90/10), 3.45 min (SOLUTION A/SOLUTION B=90/10)
injection volume: 10 μL, flow rate: 0.5 mL/min, detection method: UV 220 nm
MS conditions ionization method: ESI

2.

measurement device: Agilent LC/MS system
column: ZORBAX C18, S-1.8 μm, 3.0×30 mm (Agilent) solvent: SOLUTION A; 10 mM ammonium acetate-containing water, SOLUTION B; 10 mM ammonium acetate-containing acetonitrile gradient cycle: 0.00 min (SOLUTION A/SOLUTION B=90/10), 2.00 min (SOLUTION A/SOLUTION B=5/95), 2.75 min (SOLUTION A/SOLUTION B=5/95), 2.76 min (SOLUTION A/SOLUTION B=90/10), 3.45 min (SOLUTION A/SOLUTION B=90/10)
injection volume: 10 μL, flow rate: 1.2 mL/min, detection method: UV 220 nm
MS conditions ionization method: ESI

3.

measurement device: Quattro Micro manufactured by Micromass, HP1100 manufactured by Agilent Technologies, or high performance liquid chromatography mass spectrometer LCMS-2010A manufactured by SHIMADZU Corporation, or MUX system manufactured by Waters (Micromass ZQ)
column: CAPCELLPAK C18 UG-120, 1.5x35 mm (Shiseido Co., Ltd., or DEVELOSIL COMBI-RP-5.2×35 mm (Nomura Chemical Co., Ltd.) solvent: SOLUTION A; 5 mM ammonium acetate/2% acetonitrile/water, SOLUTION B; 5 mM ammonium acetate/95% acetonitrile/water
gradient cycle: 0.00 min (SOLUTION A/SOLUTION B=100/0), 2.00 min (SOLUTION A/SOLUTION B=0/100), 3.00 min (SOLUTION A/SOLUTION B=0/100), 3.01 min (SOLUTION A/SOLUTION B=100/0) 3.80 min (SOLUTION A/SOLUTION B=100/0)
injection volume: 10 μL, flow rate: 0.5 mL/min, detection method: UV 220 nm
MS conditions ionization method: ESI

4.

measurement device: Waters MUX-mounted 4-ch LC/MS system column: CAPCELL PAK C18 UG-120, S-3 μm, 1.5×35.mm (Shiseido Co., Ltd.)
solvent: SOLUTION A; 5 mM ammonium acetate-containing water, SOLUTION B; 5 mM ammonium acetate-containing acetonitrile gradient cycle: 0.00 min (SOLUTION A/SOLUTION B=100/0), 2.00 min (SOLUTION A/SOLUTION B=0/100), 3.00 min (SOLUTION A/SOLUTION B=0/100), 3.01 min (SOLUTION A/SOLUTION B=100/0), 3.30 min (SOLUTION A/SOLUTION B=100/0)
injection volume: 2 μL, flow rate: 0.5 mL/min, detection method: UV 220 nm
ionization method: ESI

5.

HPLC part: Agilent 1200
MS part: Agilent 6300
column: Welchrom XB-C18, 5 μm, 4.6×50 mm
solvent: SOLUTION A; water, SOLUTION B; acetonitrile
gradient cycle: 0.00 main (SOLUTION A/SOLUTION B=95/5), 6.00 min (SOLUTION A/SOLUTION B=5/95), 6.50 min (SOLUTION A/SOLUTION B=5/95); or 0.00 min (SOLUTION A/SOLUTION B=90/10), 6.00 min (SOLUTION A/SOLUTION B=5/95), 6.50 min (SOLUTION A/SOLUTION B=5/95); or 0.00 min (SOLUTION A/SOLUTION B=80/20), 6.00 min (SOLUTION A/SOLUTION B=5/95), 6.50 min (SOLUTION A/SOLUTION B=5/95); or 0.00 min (SOLUTION A/SOLUTION B=70/30), 6.00 min (SOLUTION A/SOLUTION B=5/95), 6.50 min (SOLUTION A/SOLUTION B=5/95); or 0.00 min (SOLUTION A/SOLUTION B=60/40), 6.00 min (SOLUTION A/SOLUTION B=5/95), 6.50 min (SOLUTION A/SOLUTION B=5/95); or 0.00 min (SOLUTION A/SOLUTION B=50/50), 6.00 min (SOLUTION A/SOLUTION B=5/95), 6.50 min (SOLUTION A/SOLUTION B =5/95); or 0.00 min (SOLUTION A/SOLUTION B=40/60), 6.00 min (SOLUTION A/SOLUTION B=5/95), 6.50 min (SOLUTION A/SOLUTION B=5/95)
flow rate: 1.5 mL/min, detection method: UV 214 or 254 nm
ionization method: ESI

6.

HPLC part: Agilent 1200
MS part: Finigan LCQ Advantage MAX manufactured by Thermo Electron Corp.
column: YMC Hydrosphere (C18, 4.6×50 mm, 3 μm, 120 A)
solvent: SOLUTION A; 0.01% heptafluorobutanoic acid and 0.01% isopropyl alcohol-containing water, SOLUTION B; 0.01% heptafluorobutanoic acid and 0.01% isopropyl alcohol-containing acetonitrile
flow rate: 1.0 mL/min, detection method: UV 214 or 254 nm ionization method: ESI

In addition, the purification by preparative HPLC in the Examples and Reference Examples were performed under any of the following conditions 1. - 6.

1.

device: Gilson Inc. Semi-preparative purification system
column: YMC CombiPrep Pro C18 RS, S-5 μm, 50×20 mm
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=90/10), 1.20 min (SOLUTION A/SOLUTION B=90/10), 4.75 min (SOLUTION A/SOLUTION B=0/100), 7.30 min (SOLUTION A/SOLUTION B=0/100), 7.40 min (SOLUTION A/SOLUTION B=90/10), 7.50 min (SOLUTION A/SOLUTION B=90/10)
flow rate: 25 mL/min, detection method: UV 220 nm 2.
device: Waters preparative purification system
column: Waters SunFire C18, S-5 μm, 30×50 mm
solvent: SOLUTION A; 0.1% trifluoroacetic acid containing water, SOLUTION B; 0.1% trifluoroacetic acid containing acetonitrile
gradient cycle: 0.00 min (SOLUTION A/SOLUTION B=90/10), 1.20 min (SOLUTION A/SOLUTION B=90/10), 5.20 min (SOLUTION A/SOLUTION B=0/100), 7.00 min (SOLUTION A/SOLUTION B=0/100), 7.00 min (SOLUTION A/SOLUTION B=90/10), 8.50 min (SOLUTION A/SOLUTION B=90/10)
flow rate: 70 mL/min, detection method: UV 220 nm 3.

device: Waters preparative purification system
column: YMC CombiPrep ODS-A, S-5 μm, 50x20 mm
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=90/10), 0.20 min (SOLUTION A/SOLUTION B=90/10), 4.20 min (SOLUTION A/SOLUTION B=0/100), 6.30 min (SOLUTION A/SOLUTION B=0/100), 6.30 min (SOLUTION A/SOLUTION B=90/10), 7.50 min (SOLUTION A/SOLUTION B=90/10)
flow rate: 25 mL/min, detection method: UV 220 nm 4.
device: Gilson Inc. High-Throughput purification system
column: CAPCELL PAK C18 UG-120, S-5 μm, 20×50 mm or YMC CombiPrep Hydrosphere C18 HS-340-CC, S-5 μm, 20×50 mm (Shiseido Co., Ltd.)
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=95/5), 1.10 min (SOLUTION A/SOLUTION B=95/5), 5.00 min (SOLUTION A/SOLUTION B=0/100), 6.40 min (SOLUTION A/SOLUTION B=0/100), 6.50 min (SOLUTION A/SOLUTION B=95/5)
flow rate: 20 ml/min, detection method: UV 220 nm 5.
device: Gilson Inc. High-Throughput purification system
column: YMC CombiPrep, ProC18 RS S-5 μm, 20×50 mm (YMC)
solvent: SOLUTION A; 10 mM ammonium carbonate-containing water, SOLUTION B; acetonitrile
gradient cycle: 0.00 min (SOLUTION A/SOLUTION B=95/5), 1.10 min (SOLUTION A/SOLUTION B=95/5), 4.60 min (SOLUTION A/SOLUTION B=0/100), 6.40 min (SOLUTION A/SOLUTION B=0/100), 6.50 min (SOLUTION A/SOLUTION B=95/5), 6.60 min (SOLUTION A/SOLUTION B=95/5)
injection volume: 1000 μl, flow rate: 25 ml/min, detection method: UV 220 nm, 254 nm 6.
device: Gilson Inc. purification system
column: Welchrom XB-C18, 5 μm, 150×20 mm
solvent: SOLUTION A; 0.1% trifluoroacetic acid-containing acetonitrile, SOLUTION B; 0.1% trifluoroacetic acid-containing water
gradient cycle: 0.00 min (SOLUTION A/SOLUTION B=10/90), 5.00 min (SOLUTION A/SOLUTION B=10/90), 20.00 min (SOLUTION A/SOLUTION B=70/30), 25.00 min (SOLUTION A/SOLUTION B=70/30), 30.00 min (SOLUTION A/SOLUTION B=10/90); or 0.00 min (SOLUTION A/SOLUTION B=10/90), 5.00 min (SOLUTION A/SOLUTION B=10/90), 20.00 min (SOLUTION A/SOLUTION B=80/20), 25.00 min (SOLUTION A/SOLUTION B=80/20), 30.00 min (SOLUTION A/SOLUTION B=10/90); and the like
flow rate: 25 mL/min, detection method: UV 220 nm

Example 1

8-(3,4-dichlorophenyl)-3-[3-methoxy-4-(2-methyl-1,3-oxazol-5-yl)phenyl]-5,6,7,8-tetrahydro[1,2,4]triazolo[4,3-a]pyridine

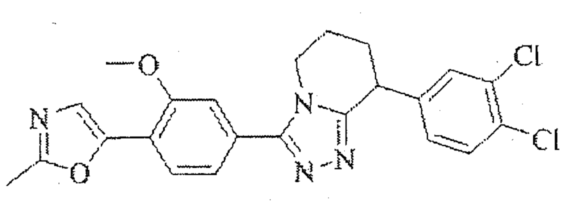

A mixture of 2-[4-chloro-1-(3,4-dichlorophenyl)butyl]-5-[3-methoxy-4-(2-methyl-1,3-oxazol-5-yl)phenyl]-1,3,4-oxadiazole (250 mg, 0.507 mmol) and sodium azide (65.9 mg, 1.01 mmol) in DMSO (2.5 mL) was stirred at 70°C for 12 hr. After cooling to room temperature, water was added to the reaction mixture, and the mixture was extracted with ethyl acetate. The obtained extract was washed with water and saturated brine, and dried over anhydrous sodium sulfate. The solvent was evaporated under reduced pressure. The residue was diluted with THF (2.5 mL), water (0.25 mL) and triphenylphosphine (265 mg, 1.01 mmol) were added, and the mixture was stirred at 60°C for 1 hr. The solvent was evaporated under reduced pressure, acetic acid (2.0 mL) was added to the residue, and the mixture was heated under reflux for 1.5 hr. The solvent was evaporated under reduced pressure, saturated aqueous sodium hydrogen carbonate

solution was added to the residue, and the mixture was extracted with ethyl acetate. The obtained extract was washed with saturated brine, and dried over anhydrous sodium sulfate. The solvent was evaporated under reduced pressure. The residue was purified by silica gel column chromatography (methanol/ethyl acetate=0/100 - 15/85) to give the title compound as a white solid (202 mg, 87%).
$^1$H NMR ($CDCl_3$) δ: 1.93 - 2.25 (3 H, m), 2.29 - 2.44 (1 H, m), 2.56 (3 H, s), 4.05 (3 H, s), 4.12 - 4.24 (2 H, m), 4.36 - 4.49 (1 H, m), 7.10 (1 H, dd, J=8.2, 2.2 Hz), 7.28 (1 H, dd, J=8.1, 1.5 Hz), 7.34 (1 H, d, J=2.2 Hz), 7.42 (1 H, d, J=8.2 Hz), 7.46 - 7.54 (2 H, m), 7.84 (1 H, d, J=8.1 Hz).
MS(ESI):455 $[M+H]^+$.

Example 2

(+)-8-(3,4-dichlorophenyl)-3-[3-methoxy-4-(2-methyl-1,3-oxazol-5-yl)phenyl]-5,6,7,8-tetrahydro[1,2,4]triazolo[4,3-a]pyridine

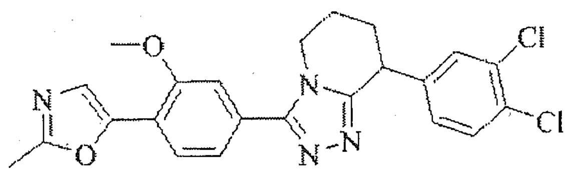

8-(3,4-Dichlorophenyl)-3-[3-methoxy-4-(2-methyl-1,3-oxazol-5-yl)phenyl]-5,6,7,8-tetrahydro[1,2,4]triazolo[4,3-a]pyridine (100 mg) was fractionated by using high performance liquid chromatography [device: K-Prep (manufactured by YMC), column: CHIRALCEL OD (50 mmID×500 mmL manufactured by DAICEL CHEMICAL INDUSTRIES, LTD.), mobile phase: A) hexane 100%, B) ethanol=100%, mixing ratio: A/B=700/300, flow rate: 80 mL/min, column temperature: 30°C, sample injection volume: 100 mg (dissolved in mobile phase (40 mL))]. A fraction solution containing an optically active form having a shorter retention time in the above-mentioned high performance liquid chromatography conditions was concentrated to give the title compound (51 mg, >99.9% ee). The enantiomer excess (ee) was measured by high performance liquid chromatography [(column: CHIRALCEL OD (4.6 mmID×250 mmL manufactured by DAICEL CHEMICAL INDUSTRIES, LTD.), mobile phase: hexane/ethanol=700/300, flow rate: 1.0 mL/min, column temperature: 30°C, sample concentration: 0.25 mg/mL (mobile phase), injection volume: 10 μL)].
$[\alpha]_D^{25}$: +112.1°(c 0.474, methanol),
$^1$H NMR ($CDCl_3$) δ:1.96 - 2.22 (3 H, m), 2. 32 - 2.44 (1 H, m), 2.56 (3 H, s), 4.05 (3 H, s), 4.14 - 4.22 (2 H, m), 4.38 - 4.45 (1 H, m), 7.11 (1 H, dd, J=8.1, 2.2 Hz), 7.28 (1 H, dd, J=8.2, 1.5 Hz), 7.34 (1 H, d, J=2.2 Hz), 7.42 (1 H, d, J=8.2 Hz), 7.46 - 7.53 (2 H, m), 7.85 (1 H, d, J=8.1 Hz).
MS(ESI):455 $[M+H]^+$.

Example 3

(-)-8-(3,4-dichlorophenyl)-3-[3-methoxy-4-(2-methyl-1,3-oxazol-5-yl)phenyl]-5,6,7,8-tetrahydro[1,2,4]triazolo[4,3-a]pyridine

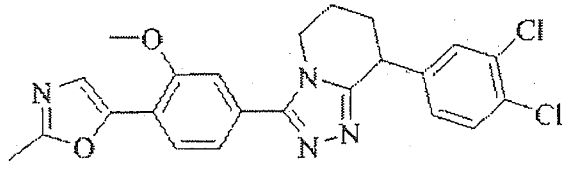

8-(3,4-Dichlorophenyl)-3-[3-methoxy-4-(2-methyl-1,3-oxazol-5-yl)phenyl]-5,6,7,8-tetrahydro[1,2,4]triazolo[4,3-a]pyridine (100 mg) was fractionated by using high performance liquid chromatography [device: K-Prep (manufactured by YMC), column: CHIRALCEL OD (50 mmID×500 mmL manufactured by DAICEL CHEMICAL INDUSTRIES,

LTD.), mobile phase: A) hexane 100%, B) ethanol=100%, mixing ratio: A/B=700/300, flow rate: 80 mL/min, column temperature: 30˚C, sample injection volume: 100 mg (dissolved in mobile phase (40 mL))]. A fraction solution containing an optically active form having a longer retention time in the above-mentioned high performance liquid chromatography conditions was concentrated to give the title compound (50 mg, >99.9% ee). The enantiomer excess (ee) was measured by high performance liquid chromatography [(column: CHIRALCEL OD (4.6 mmIDx250 mmL manufactured by DAICEL CHEMICAL INDUSTRIES, LTD.), mobile phase: hexane/ethanol=700/300, flow rate: 1.0 mL/min, column temperature: 30˚C, sample concentration: 0.25 mg/mL (mobile phase), injection volume: 10 μL)].
$[\alpha]_D^{25}$: -131.1˚(c 0.229, methanol),
$^1$H NMR ($CDCl_3$) δ:1.93 - 2.25 (3 H, m), 2.29 - 2.45 (1 H, m), 2.56 (3 H, s), 4.05 (3 H, s), 4.12 - 4.27 (2 H, m), 4.33 - 4.47 (1 H, m), 7.10 (1 H, dd, J=8.2, 2.2 Hz), 7.28 (1 H, dd, J=8.1, 1.5 Hz), 7.34 (1 H, d, J=2.2 Hz), 7.42 (1 H, d, J=8.2 Hz), 7.46 - 7.54 (2 H, m), 7.84 (1 H, d, J=8.1 Hz). MS(ESI):455 $[M+H]^+$.

Example 4

8-(3,4-dichlorophenyl)_2-[3-methoxy-4-(2-methyl-1,3-oxazol-5-yl)phenyl]-5,6,7,8-tetrahydro[1,2,4]triazolo[1;5-a]pyridine

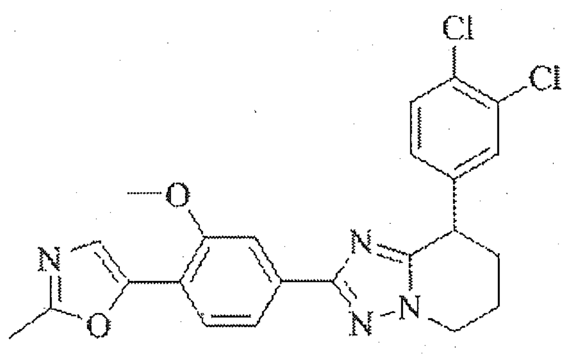

4a) ethyl 5-chloro-2-(3,4-dichlorophenyl)pentaneimidate hydrochloride

To a solution of 5-chloro-2-(3,4-dichlorophenyl)pentanenitrile (1.46 g, 5.56 mmol) in ethanol (4.34 mL) was added dropwise, under ice-cooling, acetyl chloride (3.16 mL, 44.5 mmol). After stirring at room temperature for 60 hr, the solvent was evaporated under reduced pressure. Recrystallization from ethanol-IPE gave the title compound as a white solid (1.62 g, 44%).
$^1$H NMR ($CDCl_3$) δ: 1.50 (3 H, t, J=7.0 Hz), 1.67 - 1.90 (2 H, m), 2.01 - 2.17 (1 H, m), 2.20 - 2.36 (1 H, m), 3.48 - 3.65 (2 H, m), 4.41 (1 H, t, J=7.9 Hz), 4.54 - 4.78 (2 H, m), 7.36 - 7.41 (1 H, m), 7.43 - 7.48 (1 H, m), 7.58 (1 H, d, J=1.9 Hz), 11.77 (1 H, brs), 12.79 (1 H, brs).

4b) 8-(3,4-dichlorophenyl)-2-[3-methoxy-4-(2-methyl-1,3-oxazol-5-yl)phenyl]-5,6,7,8-tetrahydro[1,2,4]triazolo[1,5-a]pyridine

A mixture of 3-methoxy-4-(2-methyl-1,3-oxazol-5-yl)benzohydrazide hydrochloride (304 mg, 1.07 mmol), ethyl 5-chloro-2-(3,4-dichlorophenyl)pentaneimidate hydrochloride (369 mg, 1.07 mmol), imidazole (874 mg, 12.8 mmol) in methanol (7.1 mL) was stirred at room temperature for 16 hr, and at 65˚C for 3 hr. The solvent was evaporated under reduced pressure, water was added to the reaction mixture, and the mixture was extracted with ethyl acetate. The obtained extract was washed with water and saturated brine, and dried over anhydrous magnesium sulfate. The solvent was evaporated under reduced pressure. The residue was purified by NH silica gel column chromatography (ethyl acetate/hexane=10/90 - 90/10), and recrystallized from ethyl acetate-hexane to give the title compound as a white solid (224 mg, 46%).
$^1$H NMR ($CDCl_3$) δ: 1.95 - 2.26 (3 H, m), 2.29 - 2.45 (1 H, m), 2.53 (3 H, s), 4.03 (3 H, s), 4.24 - 4.43 (3 H, m), 7.01 (1 H, dd, J=8.3, 1.9 Hz), 7.22 - 7.31 (1 H, m), 7.38 - 7.47 (2 H, m), 7.66 (1 H, s), 7.70 - 7.81 (2 H, m).

Example 5

2-[3-methoxy-4-(3-methyl-1H-1,2,4-triazol-1-yl)phenyl]-8-[2-(trifluoromethyl)phenyl]-5,6,7,8-tetrahydro[1,2,4]triazolo[1,5-a]pyridine

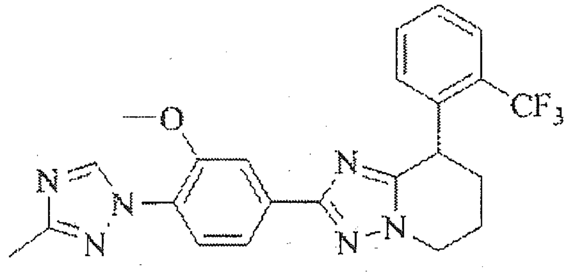

5a) 1-amino-3-[2-(trifluoromethyl)phenyl]piperidin-2-one

To a solution of [2-(trifluoromethyl)phenyl]acetic acid (1.90 g, 9.31 mmol) in methanol (38 mL) was added thionyl chloride (2.68 mL, 37.2 mmol) under ice-cooling, and the mixture was stirred for 3 hr. The solvent was evaporated, and the residue was diluted with DMF (30 mL). Sodium hydride (410 mg, 10.3 mmol) was added, and the mixture was stirred for 30 min. 1-Chloro-3-iodopropane (1.02 mL, 9.50 mmol) was added, and the mixture was further stirred for 15 hr. The reaction mixture was diluted with ethyl acetate, washed with water and saturated brine, and dried over anhydrous sodium sulfate. The solvent was evaporated under reduced pressure. The residue was diluted with ethanol (27 mL), hydrazine monohydrate (7.6 mL) was added, and the mixture was stirred at room temperature for 2 hr, and at 60°C for 20 hr. The solvent was evaporated, diluted with ethyl acetate, and washed with saturated aqueous sodium hydrogen carbonate solution and saturated brine. This was dried over anhydrous sodium sulfate and the solvent was evaporated under reduced pressure. The residue was purified by silica gel column chromatography (methanol/ethyl acetate=0/100 - 15/85), and the obtained solid was washed with IPE to give the title compound as a white solid (910 mg, 38%).
$^1H$ NMR ($CDCl_3$) δ:1.79 - 2.11 (3 H, m), 2.16 - 2.30 (1 H, m), 3.54 - 3.79 (2 H, m), 3.98 - 4.15 (1 H, m), 4.60 (2 H, br), 7.24 (1 H, d, J=7.7 Hz), 7.30 - 7.40 (1 H, m), 7.44 - 7.54 (1 H, m), 7.66 (1 H, d, J=8.0 Hz). MS(ESI):259 $[M+H]^+$.

5b) 3-methoxy-4-(3-methyl-1H-1,2,4-triazol-1-yl)-N-{2-oxo-3-[2-(trifluoromethyl)phenyl]piperidin-1-yl}benzamide

A mixture of 3-methoxy-4-(3-methyl-1H-1,2,4-triazol-1-yl)benzonitrile (214 mg, 1.00 mmol), 4M aqueous sodium hydroxide solution (2 mL) and 2-methoxyethanol (2 mL) was heated under reflux for 2 hr. The reaction solution was cooled to room temperature, 1 M hydrochloric acid (9 mL) was added, extracted with a mixed solution of ethyl acetate and methanol, and the solvent was evaporated. A mixture of the residue, 1-amino-3-[2-(trifluoromethyl)phenyl]piperidin-2-one (387 mg, 1.50 mmol), WSC (288 mg, 1.50 mmol), HOBt (203 mg, 1.50 mmol) and N-ethyldiisopropylamine (428 μL, 2.50 mmol) in DMF (5.0 mL) was stirred at room temperature for 20 hr, diluted with ethyl acetate, washed with water and saturated brine, and dried over anhydrous sodium sulfate. The solvent was evaporated under reduced pressure. The obtained residue was washed with ethyl acetate to give the title compound as a colorless solid (306 mg, 65%).
$^1H$ NMR ($CDCl_3$) δ:1.99 - 2.22 (3 H, m), 2.25 - 2.40 (1 H, m), 2.49 (3 H, s), 3.58 - 3.67 (1 H, m), 3.69 (3 H, s), 3.84 - 4.00 (1 H, m), 4.17 - 4.30 (1 H, m), 7.29 - 7.47 (3 H, m), 7.48 - 7.77 (4 H, m), 8.75 (1 H, s), 10.17 (1 H, br). MS(ESI): 474 $[M+H]^+$.

5c) 2-[3-methoxy-4-(3-methyl-1H-1,2,4-triazol-1-yl)phenyl]-8-[2-(trifluoromethyl)phenyl]-5,6,7,8-tetrahydro[1,2,4]triazolo[1,5-a]pyridine

A mixture of 3-methoxy-4-(3-methyl-1H-1,2,4-triazol-1-yl)-N-{2-oxo-3-[2-(trifluoromethyl)phenyl]piperidin-1-yl} benzamide (299 mg, 0.632 mmol) and phosphorus oxychloride (6.3 mL) was stirred at 100°C for 1 hr, and the solvent was evaporated. The residue was diluted with acetic acid (6.3 mL), ammonium acetate (974 mg, 12.6 mmol) was added, and the mixture was heated under reflux for 3.5 hr. The reaction solution was cooled to room temperature, diluted with ethyl acetate, and washed with saturated aqueous sodium hydrogen carbonate solution and saturated brine. This was dried over anhydrous sodium sulfate and the solvent was evaporated under reduced pressure. The residue was purified by NH silica gel column chromatography (ethyl acetate/hexane=20/80 - 80/20) to give the title compound as a white

solid (15.0 mg, 5%). MS(ESI):455 [M+H]+.

Example 6

8-(3,4-dichlorophenyl)-3-[3-methoxy-4-(3-methyl-1H-1,2,4-triazol-1-yl)phenyl]-5,6,7,8-tetrahydro[1,2,4]triazolo[4,3-a] pyridine

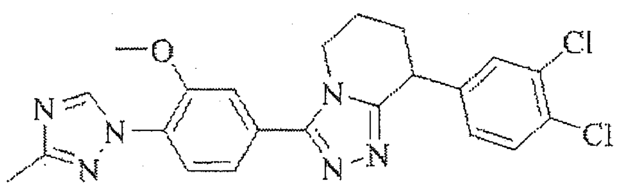

A mixture of 2-[4-chloro-1-(3,4-dichlorophenyl)butyl]-5-[3-methoxy-4-(3-methyl-1H-1,2,4-triazol-1-y1)phenyl]-1,3,4-oxadiazole (40.1 mg, 0.0813 mmol) and sodium azide (15.9 mg, 0.244 mmol) in DMSO (1.0 mL) was stirred at 70˚C for 5 hr. After cooling to room temperature, water was added to the reaction mixture, and the mixture was extracted with ethyl acetate. The obtained extract was washed with water and saturated brine, and dried over anhydrous sodium sulfate. The solvent was evaporated under reduced pressure. The residue was diluted with THF (1.0 mL), water (50 $\mu$L) and triphenylphosphine (42.6 mg, 0.163 mmol) were added, and the mixture was stirred at 60˚C for 3 hr. The solvent was evaporated under reduced pressure, acetic acid (0.5 mL) was added to the residue, and the mixture was heated under reflux for 1.5 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous sodium sulfate. The solvent was evaporated under reduced pressure. The residue was purified by silica gel column chromatography (methanol/ethyl acetate=0/100 - 20/80) to give the title compound as a white solid (32.0 mg, 86%).
$^1$H NMR ($CDCl_3$) 1.98 - 2.12 (2 H, m), 2.11 - 2.25 (1 H, m), 2.28 - 2.46 (1 H, m), 2.51 (3 H, s), 4.03 (3 H, s), 4.18 (2 H, t, J=5.4 Hz), 4.36 - 4.46 (1 H, m), 7.10 (1 H, dd, J=8.2, 2.2 Hz), 7.28 - 7.37 (2 H, m), 7.42 (1 H, d, J=8.5 Hz), 7.63 (1 H, d, J=1.6 Hz), 7.96 (1 H, d, J=8.2 Hz), 8.77 (1 H, s). MS(ESI):455 [M+H]+.

Example 7

3-[3-methoxy-4-(2-methyl-1,3-oxazol-5-yl)phenyl]-8-(3,4,5-trifluorophenyl)-5,6,7,8-tetrahydro[1,2,4]triazolo[4,3-a]pyridine

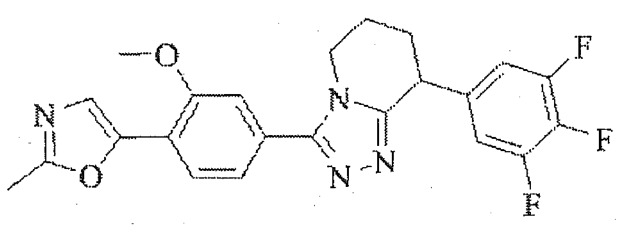

A mixture of 2-[4-chloro-1-(3,4,5-trifluorophenyl)butyl]-5-[3-methoxy-4-(2-methyl-1,3-oxazol-5-yl)phenyl]-1,3,4-oxadiazole (126 mg, 0.262 mmol) and sodium azide (51.5 mg, 0.792 mmol) in DMSO (2 mL) was stirred at 70˚C for 12 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (2 mL), water (0.10 mL) and triphenylphosphine (137 mg, 0.524 mmol) were added, and the mixture was stirred at 60˚C for 1 hr. The solvent was evaporated under reduced pressure, acetic acid (1.0 mL) was added to the residue, and the mixture was stirred at

100°C for 1 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (methanol/ethyl acetate=0/100 - 10/90), and recrystallized from ethyl acetate to give the title compound as a white solid (52.0 mg, 45%).
$^1$H NMR ($CDCl_3$) δ: 1.89 - 2.23 (3 H, m), 2.28 - 2.45 (1 H, m), 2.55 (3 H, s), 4.04 (3 H, s), 4.09 - 4.22 (2 H, m), 4.39 (1 H, t, J=7.3 Hz), 6.90 (2 H, dd, J=8.1, 6.5 Hz), 7.25 - 7.29 (1 H, m), 7.43 - 7.53 (2 H, m), 7.84 (1 H, d, J=8.2 Hz). MS (ESI):441 $[M+H]^+$.

Example 8

3-[3-methoxy-4-(2-methyl-1,3-oxazol-5-yl)phenyl]-8-[2-(trifluoromethyl)phenyl]-5,6,7,8-tetrahydro[1,2,4]triazolo[4,3-a]pyridine

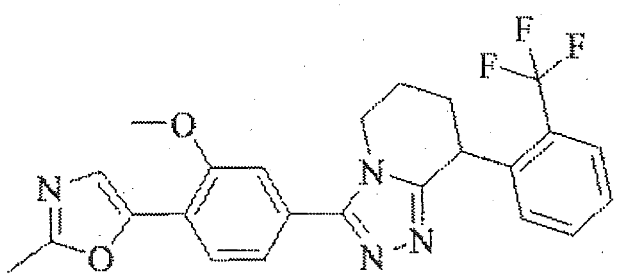

A mixture of 2-{4-chloro-1-[2-(trifluoromethyl)phenyl]butyl}-5-[3-methoxy-4-(2-methyl-1,3-oxazol-5-yl)phenyl]-1,3,4-oxadiazole (410 mg, 0.834 mmol) and sodium azide (163 mg, 2.50 mmol) in DMSO (8 mL) was stirred at 70°C for 16 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (8 mL), water (0.40 mL) and triphenylphosphine (438 mg, 1.67 mmol) were added, and the mixture was stirred at 60°C for 1 hr. The solvent was evaporated under reduced pressure, acetic acid (4.0 mL) was added to the residue, and the mixture was stirred at 100°C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (methanol/ethyl acetate=0/100 - 15/85) to give the title compound as a white solid (282 mg, 74%).
$^1$H NMR ($CDCl_3$) δ: 1.82 - 2.15 (2 H, m), 2.16 - 2.32 (1 H, m), 2.38 - 2.53 (1 H, m), 2.56 (3 H, s), 4.04 (3 H, s), 4.09 - 4.32 (2 H, m), 4.77 (1 H, dd, J=9.7, 5.9 Hz), 7.18 (1 H, d, J=7.6 Hz), 7.30 (1 H, dd, J=8.0, 1.5 Hz), 7.34 - 7.43 (1 H, m), 7.44 - 7.54 (3 H, m), 7.72 (1 H, d, J=7.2 Hz), 7.85 (1 H, d, J=8.3 Hz). MS(ESI):455 $[M+H]^+$.

Example 9

8-(4-fluorophenyl)-3-[3-methoxy-4-(2-methyl-1,3-oxazol-5-yl)phenyl]-5,6,7,8-tetrahydro[1,2,4]triazolo[4,3-a]pyridine

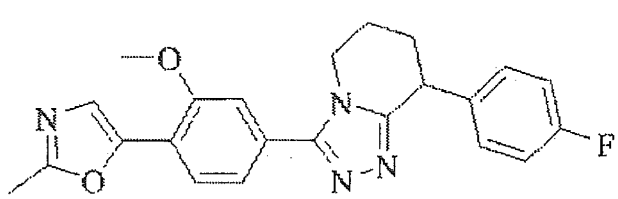

A mixture of 2-[4-chloro-1-(4-fluorophenyl)butyl]-5-[3-methoxy-4-(2-methyl-1,3-oxazol-5-yl)phenyl]-1,3,4-oxadiazole (105 mg, 0.238 mmol) and sodium azide (30.9 mg, 0.475 mmol) in DMSO (1.2 mL) was stirred at 70˚C for 16 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.2 mL), water (0.12 mL) and triphenylphosphine (125 mg, 0.475 mmol) were added, and the mixture was stirred at 60˚C for 1 hr. The solvent was evaporated under reduced pressure, acetic acid (1.2 my) was added to the residue, and the mixture was stirred for 100˚C for 1.5 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (methanol/ethyl acetate=0/100 - 15/85), and recrystallized from ethyl acetate-hexane to give the title compound as a white solid (73.2 mg, 76%).
$^1$H NMR ($CDCl_3$) δ: 1.94 - 2.22 (3 H, m), 2.27 - 2.43 (1 H, m), 2.56 (3 H, s), 4.04 (3H s), 4.17 (2 H, t, J=5.8 Hz), 4.46 (1 H, t, J=6.7 Hz), 6.98 - 7.08 (2 H, m), 7.15 - 7.24 (2 H, m), 7.29 (1 H, dd, J=8.1, 1.5 Hz), 7.44 - 7.55 (2 H, m), 7.84 (1 H, d, J=8.1 Hz). MS(ESI):405 [M+H]$^+$.

Example 10

3-[3-methoxy-4-(2-methyl-1,3-oxazol-5-yl)phenyl]-8-[3-(trifluoromethyl)phenyl]-5,6,7,8-tetrahydro[1,2,4]triazolo[4,3-a]pyridine

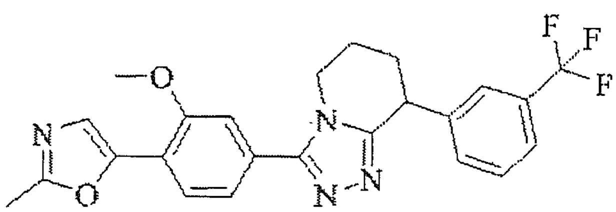

A mixture of 2-{4-chloro-1-[3-(trifluoromethyl)phenyl]butyl}-5-[3-methoxy-4-(2-methyl-1,3-oxazol-5-yl)phenyl]-1,3,4-oxadiazole (118 mg, 0.240 mmol) and sodium azide (31.2 mg, 0.480 mmol) in DMSO (1.2 mL) was stirred at 70˚C for 16 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.2 mL), water (0.12 mL) and triphenylphosphine (125 mg, 0.475 mmol) were added, and the mixture was stirred at 60˚C for 1 hr. The solvent was evaporated under reduced pressure, acetic acid (1.2 mL) was added to the residue, and the mixture was stirred at 120˚C for 1.5 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by NH silica gel column chromatography (ethyl acetate/hexane=1/1 - 9/1), and recrystallized from ethyl acetate-hexane to give the title compound as a white solid (60.0 mg, 55%).
$^1$H NMR ($CDCl_3$) 5: 1.95 - 2.26 (3 H, m), 2.33 - 2.47 (1 H, m), 2.56 (3 H, s), 4.05 (3 H, s), 4.15 - 4.25 (2 H, m), 4.51 (1 H, t, J=7.0 Hz), 7.30 (1 H, dd, J=7.9, 1.5 Hz), 7.40 - 7.62 (6 H, m), 7.85 (1 H, d, J=8.3 Hz). MS(ESI):455 [M+H]$^+$.

Example 11

2-[3-methoxy-4-(2-methyl-1,3-oxazol-5-yl)phenyl]-8-(3,4,5-trifluorophenyl)-5,6,7,8-tetrahydro[1,2,4]triazolo[1,5-a]pyridine

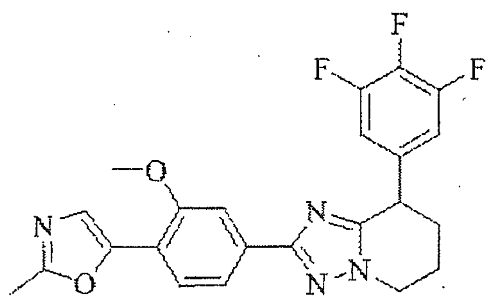

A mixture of 2-[4-chloro-1-(3,4,5-trifluorophenyl)butyl]-5-[3-methoxy-4-(2-methyl-1,3-oxazol-5-yl)phenyl]-1,3,4-oxadiazole (37.7 mg, 0.079 mmol) and ammonium acetate (60.8 mg, 0.79 mmol) in acetic acid (1 mL) was stirred at 100°C for 5 hr. Ammonium acetate (60.8 mg, 0.79 mmol) was added and the mixture was further stirred at 120°C for 15 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous magnesium sulfate. The solvent was evaporated under reduced pressure. The residue was purified by silica gel column chromatography (ethyl acetate/hexane=50/50 - 90/10), and recrystallized from ethyl acetate-hexane to give the title compound as a white solid (5.0 mg, 14%).
MS (ESI) :441 $[M+H]^+$.

Example 12

2-[3-methoxy-4-(2-methyl-1,3-oxazol-5-yl)phenyl]-8-[2-(trifluoromethyl)phenyl]-5,6,7,8-tetrahydro[1,2,4]triazolo[1,5-a]pyridine

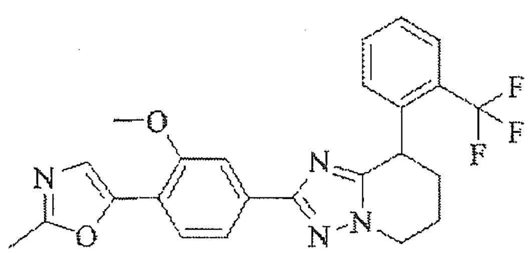

A mixture of 2-{4-chloro-1-[2-(trifluoromethyl)phenyl]butyl}-5-[3-methoxy-4-(2-methyl-1,3-oxazol-5-yl)phenyl]-1,3,4-oxadiazole (36.0 mg, 0.0732 mmol) and ammonium acetate (56.4 mg, 0.732 mmol) in acetic acid (1 mL) was stirred at 100°C for 5 hr. Ammonium acetate (56.4 mg, 0.732 mmol) was added and the mixture was further stirred at 120°C for 15 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous magnesium sulfate. The solvent was evaporated under reduced pressure. The residue was purified by silica gel column chromatography (ethyl acetate/hexane=4/6 - 8/2), and recrystallized from ethyl acetate-hexane to give the title compound as a white solid (11.1 mg, 33%).
$^1H$ NMR ($CDCl_3$) δ: 1.88 - 2.06 (1 H, m), 2.06 - 2.35 (2 H, m), 2.37 - 2.50 (1 H, m), 2.52 (3 H, s), 4.01 (3 H, s), 4.37 (2 H, t, J=6.0 Hz), 4.75 (1 H, t, J=7.0 Hz), 7.00 (1 H, d, J=7.5 Hz), 7.32 - 7.51 (3 H, m), 7.59 - 7.79 (4 H, m). MS(ESI):455 $[M+H]^+$.

Example 13

3-[3-methoxy-4-(2-methyl-1,3-oxazol-5-yl)phenyl]-8-[4-(trifluoromethyl)phenyl]-5,6,7,8-tetrahydro[1,2,4]triazolo[4,3-a]pyridine

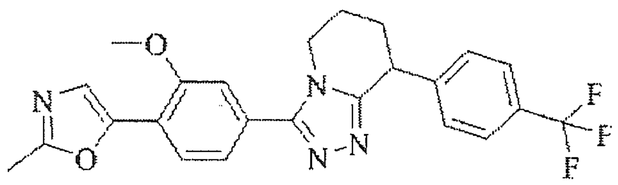

A mixture of 2-{4-chloro-1-[4-(trifluoromethyl)phenyl]butyl}-5-[3-methoxy-4-(2-methyl-1,3-oxazol-5-yl)phenyl]-1,3,4-oxadiazole (108 mg, 0.220 mmol) and sodium azide (28.5 mg, 0.440 mmol) in DMSO (1.1 mL) was stirred at 70˚C for 16 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.1 mL), water (0.11 mL) and triphenylphosphine (126 mg, 0.480 mmol) were added, and the mixture was stirred at 60˚C for 1 hr. The solvent was evaporated under reduced pressure, acetic acid (1.1 mL) was added to the residue, and the mixture was stirred at 120˚C for 1.5 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by NH silica gel column chromatography (ethyl acetate/hexane=50/50 - 90/10), and recrystallized from ethyl acetate-hexane to give the title compound as a white solid (62.1 mg, 62%).
$^1$H NMR ($CDCl_3$) δ: 1.95 - 2.25 (3 H, m), 2.32 - 2.48 (1 H, m), 2.56 (3 H, s), 4.05 (3 H, s), 4.20 (2 H, t, J=5.5 Hz), 4.52 (1 H, t, J=6.8 Hz), 7.29 (1 H, dd, J=8.3, 1.5 Hz), 7.37 (2 H, d, J=8.3 Hz), 7.47 - 7.53 (2 H, m), 7.61 (2 H, d, J=7.9 Hz), 7.85 (1 H, d, J=7.9 Hz). MS (ESI) :455 $[M+H]^+$.

Example 14

8-(2,3-dichlorophenyl)-3-[3-methoxy-4-(2-methyl-1,3-oxazol-5-yl)phenyl]-5,6,7,8-tetrahydro[1,2,4]triazolo[4,3-a]pyridine

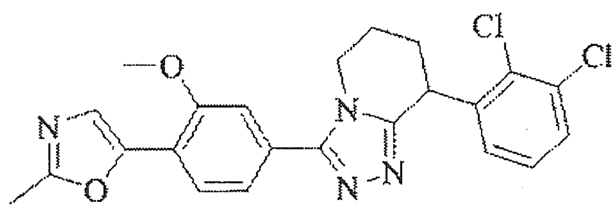

A mixture of 2-[4-chloro-1-(2,3-dichlorophenyl)butyl]-5-[3-methoxy-4-(2-methyl-1,3-oxazol-5-yl)phenyl]-1,3,4-oxadiazole (89 mg, 0.18 mmol) and sodium azide (24 mg, 0.36 mmol) in DMSO (1.0 mL) was stirred at 70˚C for 2 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.0 mL), water (0.10 mL) and triphenylphosphine (95 mg, 0.36 mmol) were added, and the mixture was stirred at 60˚C for 1 hr. The solvent was evaporated under reduced pressure, acetic acid (2.0 mL) was added to the residue, and the mixture was stirred at 140˚C for 11 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 95/5), and further separated by HPLC. Ethyl acetate and saturated aqueous sodium hydrogen carbonate were added, and the aqueous layer was extracted with ethyl acetate. The organic layers were combined, washed with saturated brine and dried over anhydrous magnesium sulfate. The solvent was evaporated under reduced pressure. The residue solidified from hexane-ethyl acetate to give the title compound as a colorless amorphous solid (11.5 mg, 14%).
$^1$H NMR ($CDC1_3$) 5: 2.03 - 2.20 (3 H, m), 2.40 - 2.48 (1 H, m), 2.58 (3 H, s), 4.07 (3 H, s), 4.22 (2 H, t, J=5.7 Hz), 4.96 (1 H, t, J=6.6 Hz), 6.92 (1 H, d, J=6.6 Hz), 7.16 (1 H, dd, J=7.8 Hz, 7.8 Hz), 7.32 (1 H, dd, J= 1.2 Hz, 8.1 Hz), 7.42 (1 H, dd, J=1.2 Hz, 8.1 Hz), 7.53 (2 H, s), 7.87 (1 H, d, J=7.8 Hz). MS(ESI):455 $[M+H]^+$.

Example 15

9-(3,4-dichlorophenyl)-3-[3-methoxy-4-(2-methyl-1,3-oxazol-5-yl)phenyl]-6,7,8,9-tetrahydro-5H-[1,2,4]triazolo[4,3-a]azepine

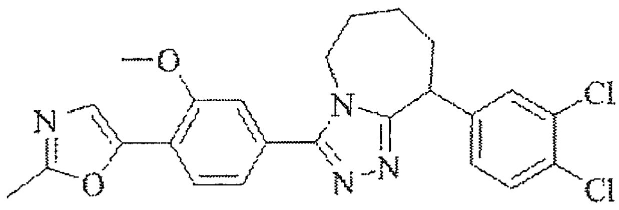

A mixture of 2-[5-chloro-1-(3,4-dichlorophenyl)pentyl]-5-[3-methoxy-4-(2-methyl-1,3-oxazol-5-yl)phenyl]-1,3,4-oxadiazole (0.13 g, 0.28 mmol) and sodium azide (33 mg, 0.51 mmol) in DMSO (1.3 mL) was stirred at 70˚C for 2 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.3 mL), water (0.13 mL) and triphenylphosphine (0.14 g, 0.51 mmol) were added, and the mixture was stirred at 60˚C for 1 hr. The solvent was evaporated under reduced pressure, acetic acid (2.6 mL) was added to the residue, and the mixture was stirred at 120˚C for 1 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. To the residue was added acetic acid (2.6 mL), and the mixture was stirred at 140˚C for 12 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 95/5) to give the title compound as a pale yellow amorphous solid (2.8 mg, 2%).
$^1$H NMR ($CDC1_3$) δ: 1.75 - 2.19 (5 H, m), 2.29 - 2.42 (1 H, m), 2.57 (3 H, s), 3.92 - 4.17 (2 H, m), 4.03 (3 H, s), 4.47 - 4.57 (1 H, m), 7.10 (2 H, d, J=8.4 Hz), 7.30 (1 H, s), 7.36 (1 H, s), 7.46 (1 H, d, J=8.4 Hz), 7.51 (1 H, s), 7.86 (1 H, d, J=8.1 Hz). MS(ESI):469 $[M+H]^+$.

Example 16

8-(benzyloxy)-3-[3-methoxy-4-(2-methyl-1,3-oxazol-5-yl)phenyl]-5,6,7,8-tetrahydro[1,2,4]triazolo[4,3-a]pyridine

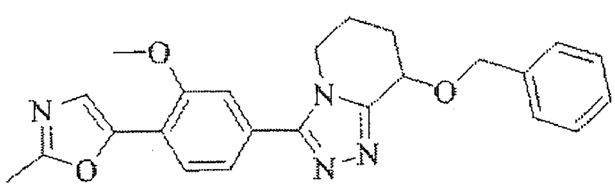

A mixture of 2-[1-(benzyloxy)-4-chlorobutyl]-5-[3-methoxy-4-(2-methyl-1,3-oxazol-5-yl)phenyl]-1,3,4-oxadiazole (327 mg, 0.721 mmol) and sodium azide (93.7 mg, 1.44 mmol) in DMSO (3.5 mL) was stirred at 70˚C for 12 hr. After cooling to room temperature, water was added to the reaction mixture, and the mixture was extracted with ethyl acetate. The obtained extract was washed with water and saturated brine, and dried over anhydrous sodium sulfate. The solvent was evaporated under reduced pressure. The residue was diluted with THF (3.5 mL), water (0.35 mL) and triphenylphosphine (378 mg, 1.44 mmol) were added, and the mixture was stirred at 60˚C for 1.5 hr. The solvent was

evaporated under reduced pressure, to the residue was added acetic acid (2.8 mL) and the mixture was heated under reflux for 2 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous sodium sulfate. The solvent was evaporated under reduced pressure. The residue was purified by silica gel column chromatography (methanol/ethyl acetate=0/100 - 20/80) to give the title compound as a white solid (250 mg, 83%).
$^1$H NMR ($CDCl_3$) δ: 1.88 - 2.06 (2 H, m), 2.26 - 2.48 (2 H, m), 2.55 (3 H, s), 3.89-4.01 (1 H, m), 4.03 (3 H, s), 4.16 - 4.30 (1 H, m), 4.79 - 5.01 (3 H, m), 7.23 - 7.54 (8 H, m), 7.83 (1 H, d, J=8.0 Hz). MS(ESI):417 $[M+H]^+$.

Example 17

8-(3,4-dichlorophenyl)-3-[3-methoxy-4-(4-methyl-1H-imidazol-1-yl)phenyl]-5,6,7,8-tetrahydro[1,2,4]triazolo[4,3-a]pyridine

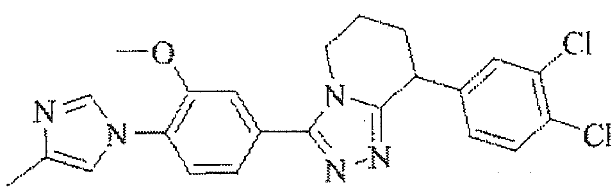

To a mixture of tert-butyl 2-{[3-methoxy-4-(4-methyl-1H-imidazol-1-yl)phenyl]carbonyl}hydrazinecarboxylate (92.5 mg, 0.267 mmol) in methanol (1.0 mL) was added hydrogen chloride 4M ethyl acetate solution (4M ethyl acetate solution, 2.5 mL), and the mixture was stirred at room temperature for 2 hr. The reaction solution was filtered, and the obtained solid was suspended in DMF (2.0 mL). 5-Chloro-2-(3,4-dichlorophenyl)pentanoic acid (82.4 mg, 0.293 mmol), triethylamine (136 μL, 0.976 mmol) and HATU (111 mg, 0.293 mmol) were added, and the mixture was stirred at room temperature for 1 hr, diluted with ethyl acetate, and washed with water and saturated brine. This was dried over anhydrous sodium sulfate and the solvent was evaporated under reduced pressure. The residue was diluted with acetonitrile (2.4 mL), and carbon tetrachloride (46.9 μL, 0.488 mmol) and triphenylphosphine (256 mg, 0.976 mmol) were added. The mixture was stirred at 80˚C for 2 hr, and the solvent was evaporated under reduced pressure. The residue was diluted with ethyl acetate, washed with saturated aqueous sodium hydrogen carbonate solution and saturated brine, dried over anhydrous sodium sulfate, and the solvent was evaporated. The residue was diluted with DMSO (1.2 mL), sodium azide (31.7 mg, 0.488 mmol) was added, and the mixture was stirred at 70˚C for 12 hr. Water was added to the reaction solution, and the mixture was extracted with ethyl acetate. The obtained extract was washed with water and saturated brine, and dried over anhydrous sodium sulfate. The solvent was evaporated under reduced pressure. The residue was diluted with THF (1.2 mL), water (0.12 mL) and triphenylphosphine (128 mg, 0.488 mmol) were added, and the mixture was stirred at 60˚C for 1.5 hr. The solvent was evaporated under reduced pressure, acetic acid (1.0 mL) was added to the residue, and the mixture was heated under reflux for 2 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous sodium sulfate. The solvent was evaporated under reduced pressure. The residue was purified by NH silica gel column chromatography (ethyl acetate/hexane=40/60 - 100/0) to give the title compound as a yellow oil (47.7 mg, 39%).
$^1$H NMR ($CDCl_3$) δ: 1.98 - 2.08 (3 H, m), 2.12 - 2.25 (1 H, m), 2.32 (3 H, s), 2.36 - 2.45 (1 H, m), 3.95 (3 H, s), 4.15 - 4.22 (2 H, m), 4.37 - 4.49 (1 H, m), 7.11 (1 H, dd, J=8.3, 2.2 Hz), 7.26 - 7.30 (1 H, m), 7.34 (1 H, d, J=2.2 Hz), 7.38 (1 H, d, J=8.0 Hz), 7.42 (1 H, d, J=8.3 Hz), 7.56 (1 H, d, J=1.6 Hz), 7.77 (1 H, d, J=1. 1 Hz). MS(ESI):454 $[M+H]^+$.

Example 18

(+)-3-[3-methoxy-4-(2-methyl-1,3-oxazol-5-yl)phenyl]-8-[2-(trifluoromethyl)phenyl]-5,6,7,8-tetrahydro[1,2,4]triazolo [4,3-a]pyridine

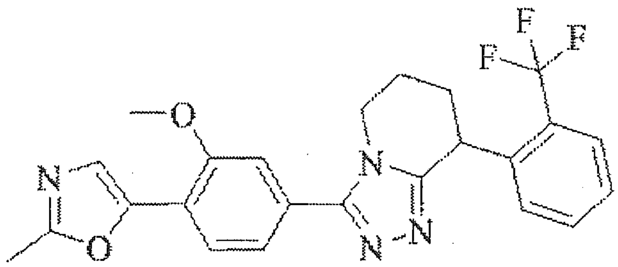

3-[3-methoxy-4-(2-methyl-1,3-oxazol-5-yl)phenyl]-8-[2-(trifluoromethyl)phenyl]-5,6,7,8-tetrahydro[1,2,4]triazolo[4,3-a]pyridine (250 mg) was fractionated by using high performance liquid chromatography [device: K-Prep (manufactured by YMC), column: CHIRALPAK AD (50 mmID×500 mmL manufactured by DAICEL CHEMICAL INDUSTRIES, LTD.), mobile phase: A) hexane 100%, B)2-propanol=100%, mixing ratio: A/B=700/300, flow rate: 80 mL/min, column temperature: 30°C, sample injection volume: 100 mg (dissolved in mobile phase (40 mL))]. A fraction solution containing an optically active form having a shorter retention time in the above-mentioned high performance liquid chromatography conditions was concentrated to give the title compound (116 mg, >99.9% ee). The enantiomer excess (ee) was measured by high performance liquid chromatography [column: CHIRALPAK AD (4.6 mmIDx250 mmL manufactured by DAICEL CHEMICAL INDUSTRIES, LTD.), mobile phase: hexane/2-propanol=700/300, flow rate: 1.0 mL/min, column temperature: 30°C, sample concentration: 0.5 mg/mL (mobile phase), injection volume: 10 $\mu$L)]. Recrystallization from ethyl acetate-hexane gave the title compound as a white solid (99.8 mg).
$[\alpha]_D^{20}$: +111.8°(c 0.479, methanol),
$^1$H NMR ($CDCl_3$) $\delta$:1.83 - 2.16 (2 H, m), 2.17 - 2.33 (1 H, m), 2.39 - 2.52 (1 H, m), 2.56 (3 H, s), 4.04 (3 H, s), 4.08 - 4.33 (2 H, m), 4.76 (1 H, dd, J=9.8, 5.9 Hz), 7.17 (1 H, d, J=7.7 Hz), 7.29 (1 H, dd, J=8.1, 1.5 Hz), 7.33 - 7.43 (1 H, m), 7.43 - 7.54 (3 H, m), 7.71 (1 H, d, J=8.0 Hz), 7.84 (1 H, d, J=8.2 Hz). MS (ESI) : 455 $[M+H]^+$.

Example 19

(-)-3-[3-methoxy-4-(2-methyl-1,3-oxazol-5-yl)phenyl]-8-[2-(trifluoromethyl)phenyl]-5,6,7,8-tetrahydro[1,2,4]triazolo [4,3-a]pyridine

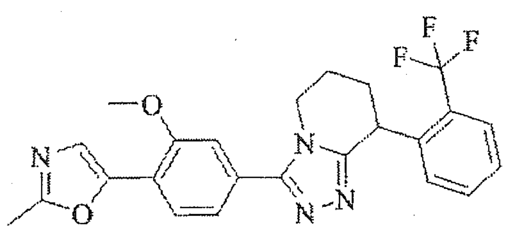

3-[3-methoxy-4-(2-methyl-1,3-oxazol-5-yl)phenyl]-8-[2-(trifluoromethyl)phenyl]-5,6,7,8-tetrahydro[l,2,4]triazolo[4,3-a]pyridine (250 mg) was fractionated by using high performance liquid chromatography [device: K-Prep (manufactured by YMC), column: CHIRALPAK AD(50 mmIDx500 mmL manufactured by DAICEL CHEMICAL INDUSTRIES, LTD.), mobile phase: A) hexane 100%, B) 2-propanol=100%, mixing ratio: A/B=700/300, flow rate: 80 mL/min, column temperature: 30°C, sample injection volume: 100 mg (dissolved in mobile phase (40 mL))]. A fraction solution containing an optically active form having a longer retention time in the above-mentioned high performance liquid chromatography conditions was concentrated to give the title compound (111 mg, >99.7% ee). The enantiomer excess (ee) was measured by high performance liquid chromatography [column: CHIRALPAK AD (4.6 mmIDx250 mmL manufactured by DAICEL CHEMICAL INDUSTRIES, LTD.), mobile phase: hexane/2-propanol=700/300, flow rate: 1.0 mL/min, column temperature: 30°C, sample concentration: 0.5 mg/mL (mobile phase), injection volume: 10 $\mu$L)]. Recrystallization from ethyl acetate-hexane gave the title compound as a white solid (95.8 mg).
$[\alpha]_D^{20}$: -110.7°(c 0.464, methanol),
$^1$H NMR ($CDCl_3$) $\delta$:1.82 - 2.15 (2 H, m), 2.17 - 2.32 (1 H, m), 2.38 - 2.52 (1 H, m), 2.56 (3 H, s), 4.04 (3 H, s), 4.08 -

4.30 (2 H, m), 4.76 (1 H, dd, J=9.6, 5.8 Hz), 7.17 (1 H, d, J=7.4 Hz), 7.29 (1 H, dd, J=8.0, 1.6 Hz), 7.33 - 7.43 (1 H, m), 7.43 - 7.54 (3 H, m), 7.72 (1 H, d, J=8.2 Hz), 7.84 (1 H, d, J=8.0 Hz). MS(ESI):455 $[M+H]^+$.

Example 20

8-(2,4-difluorophenyl)-3-[3-methoxy-4-(2-methyl-1,3-oxazol-5-yl)phenyl]-5,6,7,8-tetrahydro[1,2,4]triazolo[4,3-a]pyridine

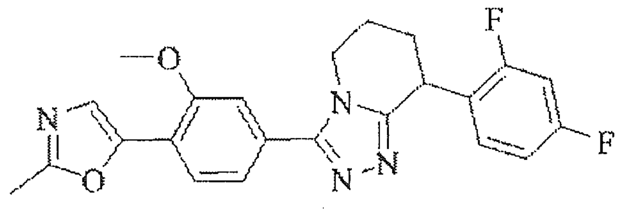

A mixture of 2-[4-chloro-1-(2,4-difluorophenyl)butyl]-5-[3-methoxy-4-(2-methyl-1,3-oxazol-5-yl)phenyl]-1,3,4-oxadiazole (139 mg, 0.303 mmol) and sodium azide (39.4 mg, 0.605 mmol) in DMSO (1.5 mL) was stirred at 70˚C for 15.5 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.5 mL), water (0.15 mL) and triphenylphosphine (159 mg, 0.605 mmol) were added, and the mixture was stirred at 60˚C for 2 hr. The solvent was evaporated under reduced pressure, acetic acid (1.5 mL) was added to the residue, and the mixture was stirred at 130˚C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 90/10), and recrystallized from ethyl acetate-hexane to give the title compound as a white solid (109 mg, 85%).
$^1$H NMR ($CDCl_3$) δ: 1.95 - 2.07 (2 H, m), 2.11 - 2.22 (1 H, m), 2.31 - 2.44 (1 H, m), 2.56 (3 H, s), 4.05 (3 H, s), 4.07 - 4.26 (2 H, m), 4.65 (1 H, t, J=7.3 Hz), 6.76 - 6.91 (2 H, m), 6.99 - 7.15 (1 H, m), 7.29 (1 H, dd, J=7.9, 1.5 Hz), 7.45 - 7.52 (2 H, m), 7.85 (1 H, d, J=8.3 Hz). MS(ESI):423 $[M\pm H]^+$.

Example 21

8-(2-chloro-4-fluorophenyl)-3-[3-methoxy-4-(2-methyl-1,3-oxazol-5-yl)phenyl]-5,6,7,8-tetrahydro[1,2,4]triazolo[4,3-a]pyridine

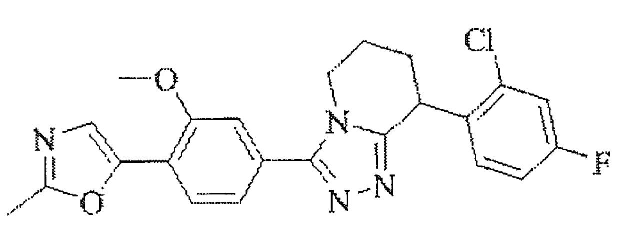

A mixture of 2-[4-chloro-1-(2-chloro-4-fluorophenyl)butyl]-5-[3-methoxy-4-(2-methyl-1,3-oxazol-5-yl)phenyl]-1,3,4-oxadiazole (78.5 mg, 0.165 mmol) and sodium azide (21.4 mg, 0.329 mmol) in DMSO (1.0 mL) was stirred at 70˚C for 15.5 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.0 mL), water (0.10

mL) and triphenylphosphine (86.3 mg, 0.329 mmol) were added, and the mixture was stirred at 60°C for 2 hr. The solvent was evaporated under reduced pressure, acetic acid (0.8 mL) was added to the residue, and the mixture was stirred at 130°C for 1.5 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 90/10), and recrystallized from ethyl acetate-hexane to give the title compound as a white solid (50.8 mg, 700).
$^1$H NMR ($CDCl_3$) δ: 0.31 - 0.64 (3 H, m), 0.72 - 0.91 (1 H, m), 0.99 (3 H, s), 2.48 (3 H, s), 2.57 - 2.70 (2 H, m), 3.20 - 3.34 (1 H, m), 5.33 - 5.48 (2 H, m), 5.61 (1 H, dd, J=8.3, 2.6 Hz), 5.71 - 5.76 (1 H, m), 5.91 - 5.96 (2 H, m), 6.29 (1 H, d, J=7.9 Hz). MS (ESI):439 $[M+H]^+$.

Example 22

8-(3,4-difluorophenyl)-3-[3-methoxy-4-(2-methyl-1,3-oxazol-5-yl)phenyl]-5,6,7,8-tetrahydro[1,2,4]triazolo[4,3-a]pyridine

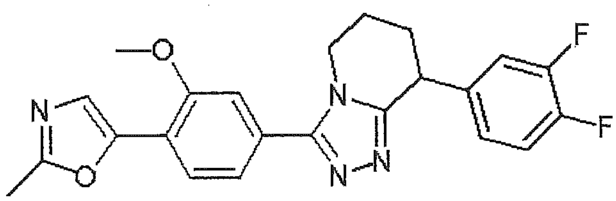

A mixture of 2-[4-chloro-1-(3,4-difluorophenyl)butyl]-5-[3-methoxy-4-(2-methyl-1,3-oxazol-5-yl)phenyl]-1,3,4-oxadiazole (58 mg, 0.13 mmol) and sodium azide (17 mg, 0.25 mmol) in DMSO (1.0 mL) was stirred at 70°C for 16 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.0 mL), water (0.10 mL) and triphenylphosphine (67 mg, 0.25 mmol) were added, and the mixture was stirred at 60°C for 1.5 hr. The solvent was evaporated under reduced pressure, acetic acid (2.0 mL) was added to the residue, and the mixture was heated under reflux for 1 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 90/10) to give the title compound as a white solid (5.8 mg, 11%).
$^1$H NMR ($CDCl_3$) δ: 2.02 - 2.22 (3 H, m), 2.35 - 2.43 (1 H, m), 2.57 (3 H, s), 4.06 (3 H, s), 4.19 (2 H, t, J=4.5 Hz), 4.45 (1 H, t, J=6.6 Hz), 6.98 - 7.20 (3 H, m), 7.31 (1 H, s), 7.51 (2 H, d, J= 7.5 Hz), 7.86 (1 H, d, J=8.1 Hz). MS (ESI) :423 $[M+H]^+$.

Example 23

3-[3-methoxy-4-(2-methyl-1,3-oxazol-5-yl)phenyl]-8-[2-(trifluoromethoxy)phenyl]-5,6,7,8-tetrahydro[1,2,4]triazolo [4,3-a]pyridine

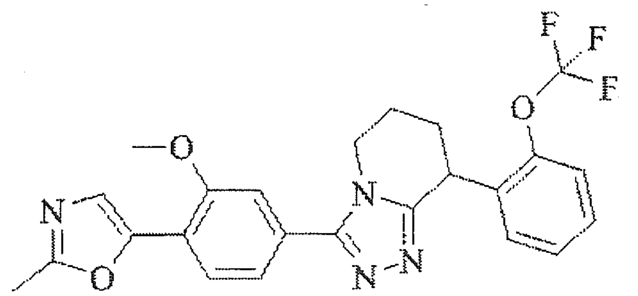

A mixture of 2-{4-chloro-1-[2-(trifluoromethoxy)phenyl]butyl}-5-[3-methoxy-4-(2-methyl-1,3-oxazol-5-yl)phenyl]-1,3,4-oxadiazole (144 mg, 0.284 mmol) and sodium azide (36.9 mg, 0.568 mmol) in DMSO (1.4 mL) was stirred at 70˚C for 15.5 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.4 mL), water (0.14 mL) and triphenylphosphine (149 mg, 0.568 mmol) were added, and the mixture was stirred at 60˚C for 2 hr. The solvent was evaporated under reduced pressure, acetic acid (1.0 mL) was added to the residue, and the mixture was stirred at 130˚C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 90/10), and recrystallized from ethyl acetate-hexane to give the title compound as a white solid (113 mg, 85%).
$^1$H NMR ($CDCl_3$) δ: 1.94 - 2.08 (2 H, m), 2.11 - 2.22 (1 H, m), 2.29 - 2.46 (1 H, m), 2.56 (3 H, s), 4.04 (3 H, s), 4.13 - 4.26 (2 H, m), 4.73 (1 H, t, J=7.6 Hz), 7.10 - 7.16 (1 H, m), 7.19 - 7.25 (1 H, m), 7.27 - 7.34 (3 H, m), 7.48 (1 H, d, J=1.5 Hz), 7.51 (1 H, s), 7.85 (1 H, d, J=8.0 Hz). MS(ESI):471 $[M+H]^+$.

Example 24

8-[4-fluoro-3-(trifluoromethoxy)phenyl]-3-[3-methoxy-4-(2-methyl-1,3-oxazol-5-yl)phenyl]-5,6,7,8-tetrahydro[1,2,4]triazolo[4,3-a]pyridine

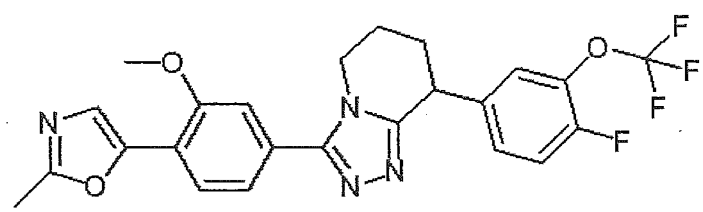

A mixture of 2-{4-chloro-l-[4-fluoro-3-(trifluoromethoxy)phenyl]butyl}-5-[3-methoxy-4-(2-methyl-1,3-oxazol-5-yl)phenyl]-1,3,4-oxadiazole (68 mg, 0.13 mmol) and sodium azide (17 mg, 0.25 mmol) in DMSO (1.0 mL) was stirred at 70˚C for 4 hr. After cooling to room temperature, water was added to the reaction mixture, and the mixture was extracted with ethyl acetate. The obtained extract was washed with water and saturated brine, and dried over anhydrous magnesium sulfate. The solvent was evaporated under reduced pressure. The residue was diluted with THF (1.0 mL), water (0.10 mL) and triphenylphosphine (68 mg, 0.26 mmol) were added, and the mixture was stirred at 60˚C for 3.5 hr. The solvent was evaporated under reduced pressure, acetic acid (2.0 mL) was added to the residue, and the mixture was heated under reflux for 1 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The solvent was evaporated under reduced pressure. The residue was purified by silica gel column chromatography (ethyl acetate/methanol=100/0 - 95/5) to give the title compound as a pale yellow white solid (17 mg, 27%).
$^1$H NMR ($CDCl_3$) δ: 1.98 - 2.24 (3 H, m), 2.37 - 2.45 (1 H, m), 2.57 (3 H, s), 4.06 (3 H, s), 4.17 - 4.20 (2 H, m), 4.45 (1 H, t, J=6.6 Hz), 7.19 (2 H, d, J=7.5 Hz), 7.24 (1 H, s), 7.31 (1 H, s), 7.51 (2 H, d, J=6.3 Hz), 7.86 (1 H, d, J=8.1 Hz). MS(ESI):489 [M+H]+.

Example 25

8-(3,4-dichlorophenoxy)-3-[3-methoxy-4-(2-methyl-1,3-oxazol-5-yl)phenyl]-5,6,7,8-tetrahydro[1,2,4]triazolo[4,3-a]pyridine

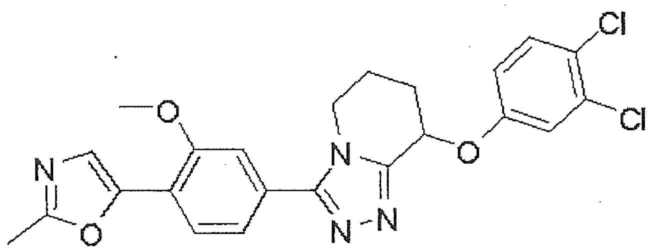

To a mixture of 8-(benzyloxy)-3-[3-methoxy-4-(2-methyl-1,3-oxazol-5-yl)phenyl]-5,6,7,8-tetrahydro[1,2,4]triazolo[4,3-a]pyridine (41.6 mg, 0.100 mmol) in toluene (0.6 mL) was added a dichloromethane solution (1.0 M, 0.4 mL, 0.400 mmol) of tribromide boron at room temperature, and the mixture was stirred for 15 hr. The reaction solution was diluted with ethyl acetate, washed with saturated aqueous sodium hydrogen carbonate solution and saturated brine, dried over anhydrous sodium sulfate and the solvent was evaporated under reduced pressure. The residue was diluted with THF (1.0 mL), a toluene solution (2.2 M, 68.2 μL, 0.150 mmol) of 3,4-dichlorophenol (24.5 mg, 0.150 mmol), triphenylphosphine (39.3 mg, 0.150 mmol) and diethyl azodicarboxylate was added, and the mixture was stirred at room temperature for 30 min. The reaction solution was concentrated, and the residue was purified by silica gel column chromatography (methanol/ethyl acetate=0/100 - 10/90) to give the title compound as a white solid (8.0 mg, 17%).
$^1$H NMR ($CDCl_3$) δ: 2.07 - 2.19 (2 H, m), 2.40 - 2.53 (2 H, m), 2.56 (3 H, s), 3.94 - 4.10 (4 H, m), 4.23 - 4.38 (1 H, m), 5.65 (1 H, t, J=3.3 Hz), 7.16 (1 H, dd, J=8.8, 2.8 Hz), 7.26 (1 H, d, J=8.2 Hz), 7.30 - 7.40 (2 H, m), 7.46 (1 H, s), 7.50 (1 H, s), 7.83 (1 H, d, J=8.0 Hz). MS(ESI):471 [M+H]+.

Example 26

8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine

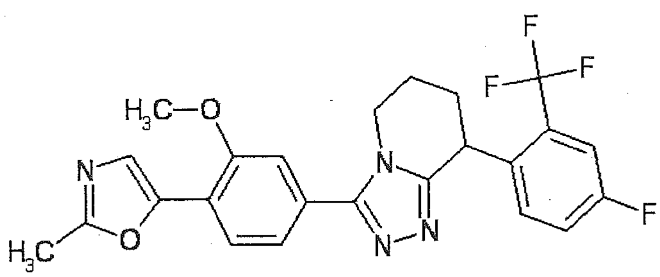

A) 5-chloro-2-[4-fluoro-2-(trifluoromethyl)phenyl]pentanoic acid

To a solution of [4-fluoro-2-(trifluoromethyl)phenyl]acetic acid (2.50 g) in THF (60 mL) was added dropwise n-butyllithium 1.6 M hexane solution (14.1 mL) under an argon atmosphere at -78°C, and the mixture was stirred while gradually raising the temperature over 15 min. 1-Bromo-3-chloropropane (1.17 mL) was added, and the mixture was stirred at room temperature overnight. To the reaction mixture was added 1 M aqueous sodium hydroxide solution, and the organic layer was extracted with 1 M aqueous sodium hydroxide solution. The extract was acidified with 6 M hydrochloric acid, and the mixture was extracted with ethyl acetate. The extract was washed with saturated brine, and dried

over anhydrous magnesium sulfate. The solvent was evaporated under reduced pressure. The residue was purified by silica gel column chromatography (ethyl acetate/hexane) to give the title compound (1.46 g).
$^1$H NMR ($CDCl_3$) δ: 1.54 - 1.75 (1H, m), 1.75 - 2.05 (2H, m), 2.15 - 2.36 (1H, m), 3.51 (2H, t, J = 6.3 Hz), 4.03 (1H, t, J = 7.3 Hz), 7.20 - 7.31 (1H, m), 7.38 (1H, dd, J = 9.1, 2.7 Hz), 7.59 (1H, dd, J = 8.7, 5.4 Hz).

B) 2-{4-chloro-1-[4-fluoro-2-(trifluoromethyl)phenyl]butyl}-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (0.63 g), 5-chloro-2-[4-fluoro-2-(trifluoromethyl)phenyl]pentanoic acid (0.80 g) and triethylamine (1.23 mL) in DMF (10 mL) was added HATU (1.02 g) at 0˚C, and the mixture was stirred at room temperature for 1.5 hr. The reaction mixture was diluted with water, and the mixture was extracted with ethyl acetate. The extract was washed with water and saturated brine, and dried over anhydrous magnesium sulfate. The solvent was evaporated under reduced pressure. The residue was dissolved in acetonitrile (10 mL), and carbon tetrachloride (408 μL) and triphenylphosphine (2.34 g) were added. The reaction mixture was stirred at 80˚C for 2 hr, and the solvent was evaporated under reduced pressure. The residue was diluted with ethyl acetate and water, and the mixture was extracted with ethyl acetate. The extract was washed with saturated brine, and dried over anhydrous magnesium sulfate. The solvent was evaporated under reduced pressure. The residue was suspended in diethyl ether and filtered. The solvent was evaporated under reduced pressure. The residue was purified by silica gel column chromatography (ethyl acetate/hexane) to give the title compound (821 mg).
$^1$H NMR ($CDCl_3$) δ: 1.67 - 1.87 (1H, m), 1.88 - 2.04 (1H, m), 2.18 - 2.38 (1H, m), 2.43 - 2.63 (4H, m), 3.57 (2H, t, J = 6.5 Hz), 4.03 (3H, s), 4.67 (1H, t, J = 7.6 Hz), 7.21 - 7.33 (1H, m), 7.43 (1H, dd, J = 8.8, 2.7 Hz), 7.51 (1H, s), 7.54 - 7.71 (3H, m), 7.80 (1H, d, J = 8.0 Hz).

C) 8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine

A mixture of 2-{4-chloro-1-[4-fluoro-2-(trifluoromethyl)phenyl]butyl}-5-[3-methoxy-4-(2-methyl-1,3-oxazol-5-yl) phenyl]-1,3,4-oxadiazole (821 mg) and sodium azide (209 mg) in DMSO (10 mL) was stirred at 70˚C for 3 hr. After cooling to room temperature, the reaction mixture was diluted with ethyl acetate, washed with water and saturated brine, and dried over anhydrous magnesium sulfate. The solvent was evaporated under reduced pressure. The residue was dissolved in THF (10 mL), water (1.0 mL) and triphenylphosphine (845 mg) were added, and the mixture was stirred at 60˚C for 1 hr. The solvent was evaporated under reduced pressure, and to the residue was added acetic acid (10 mL), and the mixture was stirred at 110˚C for 1.5 hr. The solvent was evaporated under reduced pressure, and to the residue was added saturated aqueous sodium hydrogen carbonate solution, and the mixture was extracted with ethyl acetate. The extract was washed with saturated brine, and dried over anhydrous sodium sulfate. The solvent was evaporated under reduced pressure. The residue was purified by silica gel column chromatography (methanol/ethyl acetate), and washed with hexane to give the title compound (618 mg).
$^1$H NMR ($CDCl_3$) δ: 1.75 - 1.97 (1H, m), 1.97 - 2.16 (1H, m), 2.16 - 2.35 (1H, m), 2.36 - 2.52 (1H, m), 2.56 (3H, s), 4.04 (3H, s), 4.08 - 4.21 (1H, m), 4.21 - 4.39 (1H, m), 4.71 (1H, dd, J = 9.9, 5.8 Hz), 7.19 (2H, d, J = 6.3 Hz), 7.28 (1H, dd, J = 8.2, 1.4 Hz), 7.42 (1H, d, J = 9.1 Hz), 7.49 (2H, d, J = 11.5 Hz), 7.85 (1H, d, J = 8.0 Hz). MS(ESI):473.3 $[M+H]^+$.

Example 27

8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine

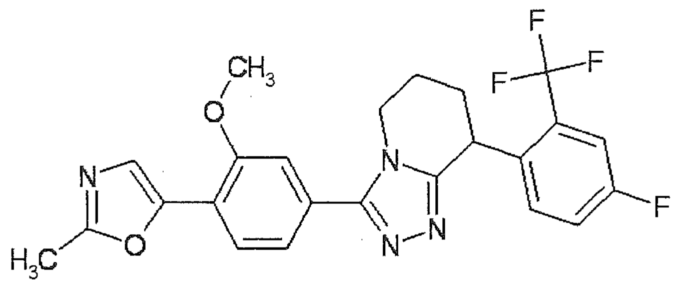

A racemate (585 mg) of 8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine was separated by HPLC (column: CHIRALPAK AD (trade name), 50 mmID$_X$500 mmL, manufactured by DAICEL CHEMICAL INDUSTRIES, LTD., mobile phase: hexane/2-propanol = 700/300) to give the title compound with a shorter retention time (294 mg).
$^1$H NMR ($CDCl_3$) δ: 1.79 - 1.98 (1H, m), 1.98 - 2.17 (1H, m), 2.17 - 2.34 (1H, m), 2.36 - 2.52 (1H, m), 2.56 (3H, s), 4.04 (3H, s), 4.08 - 4.22 (1H, m), 4.22 - 4.33 (1H, m), 4.71 (1H, dd, J = 10.0, 5.9 Hz), 7.19 (2H, dd, J = 6.4, 1. 5 Hz), 7.28 (1H, dd, J = 8.1, 1.5 Hz), 7.42 (1H, d, J = 8.8 Hz), 7.45 - 7.53 (2H, m), 7.85 (1H, d, J = 8.0 Hz). MS(ESI):473.3 $[M+H]^+$.

Example 28

8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine

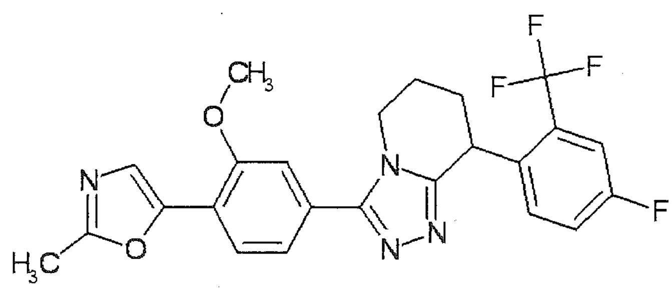

A racemate (585 mg) of 8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine was separated by HPLC (column: CHIRALPAK AD (trade name), 50 mmIDx500 mmL, manufactured by DAICEL CHEMICAL INDUSTRIES, LTD., mobile phase: hexane/2-propanol = 700/300) to give the title compound with a longer retention time (300 mg).
$^1$H NMR ($CDCl_3$) δ: 1.76 - 1.98 (1H, m), 1.98 - 2.19 (1H, m), 2.19 - 2.35 (1H, m), 2.35 - 2.52 (1H, m), 2.56 (3H, s), 4.04 (3H, s), 4.08 - 4.22 (1H, m), 4.22 - 4.34 (1H, m), 4.71 (1H, dd, J = 9.9, 6.0 Hz), 7. 13 - 7.23 (2 H, m), 7.28 (1 H, dd, J = 8.1, 1.5 Hz), 7.43 (1H, d, J = 8.8 Hz), 7.45 - 7.54 (2H, m), 7.85 (1H, d, J = 8.2 Hz). MS(ESI):473.3 $[M+H]^+$.

Example 29

3-[3-methoxy-4-(2-methyl-1,3-oxazol-5-yl)phenyl]-8-[2-(1-methylethenyl)phenyl]-5,6,7,8-tetrahydro[1,2,4]triazolo [4,3-a]pyridine

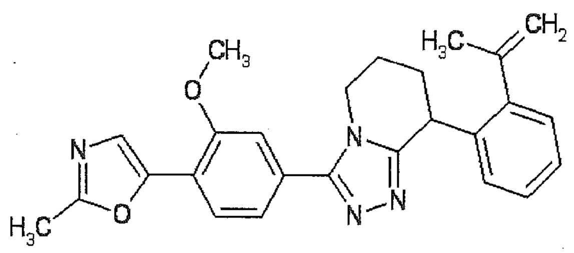

A mixture of 8-(2-bromophenyl)-3-[3-methoxy-4-(2-methyl-1,3-oxazol-5-yl)phenyl]-5,6,7,8-tetrahydro[1,2,4]triazolo[4,3-a]pyridine (Example 39; 227 mg), 4,4,5,5-tetramethyl-2-(1-methylethenyl)-1,3,2-dioxaborolane (0.138 mL), bis(triphenylphosphine)palladium dichloride (51.4 mg), potassium carbonate (202 mg), and water (0.12 mL) in dimethoxyethane (2.4 mL) was stirred at 95°C for 12 hr. After cooling to room temperature, water was added to the reaction mixture, and the mixture was extracted with ethyl acetate. The extract was dried over magnesium sulfate and the solvent was evaporated under reduced pressure. The residue was purified by silica gel column chromatography (ethyl acetate/hexane) to give the title compound (163 mg).
$^1$H NMR ($CDCl_3$) δ: 1.86 - 2.08 (2H, m), 2.12 - 2.40 (5H, m), 2.56 (3H, s), 4.03 (3H, s), 4.08 - 4.28 (2H, m), 4.60 - 4.68 (1H, m), 5.04 (1H, s), 5.27 (1H, s), 6.99 - 7.07 (1H, m), 7.16 - 7.25 (3H, m), 7.27 - 7.31 (1H, m), 7.46 - 7.53 (2H, m), 7.84 (1H, d, J = 8.3 Hz). MS(ESI):427.4 [M+H]$^+$.

Example 30

3-[3-methoxy-4-(2-methyl-1,3-oxazol-5-yl)phenyl]-8-[2-(1-methylethyl)phenyl]-5,6,7,8-tetrahydro[1,2,4]triazolo[4,3-a]pyridine

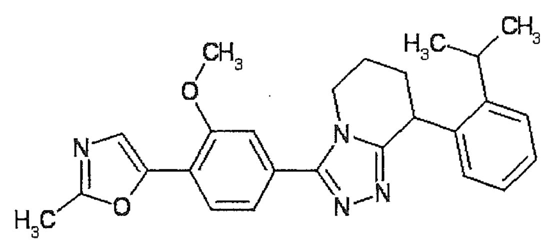

A mixture of 3-[3-methoxy-4-(2-methyl-1,3-oxazol-5-yl)phenyl]-8-[2-(1-methylethenyl)phenyl]-5,6,7,8-tetrahydro[1,2,4]triazolo[4,3-a]pyridine (Example 29; 84.0 mg), palladium hydroxide-carbon (16.8 mg) in ethanol (1.7 mL) was stirred under a hydrogen atmosphere at room temperature for 3 hr. The reaction mixture was filtered through celite and the filtrate was concentrated. The residue was washed with ethyl acetate to give the title compound (54.3 mg).
$^1$H NMR ($CDCl_3$) δ: 1.31 (3H, d, J = 6.8 Hz), 1.36 (3H, d, J = 6.8 Hz), 1.89 - 2.07 (2H, m), 2.12 - 2.26 (1H, m), 2.29 - 2.40 (1H, m), 2.56 (3H, s), 3.15 - 3.30 (1H, m), 4.05 (3H, s), 4.16 - 4.23 (2H, m), 4.72 - 4.78 (1H, m), 6.80 - 6.87 (1H, m), 7.05 - 7.14 (1H, m), 7.21 - 7.38 (3H, m), 7.51 (2H, s), 7.85 (1H, d, J = 7.9 Hz). MS(ESI):429.5 [M+H]$^+$.

Example 31

3-[3-methoxy-4-(2-methyl-1,3-oxazol-5-yl)phenyl]-8-[4-(methylsulfonyl)phenyl]-5,6,7,8-tetrahydro[1,2,4]triazolo[4,3-a]pyridine

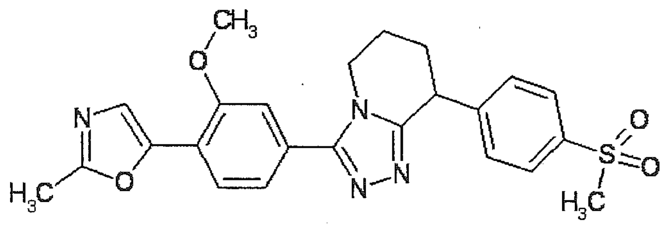

To a solution of 3-[3-methoxy-4-(2-methyl-1,3-oxazol-5-yl)phenyl]-8-[4-(methylsulfanyl)phenyl]-5,6,7,8-tetrahydro[1,2,4]triazolo[4,3-a]pyridine (Example 37; 160 mg) in acetonitrile (5.0 mL) was added m-chloroperbenzoic acid (274 mg) at 0˚C by small portions, and the mixture was stirred at the same temperature for 2 hr. The reaction mixture was diluted with saturated aqueous sodium hydrogen carbonate solution, and the mixture was extracted with ethyl acetate. The extract was washed with saturated aqueous sodium hydrogen carbonate solution and saturated brine, and dried over anhydrous magnesium sulfate. The solvent was evaporated under reduced pressure. The residue was purified by silica gel column chromatography (ethyl acetate/hexane). The obtained solid was recrystallized from ethyl acetate-hexane to give the title compound (66.8 mg) as a colorless solid.
$^1$H NMR ($CDCl_3$) δ: 2.02 - 2.28 (3H, m), 2.36 - 2.51 (1H, m), 2.56 (3H, s), 3.05 (3H, s), 4.05 (3H, s), 4.14 - 4.28 (2H, m), 4.48 - 4.59 (1H, m), 7.30 (1H, dd, J = 7.9, 1.5 Hz), 7.44 - 7.53 (4H, m), 7.86 (1H, d, J = 7.9 Hz), 7.91 - 7.97 (2H, m). MS (ESI):465.4 $[M+H]^+$.

Example 32

8-[4-chloro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine

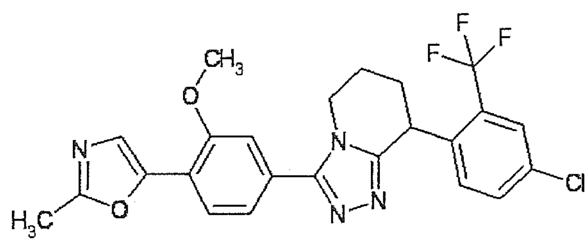

A) tert-butyl [4-chloro-2-(trifluoromethyl)phenyl]acetate

To a solution of [4-chloro-2-(trifluoromethyl)phenyl]acetic acid (1.00 g) in THF (10 mL) were added dropwise oxalyl chloride (0.395 mL) and DMF (0.0645 mL) at 0˚C, and the mixture was stirred at room temperature for 1 hr. The solvent was evaporated under reduced pressure, to a solution of the obtained residue in THF (3.0 mL) was added dropwise tert-butanol (3.0 mL) at 0˚C, and the mixture was stirred at room temperature for 1 hr. To the reaction solution was added dropwise triethylamine (0.584 mL) at 0˚C, diluted with water, and the mixture was extracted with ethyl acetate. The extract was washed with saturated brine, and dried over anhydrous magnesium sulfate. The solvent was evaporated under reduced pressure. The residue was purified by silica gel column chromatography (ethyl acetate/hexane) to give the title compound (832 mg).
$^1$H NMR ($CDCl_3$) δ: 1.43 (9H, s), 3. 62 - 3.79 (2H, m), 7.32 (1H, d, J = 8.3 Hz), 7.49 (1H, dd, J = 8.3, 2.3 Hz), 7.64 (1H, d, J = 2.3 Hz).

B) tert-butyl 5-chloro-2-[4-chloro-2-(trifluoromethyl)phenyl]pentanoate

To a solution of tert-butyl [4-chloro-2-(trifluoromethyl)phenyl]acetate (819 mg) in DMF (10 mL) was added sodium hydride (122 mg) at 0˚C, and the mixture was stirred at room temperature for 30 min. To the reaction mixture was added 1-bromo-3-chloropropane (0.301 mL) at 0˚C, and the mixture was stirred at room temperature for 13.5 hr. The reaction mixture was diluted with water, and the mixture was extracted with ethyl acetate. The extract was washed

with water and saturated brine, and dried over anhydrous magnesium sulfate. The solvent was evaporated under reduced pressure. The residue was purified by silica gel column chromatography (ethyl acetate/hexane) to give the title compound (920 mg).
$^1$H NMR ($CDCl_3$) δ: 1.38 (9H, s), 1.58 - 1.72 (1H, m), 1.75 - 1.95 (2H, m), 2.08 - 2.27 (1H, m), 3.51 (2H, t, J = 6.4 Hz), 3.88 (1H, t, J = 7.2 Hz), 7.48 - 7.55 (2H, m), 7.64 (1H, d, J = 1.5 Hz).

C) 5-chloro-2-[4-chloro-2-(trifluoromethyl)phenyl]pentanoic acid

A mixture of tert-butyl 5-chloro-2-[4-chloro-2-(trifluoromethyl)phenyl]pentanoate (908 mg) and trifluoroacetic acid (2.3 mL) was stirred at room temperature for 4 hr. The reaction mixture was diluted with 1 M aqueous sodium hydroxide solution and ethyl acetate. The aqueous layer was acidified with 3 M hydrochloric acid, and the mixture was extracted with ethyl acetate. The extract was dried over anhydrous magnesium sulfate and the solvent was evaporated under reduced pressure. The residue was purified by silica gel column chromatography (ethyl acetate/hexane) to give the title compound (611 mg).
$^1$H NMR ($CDCl_3$) δ: 1.57 - 1.74 (1H, m), 1.77 - 2.04 (2H, m), 2.14 - 2.34 (1H, m), 3.50 (2H, t, J = 6.2 Hz), 4.02 (1H, t, J = 7.4 Hz), 7.50 - 7.55 (2H, m), 7.66 (1H, d, J = 1.5 Hz).

D) 2-{4-chloro-1-[4-chloro-2-(trifluoromethyl)phenyl]butyl}-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (1.20 g), 5-chloro-2-[4-chloro-2-(trifluoromethyl)phenyl]pentanoic acid (1.84 g) and triethylamine (2.03 mL) in DMF (24 mL) was added HATU (2.22 g) at 0˚C by small portions, and the mixture was stirred at room temperature for 13 hr. The reaction mixture was diluted with water, and extracted with ethyl acetate. The extract was washed with water and saturated brine, and dried over anhydrous magnesium sulfate. The solvent was evaporated under reduced pressure. The residue was dissolved in acetonitrile (48 mL), and carbon tetrachloride (936 mL) and triphenylphosphine (5.11 g) were added. The reaction mixture was heated under reflux for 2 hr and the solvent was evaporated under reduced pressure. The residue was diluted with saturated aqueous sodium hydrogen carbonate solution, and the mixture was extracted with ethyl acetate. The extract was washed with saturated brine, and dried over anhydrous sodium sulfate. The solvent was evaporated under reduced pressure. To the residue was added diethyl ether, the precipitated solid was removed and the solvent of the filtrate was evaporated under reduced pressure. The residue was purified by silica gel column chromatography (ethyl acetate/hexane) to give the title compound (1.59 g).
$^1$H NMR ($CDCl_3$) δ: 1.71 - 1.88 (1H, m), 1.90 - 2.04 (1H, m), 2.20 - 2.38 (1H, m), 2.46 - 2.63 (4H, m), 3.57 (2H, t, J = 6.4 Hz), 4.04 (3H, s), 4.66 (1H, t, J = 7.7 Hz), 7.48 - 7.65 (5H, m), 7.72 (1H, d, J = 2.3 Hz), 7.82 (1H, d, J = 7.9 Hz).

E) 8-[4-chloro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine

A mixture of 2-{4-chloro-1-[4-chloro-2-(trifluoromethyl)phenyl]butyl}-5-[3-methoxy-4-(2-methyl-1,3-oxazol-5-yl)phenyl]-1,3,4-oxadiazole (1.59 g) and sodium azide (393 mg) in DMSO (15 mL) was stirred at 70˚C for 13 hr. After cooling to room temperature, water was added to the reaction mixture, and the mixture was extracted with ethyl acetate. The extract was washed with water and saturated brine, and dried over anhydrous sodium sulfate. The solvent was evaporated under reduced pressure. The residue was dissolved in THF (15 mL), water (1.5 mL) and triphenylphosphine (1.58 g) were added, and the mixture was stirred at 60˚C for 1.5 hr. The solvent was evaporated under reduced pressure, to the residue was added acetic acid (10 mL) and the mixture was heated under reflux for 2 hr. The solvent was evaporated under reduced pressure, and to the residue was added saturated aqueous sodium hydrogen carbonate solution, and the mixture was extracted with ethyl acetate. The extract was washed with saturated brine, and dried over anhydrous sodium sulfate. The solvent was evaporated under reduced pressure. The residue was purified by silica gel column chromatography (methanol/ethyl acetate) to give the title compound (1.29 g).
$^1$H NMR ($CDCl_3$) δ: 1.82 - 1.98 (1H, m), 1.98 - 2.18 (1H, m), 2.18 - 2.34 (1H, m), 2.38 - 2.52 (1H, m), 2.56 (3H, s), 4.04 (3H, s), 4.10 - 4.33 (2H, m), 4.71 (1H, dd, J = 10.0, 5.9 Hz), 7.15 (1H, d, J = 8.3 Hz), 7.27 - 7.31 (1H, m), 7.42 - 7.50 (2H, m), 7.51 (1H, s), 7.71 (1H, d, J = 1.9 Hz), 7.85 (1H, d, J = 8.0 Hz). MS(ESI):489.2 $[M+H]^+$.

Example 33

(S)-(-)-8-[4-chloro-2-(trifluoromethyl)phenyl]-3-[3-methoxy-4-(2-methyl-1,3-oxazol-5-yl)phenyl]-5,6,7,8-tetrahydro [1,2,4]triazolo[4,3-a]pyridine

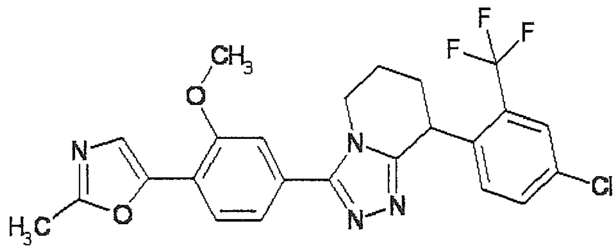

A racemate (109.5 mg) of 8-[4-chloro-2-(trifluoromethyl)phenyl]-3-[3-methoxy-4-(2-methyl-1,3-oxazol-5-yl) phenyl]-5,6,7,8-tetrahydro[1,2,4]triazolo[4,3-a]pyridine was separated by HPLC (column: CHIRALCEL AD (trade name), 50 mmIDx500 mmL, manufactured by DAICEL CHEMICAL INDUSTRIES, LTD., mobile phase: hexane/IPA = 500/500) to give the title compound with a shorter retention time (52.8 mg).
$[\alpha]_D^{25}$: +127.0°(c 0.412, methanol),
$^1$H NMR ($CDCl_3$) δ: 1.78 - 1.97 (1H, m), 1.98 - 2.16 (1H, m), 2.18 - 2.32 (1H, m), 2.39 - 2.51 (1H, m), 2.56 (3H, s), 4.04 (3H, s), 4.09 - 4.22 (1H, m), 4.22 - 4.33 (1H, m), 4.71 (1H, dd, J = 10.2, 6.1 Hz), 7.15 (1H, d, J = 8.7 Hz), 7.27 - 7.31 (1H, m), 7.44 - 7.49 (2H, m), 7.51 (1H, s), 7.71 (1H, d, J = 2.3 Hz), 7.85 (1H, d, J = 8.0 Hz). MS(ESI):489.2 $[M+H]^+$.

Example 34

(R)-(+)-8-[4-chloro-2-(trifluoromethyl)phenyl]-3-[3-methoxy-4-(2-methyl-1,3-oxazol-5-yl)phenyl]-5,6,7,8-tetrahydro [1,2,4]triazolo[4,3-a]pyridine

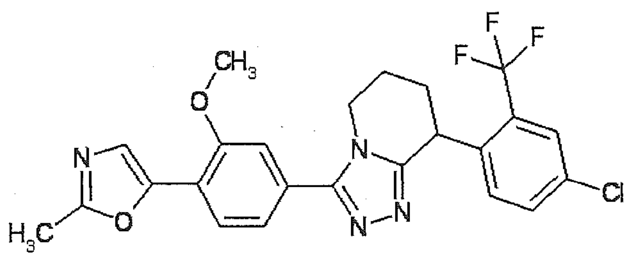

A racemate (109.5 mg) of 8-[4-chloro-2-(trifluoromethyl)phenyl]-3-[3-methoxy-4-(2-methyl-1,3-oxazol-5-yl) phenyl]-5,6,7,8-tetrahydro[1,2,4]triazolo[4,3-a]pyridine was separated by HPLC (column: CHIRALCEL AD (trade name), 50 mmID×500 mmL, manufactured by DAICEL CHEMICAL INDUSTRIES, LTD., mobile phase: hexane/IPA = 500/500) to give the title compound with a longer retention time (53.2 mg).
$[\alpha]_D^{25}$: -123.8°(c 0.473, methanol),
$^1$H NMR ($CDCl_3$) δ: 1.78 - 1.97 (1H, m), 1.98 - 2.16 (1H, m), 2.19 - 2.33 (1H, m), 2.39 - 2.53 (1H, m), 2.56 (3H, s), 4.04 (3H, s), 4.09 - 4.22 (1H, m), 4.22 - 4.33 (1H, m), 4.63 - 4.80 (1H,m); 7.15 (1H, d, J = 8.7 Hz), 7.27 - 7.31 (1H, m), 7.42 - 7.54 (3H, m), 7.71 (1H, d, J = 2.3 Hz), 7.85 (1H, d, J = 8.0 Hz). MS(ESI):489.2 $[M+H]^+$.

Example 35

3-[3-methoxy-4-(2-methyl-1,3-oxazol-5-yl)phenyl]-8-(4-morpholin-4-ylphenyl)-5,6,7,8-tetrahydro[1,2,4]triazolo[4,3-a]pyridine

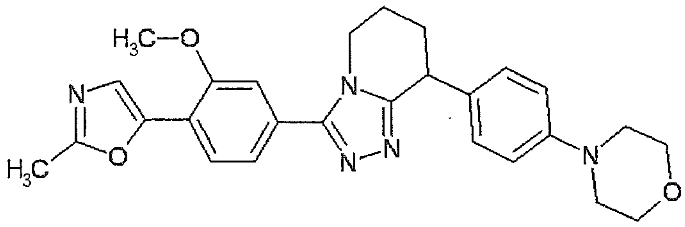

A mixture of 8-(4-bromophenyl)-3-[3-methoxy-4-(2-methyl-1,3-oxazol-5-yl)phenyl]-5,6,7,8-tetrahydro[1,2,4]triazolo[4,3-a]pyridine (Example 51; 100 mg), morpholine (20.6 μL), tris(dibenzylideneacetone)dipalladium(0) (9.8 mg), 2-dicyclohexylphosphino-2'-(dimethylamino)biphenyl (8.5 mg), and sodium tert-butoxide (41.3 mg) in toluene (2 mL) was stirred under a nitrogen atmosphere at 100°C for 2 hr. After cooling to room temperature, the reaction mixture was diluted with ethyl acetate/methanol, and filtered through celite. The solvent of the filtrate was evaporated under reduced pressure. The residue was purified by silica gel column chromatography (methanol/ethyl acetate), and recrystallized from ethyl acetate/hexane to give the title compound (19.9 mg).
$^1$H NMR ($CDCl_3$) δ: 1.94 - 2.21 (3H, m), 2.27 - 2.40 (1H, m), 2.56 (3H, s), 3.11 - 3.18 (4H, m), 3.81 - 3.91 (4H, m), 4.04 (3 H, s), 4.15 (2H, t, J = 5.8 Hz), 4.41 (1H, t, J = 6.5 Hz), 6.88 (2H, d, J = 8.5 Hz), 7.13 (2H, d, J = 8.5 Hz), 7.26 - 7.31 (1H, m), 7.48 (1H, d, J = 1.4 Hz), 7.50 (1H, s), 7.83 (1H, d, J = 8.2 Hz). MS (ESI) :472.4 $[M+H]^+$.

Example compounds shown in the following Tables 1 - 4 were produced according to the above-mentioned method or a method analogous thereto. MS in the Tables shows measured values.

Table 1

| Ex. No. | IUPAC name | structure | MS |
|---|---|---|---|
| 36 | 3-[3-methoxy-4-(2-methyl-1,3-oxazol-5-yl)phenyl]-8-(4-methoxyphenyl)-5,6,7,8-tetrahydro[1,2,4]-triazolo[4,3-a]pyridine | 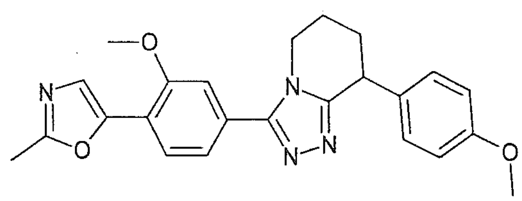 | 417.4 |
| 37 | 3-[3-methoxy-4-(2-methyl-1,3-oxazol-5-yl)phenyl]-8-[4-(methylsulfanyl)-phenyl]-5,6,7,8-tetrahydro[1,2,4]-triazolo[4,3-a] pyridine | 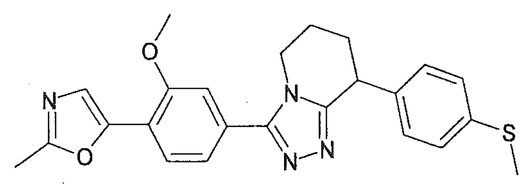 | 433.4 |
| 38 | 8-(3-chloro-4-fluorophenyl)-3-[3-methoxy-4-(2-methyl-1,3-oxazol-5-yl) phenyl]-5,6,7,8-tetrahydro[1,2,4]-triazolo [4,3-a]pyridine | 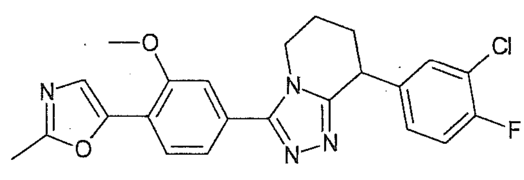 | 439.3 |

(continued)

| Ex. No. | IUPAC name | structure | MS |
|---|---|---|---|
| 39 | 8-(2-bromophenyl)-3-[3-methoxy-4-(2-methyl-1,3-oxazol-5-yl)phenyl]-5,6,7,8-tetrahydro[1,2,4]-triazolo[4,3-a]pyridine | 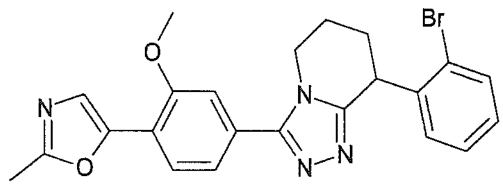 | 465.2 |
| 40 | 3-[3-methoxy-4-(2-methyl-1,3-oxazol-5-yl)phenyl]-8-[3-(trifluoromethoxy)-phenyl]-5,6,7,8-tetrahydro[1,2,4]-triazolo[4,3-a]pyridine | 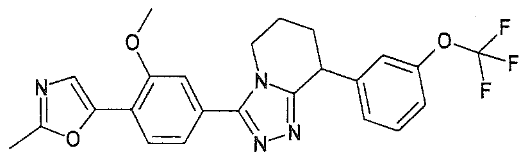 | 471.4 |

Table 2

| Ex. No. | IUPAC name | structure | MS |
|---|---|---|---|
| 41 | 8-(2,6-dichlorophenyl)-3-[3-methoxy-4-(2-methyl-1,3-oxazol-5-yl)phenyl]-5,6,7,8-tetrahydro[1,2,4]-triazolo[4,3-a]pyridine | 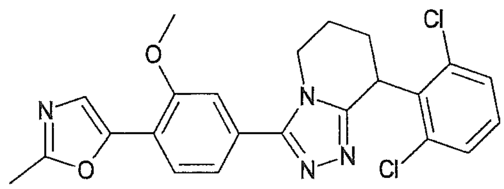 | 455.3 |
| 42 | 8-(4-fluoro-3-methoxyphenyl)-3-[3-methoxy-4-(2-methyl-1,3-oxazol-5-yl) phenyl]-5, 6, 7, 8-tetrahydro[1,2,4]-triazolo [4,3-a]pyridine | 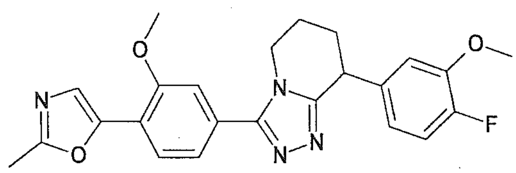 | 435.4 |
| 43 | 8-[5-fluoro-2-(trifluoromethyl)-phenyl]-3-[3-methoxy-4-(2-methyl-1,3-oxazol-5-yl)phenyl]-5,6,7,8-tetrahydro [1,2,4]-triazolo[4,3-a]pyridine | 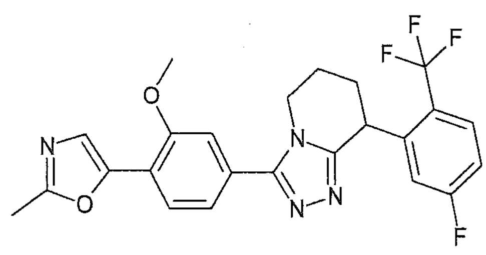 | 473.3 |

(continued)

| Ex. No. | IUPAC name | structure | MS |
|---|---|---|---|
| 44 | 3-[3-methoxy-4-(2-methyl-1,3-oxazol-5-yl)phenyl]-8-[5-(trifluoromethyl)-1H-benzimidazol-1-yl]-5,6,7,8-tetrahydro [1,2,4]-triazolo[4,3-a]pyridine | 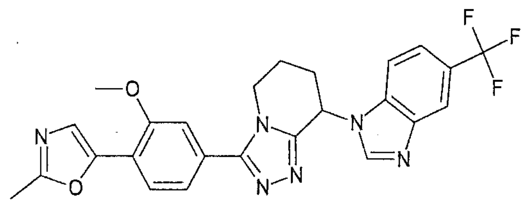 | 495.4 |
| 45 | 3-[3-methoxy-4-(2-methyl-1,3-oxazol-5-yl)phenyl]-8-{[2-(trifluoromethyl)-benzyl] oxy}-5,6,7,8-tetrahydro[1,2,4]-triazolo [4,3-a]pyridine | 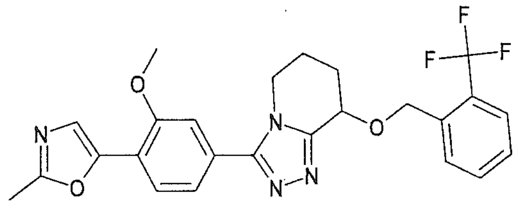 | 485.3 |

Table 3

| Ex. No. | IUPAC name | structure | MS |
|---|---|---|---|
| 46 | 3-[3-methoxy-4-(2-methyl-1,3-oxazol-5-yl)phenyl]-8-[2-(trifluoromethyl)-phenoxy]-5,6,7,8-tetrahydro[1,2,4]-triazolo[4,3-a]pyridine | 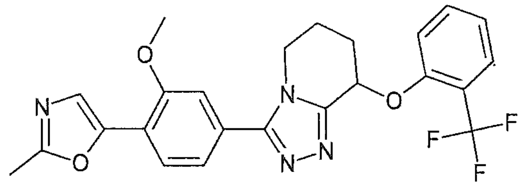 | 471.4 |
| 47 | 8-[4-chloro-2-(trifluoromethyl)-phenyl]-3-[3-methoxy-4-(4-methyl-1H-imidazol-1-yl)phenyl]-5,6,7,8-tetrahydro [1,2,4]-triazolo [4,3-a]pyridine | 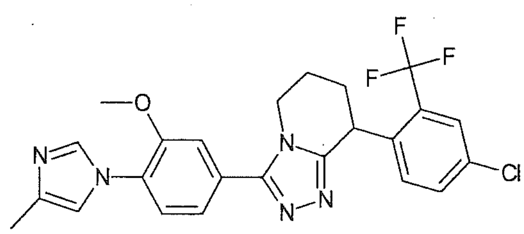 | 488.2 |
| 48 | 8-[4-chloro-2-(trifluoromethyl)-phenyl]-3-[3-fluoro-4-(2-methyl-1,3-oxazol-5-yl) phenyl]-5,6,7,8-tetrahydro[1,2,4]-triazolo [4,3-a]pyridine | 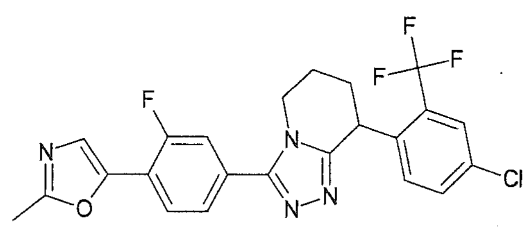 | 477.2 |

(continued)

| Ex. No. | IUPAC name | structure | MS |
|---|---|---|---|
| 49 | 8-[4-chloro-2-(trifluoromethyl)-phenyl]-3-[6-methoxy-5-(4-methyl-1H-imidazol-1-yl)pyridin-2-yl]-5,6,7,8-tetrahydro [1,2,4]-triazolo[4,3-a]pyridine | 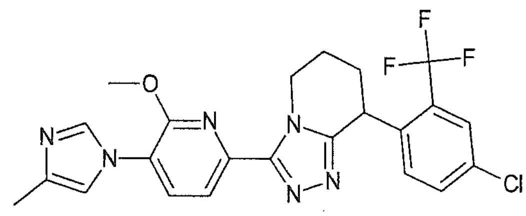 | 489.2 |

[Table 4]

| Ex. No. | IUPAC name | structure | MS |
|---|---|---|---|
| 50 | 5-{8-[4-chloro-2-(trifluoromethyl)-phenyl]-5,6,7,8-tetrahydro[1,2,4]-triazolo[4,3-a]pyridin-3-yl}-2-(2-methyl-1,3-oxazol-5-yl) benzonitrile | 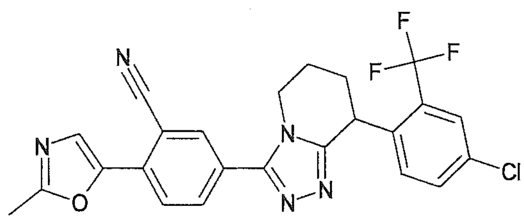 | 484.1 |
| 51 | 8-(4-bromophenyl)-3-[3-methoxy-4-(2-methyl-1,3-oxazol-5-yl)phenyl]-5, 6, 7, 8-tetrahydro[1,2,4]-triazolo[4,3-a]pyridine | 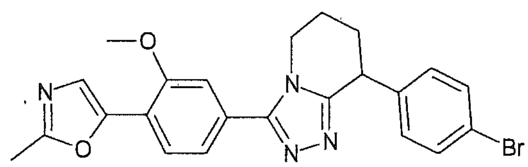 | 465.2 |

Reference Example 1

5-(4-bromo-2-fluorophenyl)-2-methyl-1,3-oxazole

1a) 4-bromo-2-fluoro-N-methoxy-N-methylbenzamide

To a solution of 4-bromo-2-fluorobenzoic acid (10 g, 46 mmol) in DMF (4.0 mL) were added dropwise N,O-dimethoxyhydroxylamine hydrochloride (5.3 g, 55 mmol), HOBt(8.0 g, 59 mmol), N-ethyldiisopropylamine (23 mL, 137 mmol) and WSC (11 g, 59 mmol), and the mixture was stirred at room temperature for 9 hr, and then at 40˚C for 38 hr. The reaction mixture was cooled to room temperature, saturated aqueous sodium hydrogen carbonate solution was added and the mixture was extracted with ethyl acetate. The obtained extract was washed with 1N aqueous sodium hydroxide solution, water and saturated brine, dried over anhydrous magnesium sulfate and the solvent was evaporated under reduced pressure to give the title compound as a pale yellow oil (13 g, quant.).
$^1H$ NMR ($CDCl_3$) 5: 3.35 (3 H, s), 3.55 (3 H, brs), 7.29 - 7.40 (3 H, m). MS(ESI):262 $[M+H]^+$.

1b) 1-(4-bromo-2-fluorophenyl)ethanone

To a solution of 4-bromo-2-fluoro-N-methoxy-N-methylbenzamide (13 g, 46 mmol) in THF (4.0 mL) was added dropwise methylmagnesium bromide (3M ethyl ether solution, 30 mL, 91 mmol) at 0˚C, and the mixture was stirred at room temperature for 3 hr. To the reaction mixture was added saturated aqueous ammonium chloride solution, and the mixture was extracted with ethyl acetate. The obtained extract was washed with water and saturated brine, and dried over anhydrous magnesium sulfate. The solvent was evaporated under reduced pressure to give the title compound as

a pale yellow oil (10 g, quant.).
$^1$H NMR ($CDCl_3$) δ: 2.63 (3 H, d, J=5.3 Hz), 7.31 - 7.44 (2 H, m), 7.77 (1 H, t, J=8.0 Hz).

1c) 5-(4-bxomo-2-fluorophenyl)-2-methyl-1,3-oxazole

To a suspension of iodobenzenediacetate (6.7 g, 21 mmol) in acetonitrile (100 mL) was added dropwise trifluoromethanesulfonic acid (3.7 mL, 42 mmol), and the mixture was stirred at room temperature for 30 min. To the reaction mixture was added 1-(4-bromo-2-fluorophenyl)ethanone (6.7 g, 21 mmol) in acetonitrile (20 mL), and the mixture was heated under reflux for 2 hr. The reaction mixture was cooled to room temperature, neutralized with saturated aqueous sodium hydrogen carbonate solution and the solvent was evaporated under reduced pressure. The residue was extracted with ethyl acetate, and the obtained extract was washed with saturated brine, and dried over anhydrous magnesium sulfate. The solvent was evaporated under reduced pressure. The obtained residue was purified by silica gel column chromatography (ethyl acetate/hexane=0/100 - 33/67) to give the title compound as a pale yellow solid (2.6 g, 75%).
$^1$H NMR ($CDCl_3$) δ: 2.54 (3 H, s), 7.31 - 7.40 (3 H, m), 7.52 - 7.65 (1 H, m). MS(ESI):256 $[M+H]^+$.

Reference Example 2

5-(4-bromo-2-methoxyphenyl)-2-methyl-1,3-oxazole

To a solution of 5-(4-bromo-2-fluorophenyl)-2-methyl-1,3-oxazole (400 mg, 1.6 mmol) in DMF (10 mL) was added dropwise sodium methoxide (28% MeOH solution, 900 μL, 4.7 mmol), and the mixture was stirred at 80˚C for 2 hr. The reaction mixture was cooled to room temperature, diluted with water, and the mixture was extracted with ethyl acetate. The obtained extract was washed with water and saturated brine, and dried over anhydrous magnesium sulfate. The solvent was evaporated under reduced pressure. The obtained residue was purified by silica gel column chromatography (ethyl acetate/hexane=0/100 - 40/60) to give the title compound as a colorless solid (370 mg, 88%). $^1$H NMR ($CDCl_3$) δ: 2.52 (3 H, s), 3.95 (3 H, s), 7.10 (1 H, d, J=1.9 Hz), 7.17 (1 H, dd, J=8.3, 1.9 Hz), 7.39 (1 H, s), 7.59 (1 H, d, J=8.3 Hz). MS (ESI) :268 $[M+H]^+$.

Reference Example 3

3-methoxy-4-(2-methyl-1,3-oxazol-5-yl)benzoic acid

3a) methyl 3-methoxy-4-(2-methyl-1,3-oxazol-5-yl)benzoate

A mixture of 5-(4-bromo-2-methoxyphenyl)-2-methyl-1,3-oxazole (10.0 g, 37.3 mmol), palladium acetate (837 mg, 3.73 mmol), 1,3-bis(diphenylphosphino)propane (1.54 g, 3.73 mmol) and triethylamine (15.6 mL, 113 mmol) in methanol (380 mL) was stirred under a carbon monoxide atmosphere at 60˚C for 2 days. The reaction mixture was filtered through celite, and the filtrate was concentrated. The residue was diluted with 2 M hydrochloric acid, and extracted twice with dichloromethane. The obtained organic layer was mixed, washed with saturated brine, and dried over anhydrous sodium sulfate. The solvent was evaporated under reduced pressure. The residue was purified by silica gel column chromatography (ethyl acetate/hexane=33/67) to give the title compound as a white solid (6.10 g, 66%)
$^1$H NMR ($CDCl_3$) δ: 2.55 (3 H, s), 3.94 (3 H, s), 4.02 (3 H, s), 7.53 (1 H, s), 7.62 (1 H, d, J=1.2 Hz), 7.70 (1 H, dd, J=8.0, 1.2Hz,), 7.78 (1 H, d, J=8.0 Hz).

3b) 3-methoxy-4-(2-methyl-1,3-oxazol-5-yl)benzoic acid

To a mixture of methyl 3-methoxy-4-(2-methyl-1,3-oxazol-5-yl)benzoate (6.10 g, 24.7 mmol) in methanol (250 mL) was added 6 M aqueous sodium hydroxide solution (41.1 mL, 247 mmol), and the mixture was stirred at room temperature for 1 hr. 2 M Hydrochloric acid (20 mL) was added, and the obtained solid was collected by filtration to give the title compound as a white solid (4.80 g, 83%).
$^1$H NMR ($CDCl_3$) δ: 2.05 (3 H, s), 4.00 (3 H, s), 7.55 (1 H, s), 7.61 (1 H, d, J=1.2 Hz), 7.65 (1 H, dd, J=8.0, 1.6Hz), 7.76 (1 H, d, J=8.0 Hz), hidden (1H). MS(ESI):234 $[M+H]^+$.

Reference Example 4

3-methoxy-4-(2-methyl-1,3-oxazol-5-yl)benzohydrazide hydrochloride

4a) tert-butyl 2-{[3-methoxy-4-(2-methyl-1,3-oxazol-5-yl)phenyl]carbonyl}hydrazinecarboxylate

To a mixture of 3-methoxy-4-(2-methyl-1,3-oxazol-5-yl)benzoic acid (608 mg, 2.61 mmol) and tert-butyl carbazate (414 mg, 3.13 mmol) in DMF (13 mL) was added diethyl cyanophosphate (505 μL, 3.39 mmol), and the mixture was stirred at room temperature for 30 min. Triethylamine (1.09 mL, 7.82 mmol) was added, and the mixture was stirred at room temperature for 18 hr. The reaction solution was diluted with water, and the mixture was extracted with ethyl acetate. The extract was washed with saturated brine, dried over anhydrous magnesium sulfate, and the solvent was evaporated under reduced pressure. The residue was purified by silica gel column chromatography (ethyl acetate/ hexane=50/50 - 100/0) to give the title compound as a white solid (663 mg, 73%).
$^1$H NMR ($CDCl_3$) δ: 1.48 (9 H, d, J=2.7 Hz), 2.54 (3 H, s), 3.98 (3 H, s), 6.69 (1 H, brs), 7.38 (1 H, d, J=8.0 Hz), 7.45 (1 H, s), 7.50 (1 H, s), 7.73 (1 H, d, J=8.3 Hz), 8.20 (1 H, brs). MS(ESI):348 $[M+H]^+$.

4b) 3-methoxy-4-(2-methyl-1,3-oxazol-5-yl)benzohydrazide hydrochloride

To a mixture of tert-butyl 2-{[3-methoxy-4-(2-methyl-1,3-oxazol-5-yl)phenyl]carbonyl}hydrazinecarboxylate (656 mg, 1.89 mmol) in ethyl acetate (10 mL) was added hydrogen chloride (4M ethyl acetate solution, 10 mL), and the mixture was stirred at room temperature for 2 hr. The reaction solution was concentrated, and the obtained solid was washed with ethyl acetate to give the title compound as a white solid (518 mg, 96%).
$^1$H NMR (DMSO-$d_6$) δ: 2.50 (3 H, s), 3.82 (3 H, brs), 4.04 (3 H, s), 7.57 (1 H, s), 7.60 - 7.72 (2 H, m), 7.81 (1 H, d, J=8.3 Hz), 11.80 (1 H, s).

Reference Example 5

1-(4-bromo-2-methoxyphenyl)-3-methyl-1H-1,2,4-triazole

5a) (4-bromo-2-methoxyphenyl)hydrazine

To a solution of 4-bromo-2-methoxyaniline (4.0 g, 20 mmol) in concentrated hydrochloric acid (40 mL) was added dropwise sodium nitrite (1.4 g, 21 mmol) and water (2 mL) solution while maintaining at -20˚C, and the mixture was stirred at the same temperature for 15 min. The mixture was warmed to 0˚C, and the mixture was stirred at the same temperature for 20 min, and further, a solution of tin chloride·2 hydrate (17 g, 74 mmol) in concentrated hydrochloric acid (140 mL) was added dropwise to the reaction mixture while maintaining at -20˚C. After stirring at the same temperature for 10 min, and the mixture was stirred at room temperature for 40 min. The precipitate was collected by filtration, and washed with ice water and diethyl ether. To the filtered material was added ethyl acetate and 10% aqueous potassium carbonate, and the insoluble material was filtered off. The aqueous layer of the filtrate was extracted with ethyl acetate, the organic layer was combined, and the mixture was washed with saturated brine. The organic layer was dried over anhydrous magnesium sulfate and the solvent was evaporated under reduced pressure to give the title compound as a pale yellow solid (3.4 g, 78%).
$^1$H NMR ($CDCl_3$) δ: 3.49 (2 H, br), 3. 84 (3 H, s), 5.61 (1 H, br), 6.85 - 6.88(2 H, m), 7.06 (1 H, dd, J=2.1, 8.7 Hz).

5b) 1-(4-bromo-2-methoxyphenyl)-3-methyl-1H-1,2,4-triazole

To a solution of (4-bromo-2-methoxyphenyl)hydrazine(2.8 g, 13 mmol) in MeOH (25 mL) was added methyl ethaneimidethioate hydroiodide (2.8 g, 13 mmol), and the mixture was stirred at room temperature for 30 min. The reaction mixture was concentrated under reduced pressure, to the residue were added trimethyl orthoformate (13 mL), toluene (25 mL), further, pyridine (25 mL), and the mixture was stirred for 100˚C for 16 hr. The reaction mixture was cooled to room temperature, and concentrated under reduced pressure. To the residue was added ethyl acetate-saturated aqueous sodium hydrogen carbonate-water, and the aqueous layer was extracted with ethyl acetate. The organic layer was dried over anhydrous magnesium sulfate, and the solvent was evaporated under reduced pressure. The obtained residue was purified by silica gel column chromatography (hexane/ethyl acetate=8/2 - 6/4) to give the title compound as a pale yellow solid (2.6 g, 74%).
$^1$H NMR ($CDCl_3$) δ 2.47 (3 H, s), 3.93 (3 H, s), 7.19 - 7.22 (2 H, m), 7.64 (1 H, d, J=8.1 Hz), 8.59 (1 H, br). MS(ESI):268 $[M+H]^+$.

Reference Example 6

3-methoxy-4-(3-methyl-1H-1,2,4-triazol-1-yl)benzoic acid

6a) 3-methoxy-4-(3-methyl-1H-1,2,4-triazol-1-yl)benzonitrile

A mixture of 1-(4-bromo-2-methoxyphenyl)-3-methyl-1H-1,2,4-triazole (1.00 g, 3.73 mmol), zinc cyanide (2.19 g, 18.7 mmol), $Pd_2(dba)_3$ (174 mg, 0.19 mmol) and 2-(di-tert-butylphosphino)biphenyl (110 mg, 0.37 mmol) in DMF (10 mL) was heated under reflux for 24 hr. The reaction solution was diluted with water, extracted with ethyl acetate, and washed with water and saturated brine. This was dried over anhydrous magnesium sulfate, and the solvent was evaporated. The residue was purified by silica gel column chromatography (ethyl acetate/hexane=50/50 - 80/20) to give the title compound as a white solid (320 mg, 40%).
$^1H$ NMR ($CDCl_3$) δ: 2.49 (3 H, s), 4.02 (3 H, s), 7.31 (1 H, s), 7.40 (1 H, dd, J=8.4, 1.5 Hz), 8.00 (1 H, d, J=8.2 Hz), 8.80 (1 H, s). MS(ESI):215 $[M+H]^+$.

6b) 3-methoxy-4-(3-methyl-1H-1,2,4-triazol-1-yl)benzoic acid

A mixture of 1-(4-bromo-2-methoxyphenyl)-3-methyl-1H-1,2,4-triazole (536 mg, 2.00 mmol), zinc cyanide (1.17 g, 10.0 mmol), $Pd_2(dba)_3$ (183 mg, 0.200 mmol) and 2-(di-tert-butylphosphino)biphenyl (119 mg, 0.400 mmol) in DMF (4.0 mL) was stirred at 120˚C for 4 hr. The reaction solution was diluted with ethyl acetate, and filtered. The filtrate was washed with water and saturated brine, dried over anhydrous sodium sulfate, and the solvent was evaporated. To the residue was added 4 M aqueous sodium hydroxide solution (3 mL) and 2-methoxyethanol(3 mL), and the mixture was heated under reflux for 2 hr. The reaction solution was cooled to room temperature, diluted with ethyl acetate, and extracted with water. The aqueous layer was neutralized with 6 M hydrochloric acid, extracted with ethyl acetate, and the solvent was evaporated to give the title compound as a white solid (122 mg, 26%). MS (ESI) :234 $[M+H]^+$.

Reference Example 7

1-(4-iodo-2-methoxyphenyl)-4-methyl-1H-imidazole

7a) N-(4-iodo-2-methoxyphenyl)formamide

A mixture of 4-iodo-2-methoxyaniline (31.0 g, 124 mmol) and zinc oxide (10.1 g, 124 mmol) in formic acid (41 mL) was stirred at 70˚C for 45 min, cooled to room temperature, and 2 M hydrochloric acid (200 mL) was added. The reaction solution was further stirred for 20 min and collected by filtration. The obtained solid was washed with a mixed solution of hexane and dichloromethane to give the title compound as a black solid (30.8 g, 66%).
$^1H$ NMR (DMSO-$d_6$) δ: 3.86 (3 H, s), 7.27 (1 H, dd, J=8.0, 1.5 Hz), 7.34 (1 H, d, J=1.5 Hz), 7.97 (1 H, d, J=8.5 Hz), 8.30 (1 H, d, J=1.5 Hz), 9.71 (1 H, s).

7b) N-(4-iodo-2-methoxyphenyl)-N-(2-oxopropyl)formamide

To a mixture of N-(4-iodo-2-methoxyphenyl)formamide (30.5 g, 110 mmol), cesium carbonate (108 g, 330 mmol) and potassium iodide (7.30 g, 44.0 mmol) in DMF (130 mL) was added 1-chloropropan-2-one (18.6 g, 198 mmol) at 0˚C. The reaction mixture was stirred at room temperature overnight, filtered, and washed with water. This was dried over anhydrous sodium sulfate, and the solvent was evaporated. The residue was purified by silica gel column chromatography (aqueous ammonia/methanol/dichloromethane=0/0/1000 - 3/50/947) to give the title compound as a brown solid (28.0 g, 76%).
$^1H$ NMR (DMSO-$d_6$) δ: 2. 08 (3 H, s), 3. 82 (3 H, s), 4. 41 (2 H, s), 7.02 (1 H, d, J=8.0 Hz), 7.36 (1 H, dd, J=8.5, 2.0 Hz), 7.44 (1 H, d, J=1.5 Hz), 8.19 (1 H, s). MS(ESI):315 $[M+H]^+$.

7c) 1-(4-iodo-2-methoxyphenyl)-4-methyl-1H-imidazole

A mixture of N-(4-iodo-2-methoxyphenyl)-N-(2-oxopropyl)formamide (28.0 g, 84.1 mmol) and ammonium acetate (32.4 g, 420 mmol) in acetic acid (100 mL) was stirred at 140˚C for 90 min. The reaction solution was diluted with water, neutralized with sodium hydrogen carbonate, and the mixture was extracted with ethyl acetate. This was dried over anhydrous sodium sulfate, and the solvent was evaporated. The residue was purified by silica gel column chromatography (ethyl acetate/dichloromethane=0/100 - 50/50) to give the title compound (18.9 g. 72%).
$^1H$ NMR (DMSO-$d_6$) δ: 2.14 (3 H, s), 3.83 (3 H, s), 7.10 (1 H, t, J=1.0 Hz), 7.14 (1 H, d, J=8.0 Hz), 7.41 (1 H, dd, J=8.0,

1.5 Hz), 7.53 (1 H, d, J=2.0 Hz), 7.74 (1 H, d, J=1.0 Hz). MS (ESI) :314 $[M+H]^+$.

Reference Example 8

t-butyl 2-{[3-methoxy-4-(4-methyl-1H-imidazol-1-yl)phenyl]carbonyl}hydrazinecarboxylate

A mixture of 1-(4-iodo-2-methoxyphenyl)-4-methyl-1H-imidazole (314 mg, 1.00 mmol), palladium acetate (11.2 mg, 0.05 mmol), sodium carbonate (106 mg, 1.00 mmol) and isopropanol (0.05 mL) in DMF (1.0 mL) was heated to 140˚C, potassium hexacyanoferrate (II) trihydrate (169 mg, 0.400 mmol) was added. After 30 hr, the reaction solution was diluted with water, extracted with a mixed solution of ethyl acetate and THF, and the solvent was evaporated. The residue was diluted with ethanol (1.8 mL), 8 M aqueous sodium hydroxide solution (0.2 mL) was added, and the mixture was heated under reflux for 3 days. The solvent was evaporated, diluted with DMF (5.0 mL), tert-butyl carbazate (264 mg, 2.00 mmol) and HATU (760 mg, 2.00 mmol) were added, and the mixture was stirred at room temperature for 1 hr. The reaction solution was diluted with ethyl acetate, and washed with saturated aqueous sodium hydrogen carbonate solution and saturated brine. This was dried over anhydrous sodium sulfate and the solvent was evaporated under reduced pressure. The residue was purified by silica gel column chromatography (methanol/ethyl acetate=0/100 - 5/95) to give the title compound as a colorless solid (92.5 mg, 27%).
$^1H$ NMR ($CDCl_3$) δ: 1.52 (9 H, s), 2.30 (3 H, s), 3.90 (3 H, s), 6.70 (1 H, br), 6.92 - 6.96 (1 H, m), 7.28 (1 H, d, J=8.2 Hz), 7.40 (1 H, dd, J=8.3, 1.6 Hz), 7.55 (1 H, d, J=1.6 Hz), 7.76 (1 H, d, J=1.4 Hz), 8.21 (1 H, br). MS(ESI):347 $[M+H]^+$.

Reference Example 9

2-[4-chloro-1-(3,4-dichlorophenyl)butyl]-5-[3-methoxy-4-(2-methyl-1,3-oxazol-5-yl)phenyl]-1,3,4-oxadiazole

9a) 5-chloro-2-(3,4-dichlorophenyl)pentanoic acid

To a solution of (3,4-dichlorophenyl)acetic acid (2.00 g, 9.75 mmol) in THF (30 mL) was added dropwise n-butyllithium 1.6 M hexane solution (12.2 mL, 19.5 mmol) at -78˚C and, 1 hr later, and the mixture was stirred for 1 hr under ice-cooling. 1-Bromo-3-chloropropane (1.06 mL, 10.7 mmol) was added, and the mixture was stirred at room temperature for 18 hr. The reaction mixture was diluted with 1 M hydrochloric acid, and extracted with ethyl acetate. The extract was washed with water and saturated brine, dried over anhydrous sodium sulfate, and the solvent was evaporated. The obtained residue was purified by silica gel column chromatography (hexane/ethyl acetate=0/100 - 50/50) to give the title compound as a white solid (740 mg, 27%).
$^1H$ NMR ($CDCl_3$) δ: 1.63 - 1.87 (2 H, m), 1.87 - 2.02 (1 H, m), 2.12 - 2.31 (1 H, m), 3.48 - 3.60 (3 H, m), 7.16 (1 H, dd, J=8.2, 2.2 Hz), 7.38 - 7.45 (2 H, m).

9b) 2-[4-chloro-1-(3,4-dichlorophenyl)butyl]-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (199 mg, 0.700 mmol), 5-chloro-2-(3,4-dichlorophenyl)pentanoic acid (237 mg, 0.84 mmol) and triethylamine (293 μL, 2.10 mmol) in DMF (3.5 mL) was added HATU (319 mg, 0.840 mol); and the mixture was stirred at room temperature for 1 hr. The reaction solution was diluted with ethyl acetate, and washed with water and saturated brine. This was dried over anhydrous sodium sulfate and the solvent was evaporated under reduced pressure. The residue was diluted with acetonitrile (7.0 mL), and carbon tetrachloride (0.135 mL, 1.40 mmol) and triphenylphosphine (734 mg, 2.80 mmol) were added. The mixture was heated under reflux for 2 hr, and the solvent was evaporated under reduced pressure. The residue was diluted with ethyl acetate, washed with saturated aqueous sodium hydrogen carbonate solution and saturated brine, dried over anhydrous sodium sulfate, and the solvent was evaporated. The obtained residue was purified by silica gel column chromatography (hexane/ethyl acetate=0/100 - 40/60) to give the title compound as a colorless oil (201 mg, 58%).
$^1H$ NMR ($CDCl_3$) δ: 1.75 - 1.96 (2 H, m), 2.20 - 2.35 (1 H, m), 2.41 - 2.54 (1 H, m), 2.55 (3 H, s), 3.59 (2 H, t, J=6.3 Hz), 4.05 (3 H, s), 4.25 (1 H, t, J=7.8 Hz), 7.22 (1 H, dd, J=8.2, 2.2 Hz), 7.41 - 7.54 (3 H, m), 7.57 - 7.65 (2 H, m), 7.82 (1 H, d, J=8.0 Hz). MS (ESI) :492 $[M+H]^+$.

Reference Example 10

2-[4-chloro-1-(3,4-dichlorophenyl)butyl]-5-[3-methoxy-4-(3-methyl-1H-1,2,4-triazol-1-yl)phenyl]-1,3,4 oxadiazole

10a) N'-[5-chloro-2-(3,4-dichlorophenyl)pentanoyl]-3-methoxy-4-(3-methyl-1H-1,2,4-triazol-1-yl)benzohydrazide

To a mixture of 3-methoxy-4-(3-methyl-1H-1,2,4-triazol-1-yl)benzoic acid (122 mg, 0.523 mmol) and tert-butyl carbazate (69.1 mg, 0.523 mmol) in DMF (2.5 mL) was added diethyl cyanophosphate (81.8 μL, 0.549 mmol), and the mixture was stirred at room temperature for 30 min. Triethylamine (146 μL, 1.05 mmol) was added, and the mixture was stirred at room temperature for 3 hr. The reaction solution was diluted with ethyl acetate, washed with water and saturated brine, and dried over anhydrous sodium sulfate. The solvent was evaporated under reduced pressure. The obtained residue was diluted with methanol (0.5 mL), hydrogen chloride (4M ethyl acetate solution, 2.5 mL) was added, and the mixture was stirred at room temperature for 24 hr. The reaction solution was concentrated, filtered, and the obtained solid was suspended in DMF (2.0 mL). 5-Chloro-2-(3,4-dichlorophenyl)pentanoic acid (124 mg, 0.442 mmol), WSC (84.7 mg, 0.442 mmol), HOBt (59.7 mg, 0.442 mmol) and N-ethyldiisopropylamine (233 μL, 1.36 mmol) were added. The mixture was stirred at room temperature for 2 hr, diluted with ethyl acetate, and washed with water and saturated brine. This was dried over anhydrous sodium sulfate and the solvent was evaporated under reduced pressure. The residue was purified by silica gel column chromatography (ethyl acetate/hexane=50/50 - 100/0) to give the title compound as a colorless oil (98.1 mg, 37%).
$^1H$ NMR ($CDCl_3$) δ: 1.60 - 1.77 (2 H, m), 1.86 - 2.02 (1 H, m), 2.12 - 2.31 (1 H, m), 2.47 (3 H, s), 3.44 - 3.64 (3 H, m), 3.89 (3 H, s), 7.17 (1 H, dd, J=8.2, 1.9 Hz), 7.35 (1 H, d, J=8.2 Hz), 7.41 - 7.56 (3 H, m), 7.86 (1 H, d, J=8.2 Hz), 8.78 (1 H, s), 9.49 (1 H, br), 9.76 (1 H, br). MS(ESI):510 $[M+H]^+$.

10b) 2-[4-chloro-1-(3,4-dichlorophenyl)butyl]-5-[3-methoxy-4-(3-methyl-1H-1,2,4-triazol-1-yl)phenyl]-1,3,4-oxadiazole

To N'-[5-chloro-2-(3,4-dichlorophenyl)pentanoyl]-3-methoxy-4-(3-methyl-1H-1,2,4-triazol-1-yl)benzohydrazide (98.1 mg, 0.192 mmol) was added phosphorus oxychloride (1.0 mL), and the mixture was heated under reflux for 5 hr. The solvent was evaporated, and the residue was diluted with ethyl acetate, and washed with saturated aqueous sodium hydrogen carbonate solution and saturated brine. This was dried over anhydrous sodium sulfate and the solvent was evaporated under reduced pressure. The residue was purified by silica gel column chromatography (ethyl acetate/hexane=50/50 - 100/0) to give the title compound as a colorless oil (40.1 mg, 42%).
$^1H$ NMR ($CDCl_3$) δ: 1.76 - 1.94 (2 H, m), 2.20 - 2.36 (1 H, m), 2.42 - 2.53 (1 H, m), 2.50 (3 H, s), 3.59 (2 H, t, J=6.3 Hz), 4.05 (3 H, s), 4.25 (1 H, t, J=7.7 Hz), 7.22 (1 H, dd, J=8.2, 2.2 Hz), 7.41 - 7.51 (2 H, m), 7.65 (1 H, dd, J=8.4, 1.6 Hz), 7.75 (1 H, d, J=1.6 Hz), 7.98 (1 H, d, J=8.4 Hz), 8.78 (1 H, s). MS(ESI):492 $[M+H]^+$.

Reference Example 11

2-[4-chloro-1-(3,4,5-trifluorophenyl)butyl]-5-[3-methoxy-4-(2-methyl-1,3-oxazol-5-yl)phenyl]-1,3,4-oxadiazole

11a) 5-chloro-2-(3,4,5-trifluorophenyl)pentanoic acid

To a solution of (3,4,5-trifluorophenyl)acetic acid (561 mg, 2.95 mmol) in THF (12 mL) was added dropwise n-butyllithium 1.6 M hexane solution (3.69 mL, 5.90 mmol) at - 78°C, and the mixture was stirred under ice-cooling for 1 hr. 1-Bromo-3-chloropropane (0.305 mL, 3.10 mmol) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (ethyl acetate/hexane=20/80 - 50/50) to give the title compound as a colorless oil (0.535 g, 68%).
$^1H$ NMR ($CDCl_3$) δ: 1.60 - 2.01 (3 H, m), 2.11 - 2.27 (1 H, m), 3.41 - 3.62 (3 H, m), 6.86 - 7.06 (2 H, m).

11b) 2-[4-chloro-1-(3,4,5-trifluorophenyl)butyl]-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (150 mg, 0.635 mmol), 5-chloro-2-(3,4,5-trifluorophenyl)pentanoic acid (169 mg, 0.634 mmol) and triethylamine(0.074 mL, 0.53 mmol) in DMF (2.5 mL) was added diethyl cyanophosphate (0.102 mL, 0.687 mmol), and the mixture was stirred at room temperature for 30 min. Triethylamine (0.222 mL, 1.59 mmol) was added, and the mixture was stirred at room temperature for 14 hr. Water was added to the reaction mixture, and the mixture was extracted with ethyl acetate. The obtained extract was

washed with water and saturated brine, and dried over anhydrous magnesium sulfate. The solvent was evaporated under reduced pressure, and the residue was diluted with acetonitrile (5.0 mL), and carbon tetrachloride (0.102 mL, 1.06 mmol) and triphenylphosphine (556 mg, 2.12 mmol) were added. The mixture was heated under reflux for 3 hr, and the solvent was evaporated under reduced pressure. The residue was diluted with ethyl acetate, washed with saturated aqueous sodium hydrogen carbonate solution and saturated brine, dried over anhydrous magnesium sulfate and the solvent was evaporated. The obtained residue was purified by silica gel column chromatography (ethyl acetate/hexane=30/70 - 80/20) to give the title compound as a colorless oil (169 mg, 67%).
$^1$H NMR ($CDCl_3$) δ: 1.73 - 1.99 (2 H, m), 2.16 - 2.35 (1 H, m), 2.36 - 2.52 (1 H, m), 2.55 (3 H, s), 3.46 - 3.69 (2 H, m), 4.08 (3 H, s), 4.23 (1 H, t, J=7.8 Hz), 6.90 - 7.16 (2 H, m), 7.42 - 7.71 (3 H, m), 7.82 (1 H, d, J=8.0 Hz). MS(ESI):478 $[M+H]^+$.

Reference Example 12

2-{4-chloro-1-[2-(trifluoromethyl)phenyl]butyl}-5-[3-methoxy-4-(2-methyl-1,3-oxazol-5-yl)phenyl]-1,3,4-oxadiazole

12a) 5-chloro-2-[2-(trifluoromethyl)phenyl]pentanoic acid

A solution of [2-(trifluoromethyl)phenyl]acetic acid (3.06 g, 15.0 mmol) in toluene (30 mL) was heated to 80˚C, 1,1-di-tert-butoxy-N,N-dimethylmethaneamine (14.4 g, 60.0 mmol) was added and the mixture was stirred at 80˚C for 2 hr. After cooling to room temperature, the solvent was evaporated under reduced pressure, and the residue was extracted with ethyl acetate. The obtained extract was washed with water, and dried over anhydrous magnesium sulfate. The solvent was evaporated under reduced pressure, and the residue was purified by silica gel column chromatography (ethyl acetate/hexane=0/100 - 30/70) to give tert-butyl [2-(trifluoromethyl)phenyl]acetate (3.16 g, 81%). To a solution of tert-butyl [2-(trifluoromethyl)phenyl]acetate (3.10 g, 11.9 mmol) in DMF (36 mL) was added sodium hydride (60% oil, 524 mg, 13.1 mmol), and the mixture was stirred at room temperature for 30 min. 1-Chloro-3-iodopropane (1.34 mL, 12.5 mmol) was added, and the mixture was stirred at room temperature for 15 hr. Water was added to the reaction mixture, and the mixture was extracted with ethyl acetate. The organic layer was washed with water and saturated brine, dried over anhydrous magnesium sulfate, and the solvent was evaporated under reduced pressure. The obtained residue was purified by silica gel column chromatography (ethyl acetate/hexane=0/100 - 30/70) to give tert-butyl 5-chloro-2-[2-(trifluoromethyl)phenyl]pentanoate (3.63 g, 91%). A solution of tert-butyl 5-chloro-2-[2-(trifluoromethyl)phenyl]pentanoate (3.56 g, 9.83 mmol) in TFA (10 mL) was stirred at room temperature for 18 hr. The solvent was evaporated under reduced pressure, ethyl acetate was added, and the mixture was extracted with 1 M aqueous sodium hydroxide solution. The extract was acidified with 6 M hydrochloric acid, extracted with ethyl acetate, dried over anhydrous magnesium sulfate, and the solvent was evaporated under reduced pressure to give the title compound as a colorless oil (2.74 g, 99%).
$^1$H NMR ($CDCl_3$) δ: 1.55 - 1.75 (1 H, m), 1.76 - 2.04 (2 H, m), 2.16 - 2.34 (1 H, m), 3.50 (2 H, t, J=6.3 Hz), 4.06 (1 H, t, J=7.3 Hz), 7.31 - 7.45 (1 H, m), 7.46 - 7.62 (2 H, m), 7.67 (1 H, d, J=7.7 Hz).

12b) 2-{4-chloro-1-[2-(trifluoromethyl)phenyl]butyl}-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (150 mg, 0.635 mmol), 5-chloro-2-(3,4,5-trifluorophenyl)pentanoic acid (178 mg, 0.635 mmol) and triethylamine(0.074 mL, 0.53 mmol) in DMF (2.5 mL) was added diethyl cyanophosphate (0.102 mL, 0.687 mmol), and the mixture was stirred at room temperature for 30 min. Triethylamine (0.222 mL, 1.59 mmol) was added, and the mixture was stirred at room temperature for 14 hr. Water was added to the reaction mixture, and the mixture was extracted with ethyl acetate. The obtained extract was washed with water and saturated brine, and dried over anhydrous magnesium sulfate. The solvent was evaporated under reduced pressure, and the residue was diluted with acetonitrile (5.0 mL), carbon tetrachloride (0.102 mL, 1.06 mmol) and triphenylphosphine (556 mg, 2.12 mmol) was added. The mixture was heated under reflux for 3 hr, and the solvent was evaporated under reduced pressure. The residue was diluted with ethyl acetate, washed with saturated aqueous sodium hydrogen carbonate solution and saturated brine, dried over anhydrous magnesium sulfate and the solvent was evaporated. The obtained residue was purified by silica gel column chromatography (ethyl acetate/hexane=30/70 - 80/20) to give the title compound as a colorless oil (158 mg, 61%).
$^1$H NMR ($CDCl_3$) δ: 1.55 - 1.88 (2 H, m), 1.89 - 2.10 (1 H, m), 2.18 - 2.43 (1 H, m), 2.54 (3 H, s), 3.57 (2 H, t, J=6.4 Hz), 4.02 (3 H, s), 4.70 (1 H, t, J=7.5 Hz), 7.32 - 7.75 (7 H, m), 7.79 (1 H, d, J=8.0 Hz). MS(ESI):492 $[M+H]^+$.

Reference Example 13

2-[4-chloro-1-(4-fluorophenyl)butyl]-5-[3-methoxy-4-(2-methyl-1,3-oxazol-5-yl)phenyl]-1,3,4-oxadiazole

13a) 5-chloro-2-(4-fluorophenyl)pentanoic acid

To a solution of (4-fluorophenyl)acetic acid (3.85 g, 25.0 mmol) in THF (100 mL) was added dropwise n-butyllithium 1.6 M hexane solution (31.3 mL, 50.0 mmol) at -78˚C, and the mixture was stirred under ice-cooling for 2 hr, 1-bromo-3-chloropropane (2.58 mL, 26.3 mmol) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (ethyl acetate/hexane=20/80 - 50/50) to give the title compound as a pale yellow oil (3.50 g, 61%).
$^1$H NMR ($CDC1_3$) δ: 1.58 - 1.86 (2 H, m), 1. 87 - 2.01 (1 H, m), 2.12 - 2.27 (1 H, m), 3.45 - 3.65 (3 H, m), 6.97 - 7.08 (2 H, m), 7.23 - 7.33 (2 H, m).

13b) N'-[5-chloro-2-(4-fluorophenyl)pentanoyl]-3-methoxy-4-(2-methyl-1,3-oxazol-5-yl)benzohydrazide

A mixture of 3-methoxy-4-(2-methyl-1,3-oxazol-5-yl)benzohydrazide hydrochloride (148 mg, 0.522 mmol), 5-chloro-2-(4-fluorophenyl)pentanoic acid (120 mg, 0.522 mmol), WSC (110 mg, 0.574 mmol), HOBt (8.8 mg, 0.0574 mmol), triethylamine (0.0218 mL, 1.57 mmol) in acetonitrile (5.2 mL) was stirred at room temperature for 16 hr. Water was added to the reaction mixture, and the mixture was extracted with ethyl acetate. The obtained extract was washed with water and saturated brine, and dried over anhydrous magnesium sulfate. The solvent was evaporated under reduced pressure to give the title compound as white crystals (177 mg, 74%).
$^1$H NMR ($CDCl_3$) δ: 1. 66 - 1.88 (2 H, m), 1.92 - 2.08 (1 H, m), 2.19 - 2.37 (1 H, m), 2.53 (3 H, s), 3.46 - 3.60 (3 H, m), 3.93 (3 H, s), 7.03 (2 H, t, J=8.7 Hz), 7.30 - 7.41 (4 H, m), 7.50 (1 H, s), 7.72 (1 H, d, J=7.9 Hz), 8.81 (1 H, brs), 9.25 (1 H, brs).

13c) 2-[4-chloro-1-(4-fluorophenyl)butyl]-5-[3-methoxy-4-(2-methyl-1,3-oxazol-5-yl)phenyl]-1,3,4-oxadiazole

A mixture of N'-[5-chloro-2-(4-fluorophenyl)pentanoyl]-3-methoxy-4-(2-methyl-1,3-oxazol-5-yl)benzohydrazide (177 mg, 0.385 mmol), carbon tetrachloride (0.0740 mL, 0.770 mmol) and triphenylphosphine (404 mg, 1.54 mmol) in acetonitrile (3.8 mL) was stirred at 80˚C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The residue was purified by silica gel column chromatography (ethyl acetate/hexane=10/90 - 50/50) to give the title compound as a pale yellow oil (138 mg, 81%).
$^1$H NMR ($CDCl_3$) δ: 1.73 - 1.96 (2 H, m), 2.21 - 2.35 (1 H, m), 2.40 - 2.59 (4 H, m), 3.58 (2 H, t, J=6.6 Hz), 4.04 (3 H, s), 4.28 (1 H, t, J=7.9 Hz), 7.01 - 7.12 (2 H, m), 7.30 - 7.39 (2 H, m), 7.41 - 7.74 (3 H, m), 7.82 (1 H, d, J=7.9 Hz).

Reference Example 14

2-{4-chloro-1-[3-(trifluoromethyl)phenyl]butyl}-5-[3-methoxy-4-(2-methyl-1,3-oxazol-5-yl)phenyl]-1,3,4-oxadiazole

14a) 5-chloro-2-[3-(trifluoromethyl)phenyl]pentanoic acid

To a solution of [3-(trifluoromethyl)phenyl]acetic acid (1.47 g, 7.20 mmol) in THF (29 mL) was added dropwise n-butyllithium 1.6 M hexane solution (9.00 mL, 14.4 mmol) at - 78˚C, and the mixture was stirred under ice-cooling for 2 hr. 1-Bromo-3-chloropropane (7.56 mmol, 0.744 mL) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (ethyl acetate/hexane=20/80 - 50/50) to give the title compound as a colorless oil (0.538 g, 27%).
$^1$H NMR ($CDCl_3$) δ: 1.60 - 1.90 (2 H, m), 1.91 - 2.04 (1 H, m), 2.18 - 2.33 (1 H, m), 3.53 (2 H, t, J=6.4 Hz), 3.65 (1 H, t, J=7.7 Hz), 7.39 - 7.65 (4 H, m).

14b) N'-{5-chloro-2-[3-(trifluoromethyl)phenyl]pentanoyl}-3-methoxy-4-(2-methyl-1,3-oxazol-5-yl)benzohydrazide

To a mixture of 3-methoxy-4-(2-methyl-1,3-oxazol-5-yl)benzohydrazide hydrochloride (150 mg, 0.529 mmol), 5-chloro-2-[3-(trifluoromethyl)phenyl]pentanoic acid (178 mg, 0.634 mmol) and triethylamine (0.0737 mL, 0.529 mmol) in DMF (2.6 mL) was added diethyl cyanophosphate (0.102 mL, 0.688 mmol), and the mixture was stirred at room temperature for 1 hr. Triethylamine (0.221 mL, 1.59 mmol) was added, and the mixture was stirred at room temperature for 16 hr. Water was added to the reaction mixture, and the mixture was extracted with ethyl acetate. The obtained extract was washed with water and saturated brine, and dried over anhydrous magnesium sulfate. The solvent was evaporated under reduced pressure to give the title compound as white crystals (198 mg, 73%).
$^1$H NMR ($CDCl_3$) δ: 1. 63 - 1.94 (2 H, m), 1. 95 - 2.14 (1 H, m), 2.22 - 2.43 (1 H, m), 2.54 (3 H, s), 3.47 - 3.67 (3 H, m), 3.96 (3 H, s), 7.31 - 7.67 (7 H, m), 7.75 (1 H, d, J=8.3 Hz), 8.81 (1 H, brs), 9.04 (1 H, brs).

14c) 2-{4-chloro-1-[3-(trifluoromethyl)phenyl]butyl}-5-[3-methoxy-4-(2-methyl-1,3-oxazol-5-yl)phenyl]-1,3,4-oxadiazole

A mixture of N'-{5-chloro-2-[3-(trifluoromethyl)phenyl]pentanoyl}-3-methoxy-4-(2-methyl-1,3-oxazol-5-yl)benzohydrazide (198 mg, 0.388 mmol), carbon tetrachloride (0.0744 mL, 0.776 mmol) and triphenylphosphine (407 mg, 1.55 mmol) in acetonitrile (3.9 mL) was stirred at 80°C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The residue was purified by silica gel column chromatography (ethyl acetate/hexane=10/90 - 50/50) to give the title compound as a pale yellow oil (138 mg, 81%).
$^1$H NMR ($CDCl_3$) δ: 1.74 - 1.97 (2 H, m), 2.20 - 2.36 (1 H, m), 2.40 - 2.59 (4 H, m), 3.58 (2 H, t, J=6.6 Hz), 4.04 (3 H, s), 4.28 (1 H, t, J=7.9 Hz), 7.01 - 7.12 (2 H, m), 7.31 - 7.40 (2 H, m), 7.41 - 7.73 (3 H, m), 7.82 (1 H, d, J=7.9 Hz).

Reference Example 15

2-{4-chloro-1-[4-(trifluoromethyl)phenyl]butyl}-5-[3-methoxy-4-(2-methyl-1,3-oxazol-5-yl)phenyl]-1,3,4-oxadiazole

15a) 5-chloro-2-[4-(trifluoromethyl)phenyl]pentanoic acid

To a solution of [4-(trifluoromethyl)phenyl]acetic acid (1.47 g, 7.20 mmol) in THF (29 mL)-DMPU(10 mL) was added dropwise n-butyllithium 1.6 M hexane solution (9.00 mL, 14.4 mmol) at -78°C, and the mixture was stirred under ice-cooling for 2 hr. 1-Bromo-3-chloropropane (7.56 mmol, 0.744 mL) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (ethyl acetate/hexane=20/80 - 50/50) to give the title compound as white crystals (0.556 g, 24%).
$^1$H NMR ($CDCl_3$) δ: 1.60 - 1.89 (2 H, m), 1.92 - 2.04 (1 H, m), 2.17 - 2.32 (1 H, m), 3.53 (2 H, t, J=6.4 Hz), 3.66 (1 H, t, J=7.7 Hz), 7.44 (2 H, d, J=8.3 Hz), 7. 60 (2 H, d, J=7.9 Hz).

15b) N'-{5-chloro-2-[4-(trifluoromethyl)phenyl]pentanoyl}-3-methoxy-4-(2-methyl-1,3-oxazol-5-yl)benzohydrazide

To a mixture of 3-methoxy-4-(2-methyl-1,3-oxazol-5-yl)benzohydrazide hydrochloride (200 mg, 0.705 mmol), 5-chloro-2-[4-(trifluoromethyl)phenyl]pentanoic acid (211 mg, 0.750 mmol) and triethylamine (0.0870 mL, 0.625 mmol) in DMF (3.1 mL) was added diethyl cyanophosphate (0.121 mL, 0.812 mmol), and the mixture was stirred at room temperature for 1 hr. Triethylamine (0.348 mL, 2.50 mmol) was added, and the mixture was stirred at room temperature for 16 hr. Water was added to the reaction mixture, and the mixture was extracted with ethyl acetate. The obtained extract was washed with water and saturated brine, and dried over anhydrous magnesium sulfate. The solvent was evaporated under reduced pressure to give the title compound as pale yellow crystals (223 mg, 62%). $^1$H NMR ($CDCl_3$) δ: 1. 65 - 1.94 (2 H, m), 1.94 - 2.15 (1 H, m), 2.22 - 2.40 (1 H, m), 2.49 - 2.58 (3 H, m), 3.45 - 3.68 (3 H, m), 3.96 (3 H, s), 7.27 - 7.68 (7 H, m), 7.72 - 7.80 (1 H, m), 8.77 (1 H, brs), 9.02 (1 H, brs).

15c) 2-{4-chloro-1-[4-(trifluoromethyl)phenyl]butyl}-5-[3-methoxy-4-(2-methyl-1,3-oxazol-5-yl)phenyl]-1,3,4-oxadiazole

A mixture of N'-{5-chloro-2-[4-(trifluoromethyl)phenyl]pentanoyl}-3-methoxy-4-(2-methyl-1,3-oxazol-5-yl)benzohydrazide (223 mg, 0.437 mmol), carbon tetrachloride (0.0841 mL, 0.875 mmol) and triphenylphosphine (459 mg, 1.75 mmol) in acetonitrile (4.3 mL) was stirred at 80°C for 2 hr. The solvent was evaporated under reduced pressure,

saturated aqueous sodium hydrogen carbonate solution was added to the residue, and the mixture was extracted with ethyl acetate. The obtained extract was washed with water and saturated brine, and dried over anhydrous magnesium sulfate. The residue was purified by silica gel column chromatography (ethyl acetate/hexane=10/90 - 50/50) to give the title compound as a pale yellow oil (110 mg, 50%).
$^1$H NMR ($CDCl_3$) δ: 1.75 - 1.98 (2 H, m), 2.24 - 2.40 (1 H, m), 2.46 - 2.61 (4 H, m), 3.59 (2 H, t, J=6.6 Hz), 4.02 - 4.07 (3 H, m), 4.37 (1 H, t, J=7.9 Hz), 7.44 - 7.71 (7 H, m), 7.82 (1 H, d, J=8.3 Hz).

Reference Example 16

2-[4-chloro-1-(2,3-dichlorophenyl)butyl]-5-[3-methoxy-4-(2-methyl-1,3-oxazol-5-yl)phenyl]-1,3,4-oxadiazole

16a) 5-chloro-2-(2,3-dichlorophenyl)pentanoic acid

To a solution of diisopropylamine (0.71 mL, 5.0 mmol) in THF (5 mL) was added dropwise n-butyllithium 1.6 M hexane solution (3.1 mL, 5.0 mol) at -30˚C, and the mixture was stirred at the same temperature for 30 min. Thereto was added dropwise a solution of (2,3-dichlorophenyl)acetic acid (0.47 g, 2.3 mmol) in THF (5 mL) at -70˚C, and the mixture was stirred at the same temperature for 30 min. 1-Bromo-3-chloropropane (2.3 mL, 22.7 mmol) was further added at the same temperature, and the mixture was stirred at the same temperature for 30 min and then at room temperature for 2 hr. The reaction mixture was added to ice-cooled water, and the mixture was washed with ethyl acetate. The aqueous layer was adjusted to pH 3 - 4 with 6 M hydrochloric acid, and the mixture was extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=100/0 - 50/50) to give the title compound as a colorless oil (0.61 g, 95%).
$^1$H NMR ($CDCl_3$) δ: 1.69 - 2.04 (3 H, m), 2.20 - 2.32 (1 H, m), 3.55 (2 H, t, J=6.0 Hz), 4.29 (1 H, t, J=7.5 Hz), 7.23 (1 H, dd, J=8.1 Hz, 7.8 Hz), 7.31 (1 H, dd, J=1.8 Hz, 7.8 Hz), 7.43 (1H, dd, J=1.8 Hz, 7.8 Hz).

16b) 2-[4-chloro-1-(2,3-dichlorophenyl)butyl]-5-[3-methoxy-4-(2-methyl-1,3-oxazol-5-yl)phenyl]-1,3,4-oxadiazole

To a solution of 3-methoxy-4-(2-methyl-1,3-oxazol-5-yl)benzohydrazide hydrochloride (0.25 g, 0.88 mmol) and 5-chloro-2-(2,3-dichlorophenyl)pentanoic acid (0.22 g, 0.78 mmol) in DMF (2 mL) was added triethylamine (0.44 mL, 3.1 mmol), and the mixture was stirred at room temperature for 5 min. HATU (0.36 g, 0.94 mmol) was further added, and the mixture was stirred at room temperature for 2 hr. To the reaction mixture was added saturated brine, and the mixture was extracted with ethyl acetate. The obtained extract was washed with saturated brine, and dried over anhydrous magnesium sulfate. The solvent was evaporated under reduced pressure, carbon tetrachloride (0.15 μL, 1.6 mmol), triphenylphosphine (0.82 g, 3.1 mmol) and acetonitrile (8.0 mL) were added, and the mixture was heated under reflux for 2 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous magnesium sulfate. The residue was purified by silica gel column chromatography (hexane/ethyl acetate=100/0 - 50/50) to give the title compound as a colorless oil (89 mg, 23%).
$^1$H NMR ($CDCl_3$) δ: 1.49 - 1.56 (2 H, m), 2.26 - 2.37 (1 H, m), 2.46 - 2.57 (4 H, m), 3.62 (2 H, t, J=6.6 Hz), 4.06 (3 H, s), 4.96 (1 H, t, J=7.8 Hz), 7.20 - 7.34 (2 H, m), 7.45 (1 H, dd, J=1.8 Hz, 7.5 Hz), 7.53 (1 H, s), 7.62 - 7.64 (2 H, m), 7.83 (1 H, d, J=8.4 Hz). MS(ESI):494 $[M+H]^+$.

Reference Example 17

2-[5-chloro-1-(3,4-dichlorophenyl)pentyl]-5-[3-methoxy-4-(2-methyl-1,3-oxazol-5-yl)phenyl]-1,3,4-oxadiazole

17a) 6-chloro-2-(3,4-dichlorophenyl)hexanoic acid

To a solution of diisopropylamine (0.71 mL, 5.0 mmol) in THF (5 mL) was added dropwise n-butyllithium 1.6 M hexane solution (3.1 mL, 5.0 mmol) at -30˚C, and the mixture was stirred at the same temperature for 30 min. Thereto was added dropwise a solution of (3,4-dichlorophenyl)acetic acid (0.47 g, 2.3 mmol) in THF (5 mL) at -70˚C, and the mixture was stirred at the same temperature for 30 min. Further, at the same temperature, 1-bromo-4-chlorobutane (2.6 mL, 22.7 mmol) was added, and the mixture was stirred at the same temperature for 30 min and then at room temperature for 2 hr. The reaction mixture was added to ice-cooled water, and the mixture was washed with ethyl acetate. The aqueous layer was adjusted to pH 3 - 4 with 6 M hydrochloric acid, and the mixture was extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=100/0 - 50/50) to give the title compound as a colorless oil (0.48 g, 66%).
$^1$H NMR ($CDCl_3$) δ: 1.37 - 1.51 (2 H, m), 1.74 - 1.86 (2 H, m), 2.04 - 2.16 (1 H, m), 3.52 (2 H, t, J=6.6 Hz), 3.54 (1 H, t, J=6.9 Hz), 7.17 (1 H, dd, J=2.1 Hz, 8.4 Hz), 7.41 - 7.43 (2 H, m) .

17b) 2-[5-chloro-1-(3,4-dichlorophenyl)pentyl]-5-[3-methoxy-4-(2-methyl-1,3-oxazol-5-yl)phenyl]-1,3,4-oxadiazole

To a solution of 3-methoxy-4-(2-methyl-1,3-oxazol-5-yl)benzohydrazide hydrochloride (0.25 g, 0.88 mmol) and 6-chloro-2-(3,4-dichlorophenyl)hexanoic acid (0.25 g, 0.78 mmol) in DMF (2 mL) was added triethylamine (0.44 mL, 3.1 mmol), and the mixture was stirred at room temperature for 5 min. Further, HATU (0.36 g, 0.94 mmol) was added, and the mixture was stirred at room temperature for 2 hr. To the reaction mixture was added saturated brine was added, and the mixture was extracted with ethyl acetate. The obtained extract was washed with saturated brine, and dried over anhydrous magnesium sulfate. The solvent was evaporated under reduced pressure, carbon tetrachloride (0.15 μL, 1.6 mmol), triphenylphosphine (0.82 g, 3.1 mmol) and acetonitrile (8.0 mL) were added, and the mixture was heated under reflux for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. The residue was purified by silica gel column chromatography (hexane/ethyl acetate=100/0 - 50/50) to give the title compound as a colorless oil (0.13 g, 33%).
$^1$H NMR ($CDCl_3$) δ: 1.50 - 1.63 (2 H, m), 1.82 - 1.91 (1 H, m), 2.06 - 2.19 (1 H, m), 2.57 (3 H, s), 3.55 (2 H, t, J=6.6 Hz), 4.06 (3 H, s), 4.23 (1 H, t, J=7.5 Hz), 7.21 - 7.34 (2 H, m), 7.44 - 7.54 (2 H, m), 7.63 - 7.72 (2 H, m), 7.84 (1 H, d, J=8.1 Hz). MS(ESI):508 $[M+H]^+$.

Reference Example 18

2-[1-(benzyloxy)-4-chlorobutyl]-5-[3-methoxy-4-(2-methyl-1,3-oxazol-5-yl)phenyl]-1,3,4-oxadiazole

18a) 2-(benzyloxy)-5-chloropentanoic acid

To a solution of diisopropylamine (1.1 mL, 8.0 mmol) in THF (10 mL) was added dropwise n-butyllithium 1.6 M hexane solution (5.0 mL, 8.0 mmol) at -30˚C, and the mixture was stirred at the same temperature for 30 min. Thereto was added dropwise a solution of (benzyloxy)acetic acid (0.60 g, 3.6 mmol) in THF (5 mL) at -70˚C, and the mixture was stirred at the same temperature for 30 min. Further, at the same temperature, 1-bromo-3-chloropropane (3.6 mL, 36 mmol) was added, and the mixture was stirred at the same temperature for 30 min and then at room temperature for 2 hr. The reaction mixture was added to ice-cooled water, and the mixture was washed with ethyl acetate. The aqueous layer was adjusted to pH 3 - 4 with 6 M hydrochloric acid, and the mixture was extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=100/0 - 50/50) to give the title compound as a colorless oil (0.28 g, 32%).
$^1$H NMR ($CDCl_3$) δ: 1. 91 - 2.06 (4 H, m), 3.54 (2 H, t, J=5.1 Hz), 4.08 (1 H, t, J=6.6 Hz), 4.55 and 4.74 (2 H, d, J=11.4 Hz), 7.34 - 7.39 (5 H, m).

18b) N'-[2-(benzyloxy)-5-chloropentanoyl]-3-methoxy-4-(2-methyl-1,3-oxazol-5-yl)benzohydrazide

To a mixture of 3-methoxy-4-(2-methyl-1,3-oxazol-5-yl)benzohydrazide hydrochloride (323 mg, 1.14 mmol) and 2-(benzyloxy)-5-chloropentanoic acid (294 mg, 1.21 mmol) in DMF (5.0 mL) was added triethylamine (563 μL, 4.04 mmol), and the mixture was stirred at room temperature for 15 min. HATU (460 mg, 1.21 mmol) was added, and the mixture was stirred at room temperature for 2 hr. The reaction solution was diluted with ethyl acetate, and washed with water and saturated brine. This was dried over anhydrous sodium sulfate and the solvent was evaporated under reduced pressure. The obtained residue was purified by silica gel column chromatography (hexane/ethyl acetate=0/100 - 80/20) to give the title compound as a colorless oil (374 mg, 69%).
$^1$H NMR ($CDCl_3$) 5: 1.88 - 2. 06 (4 H, m), 2. 55 (3 H, s), 3.43 - 3.60 (2 H, m), 3.96 (3 H, s), 4.06 - 4.17 (1 H, m), 4.63 (1 H, d, J=11.3 Hz), 4.76 (1 H, d, J=11.3 Hz), 7.29 - 7.48 (7 H, m), 7.51 (1 H, s), 7.75 (1 H, d, J=8.2 Hz), 9.05 (2 H, br). MS (ESI) : 472 $[M+H]^+$.

18c) 2-[1-(benzyloxy)-4-chlorobutyl]-5-[3-methoxy-4-(2-methyl-1,3-oxazol-5-yl)phenyl]-1,3,4-oxadiazole

A mixture of N'-[2-(benzyloxy)-5-chloropentanoyl]-3-methoxy-4-(2-methyl-1,3-oxazol-5-yl)benzohydrazide (374 mg, 0.792 mmol), carbon tetrachloride (0.152 mL, 1.58 mmol) and triphenylphosphine (831 mg, 3.17 mmol) in acetonitrile (7.9 mL) was heated under reflux for 2 hr and the solvent was evaporated under reduced pressure. The

residue was diluted with ethyl acetate, washed with saturated aqueous sodium hydrogen carbonate solution and saturated brine dried over anhydrous sodium sulfate, and the solvent was evaporated. The obtained residue was purified by silica gel column chromatography (hexane/ethyl acetate=0/100 - 50/50) to give the title compound as a yellow oil (327 mg, 91%).
$^1$H NMR ($CDCl_3$) δ: 1.77 - 1.97 (1 H, m), 2.01 - 2.37 (3 H, m), 2.57 (3 H, s), 3.57 (2 H, t, J=6.2 Hz), 4.07 (3 H, s), 4.53 (1 H, d, J=11.8 Hz), 4.66 (1 H, d, J=11.8 Hz), 4.72 - 4.83 (1 H, m), 7.27 - 7.37 (5 H, m), 7.54 (1 H, s), 7.63 - 7.73 (2 H, m), 7.86 (1 H, d, J=8.2 Hz). MS(ESI):454 $[M+H]^+$.

Reference Example 19

2-[4-chloro-1-(2,4-difluorophenyl)butyl]-5-[3-methoxy-4-(2-methyl-1,3-oxazol-5-yl)phenyl]-1,3,4-oxadiazole

19a) 5-chloro-2-(2,4-difluorophenyl)pentanoic acid

To a solution of (2,4-difluorophenyl)acetic acid (2.50 g, 14.5 mmol) in THF (58 mL) was added dropwise n-butyllithium 1.6 M hexane solution (18.1 mL, 29.0 mmol) at -78°C, and the mixture was stirred under ice-cooling for 2 hr. 1-Bromo-3-chloropropane (1.50 mL, 15.2 mmol) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=100/0 - 50/50) to give the title compound as a colorless oil (1.46 g, 41%).
$^1$H NMR ($CDCl_3$) δ: 1.61 - 2.03 (3 H, m), 2.14 - 2.34 (1 H, m), 3.53 (2 H, t, J=6.4 Hz), 3.93 (1 H, t, J=7.6 Hz), 6.77 - 6.94 (2 H, m), 7.28 - 7.38 (1 H, m).

19b) 2-[4-chloro-1-(2,4-difluorophenyl)butyl]-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (150 mg, 0.529 mmol), 5-chloro-2-(2,4-difluorophenyl)pentanoic acid (158 mg, 0.635 mmol) and triethylamine (0.0737 mL, 0.529 mmol) in DMF (2.6 mL) was added diethyl cyanophosphate (0.102 mL, 0.688 mmol), and the mixture was stirred at room temperature for 1 hr. Triethylamine (0.221 mL, 1.59 mmol) was added, and the mixture was stirred at room temperature for 6 hr. Water was added to the reaction mixture, and the mixture was extracted with ethyl acetate. The obtained extract was washed with water and saturated brine, and dried over anhydrous magnesium sulfate. The solvent was evaporated under reduced pressure. A mixture of the obtained residue, carbon tetrachloride (0.0934 mL, 0.973 mmol) and triphenylphosphine (510 mg, 1.95 mmol) in acetonitrile (4.8 mL) was stirred at 80°C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. To the residue was added diethyl ether, the precipitate was filtered off, and the filtrate was evaporated under reduced pressure. The residue was purified by silica gel column chromatography (hexane/ethyl acetate=100/0 - 50/50) to give the title compound as a colorless amorphous solid (140 mg, 58%).
$^1$H NMR ($CDCl_3$) δ: 1.78 - 1.99 (2 H, m), 2.20 - 2.34 (1 H, m), 2.42 - 2.53 (1 H, m), 2.55 (3 H, s), 3.59 (2 H, t, J=6.4 Hz), 4.05 (3 H, s), 4.63 (1 H, t, J=7.8 Hz), 6.82 - 6.96 (2 H, m), 7.32 - 7.40 (1 H, m), 7.52 (1 H, s), 7.58 - 7.67 (2 H, m), 7.83 (1 H, d, J=8.0 Hz). MS (ESI) :460 $[M+H]^+$.

Reference Example 20

2-[4-chloro-1-(2,4-difluorophenyl)butyl]-5-[3-methoxy-4-(2-methyl-1,3-oxazol-5-yl)phenyl]-1,3,4-oxadiazole

20a) 5-chloro-2-(2-chloro-4-fluorophenyl)pentanoic acid

To a solution of (2-chloro-4-fluorophenyl)acetic acid (2.52 g, 13.4 mmol) in THF (54 mL) was added dropwise n-butyllithium 1.6 M hexane solution (16.7 mL, 26.1 mmol) at - 78°C, and the mixture was stirred under ice-cooling for 2 hr. 1-Bromo-3-chloropropane (1.38 mL, 14.1 mmol) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=90/10 - 50/50) to give the title compound as a yellow oil (1.72 g, 48%).
$^1$H NMR ($CDCl_3$) δ: 1.66 - 2.02 (3 H, m), 2.14 - 2.31 (1 H, m), 3.53 (2 H, t, J=6.4 Hz), 4.18 (1 H, t, J=7.4 Hz), 6.96 - 7.06

(1 H, m), 7.16 (1 H, dd, J=8.3, 2.7 Hz), 7.37 (1 H, dd, J=8.7, 6.1 Hz).

20b) 2-[4-chloro-1-(2-chloro-4-fluorophenyl)butyl]-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (150 mg, 0.529 mmol), 5-chloro-2-(4-chloro-2-fluorophenyl)pentanoic acid (168 mg, 0.635 mmol) and triethylamine (0.0737 mL, 0.529 mmol) in DMF (2.6 mL) was added diethyl cyanophosphate (0.102 mL, 0.688 mmol), and the mixture was stirred at room temperature for 1 hr. Triethylamine (0.221 mL, 1.59 mmol) was added, and the mixture was stirred at room temperature for 5 hr. Water was added to the reaction mixture, and the mixture was extracted with ethyl acetate. The obtained extract was washed with water and saturated brine, and dried over anhydrous magnesium sulfate. The solvent was evaporated under reduced pressure. A mixture of the obtained residue, carbon tetrachloride (0.0881 mL, 0.916 mmol) and triphenylphosphine (481 mg, 1.83 mmol) in acetonitrile (4.5 mL) was stirred at 80°C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. To the residue was added diethyl ether was added, the precipitate was filtered off, and the filtrate was evaporated under reduced pressure. The residue was purified by silica gel column chromatography (hexane/ethyl acetate=100/0 - 50/50) to give the title compound as a colorless amorphous solid (80 mg, 32%).
$^1$H NMR ($CDCl_3$) δ: 1.80 - 2.03 (2 H, m), 2.17 - 2.37 (1 H, m), 2.42 - 2.54 (1 H, m), 2.55 (3 H, s), 3.59 (2 H, t, J=6.6 Hz), 4.05 (3 H, s), 4.85 (1 H, t, J=7.6 Hz), 6.95 - 7.06 (1 H, m), 7.21 (1 H, dd, J=8.3, 2.7 Hz), 7.37 (1 H, dd, J=8.7, 5.7 Hz), 7.52 (1 H, s), 7.57 - 7.66 (2 H, m), 7.82 (1 H, d, J=8.0 Hz). MS(ESI) :476 $[M+H]^+$.

Reference Example 21

2-[4-chloro-1-(3,4-difluorophenyl)butyl]-5-[3-methoxy-4-(2-methyl-1,3-oxazol 5-yl)phenyl]-1,3,4-oxadiazole

21a) 5-chloro-2-(3,4-difluorophenyl)pentanoic acid

To a solution of (3,4-difluorophenyl)acetic acid (2.5 g, 15 mmol) in THF (60 mL) was added dropwise n-butyllithium 1.6 M hexane solution (18 mL, 29 mol) at -78°C, and the mixture was stirred under ice-cooling for 2 hr. 1-Bromo-3-chloropropane (1.5 mL, 15 mmol) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=20/80 - 50/50) to give the title compound as a colorless oil (2.6 g, 71%).
$^1$H NMR ($CDCl_3$) δ: 1.66 - 2.01 (3 H, m), 2.11 - 2.27 (1 H, m), 3.52 - 3.59 (3 H, m), 7.02 - 7.21 (3 H, m).

21b) 2-[4-chloro-1-(3,4-difluorophenyl)butyl]-5-[3-methoxy-4-(2-methyl-1,3-oxazol-5-yl)phenyl]-1,3,4-oxadiazole

To a solution of 3-methoxy-4-(2-methyl-1,3-oxazol-5-yl)benzohydrazide hydrochloride (0.15 g, 0.53 mmol), 5-chloro-2-(3,4-difluorophenyl)pentanoic acid (0.16 g, 0.63 mmol) and triethylamine (74 μL, 0.53 mmol) in DMF (2.6 mL) was added DEPC (0.10 mL, 0.69 mmol), and the mixture was stirred at room temperature for 1 hr. Further, triethylamine (0.22 mL, 1.6 mmol) was added, and the mixture was stirred at room temperature for 12 hr. Water was added to the reaction mixture, and the mixture was extracted with ethyl acetate. The obtained extract was washed with saturated brine, and dried over anhydrous magnesium sulfate. The solvent was evaporated under reduced pressure, carbon tetrachloride (0.15 mL, 1.6 mmol), triphenylphosphine (0.82 g, 3.1 mmol) and acetonitrile (8.0 mL) were added, and the mixture was heated under reflux for 2 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous magnesium sulfate. The residue was purified by silica gel column chromatography (hexane/ethyl acetate=100/0 - 50/50) to give the title compound as a colorless oil (89 mg, 23%).
$^1$H NMR ($CDCl_3$) δ: 1.49 - 1.56 (2 H, m), 2.26 - 2.37 (1 H, m), 2.46 - 2.57 (4 H, m), 3.62 (2 H, t, J=6.6 Hz), 4.06 (3 H, s), 4.96 (1 H, t, J=7.8 Hz), 7.20 - 7.34 (2 H, m), 7.45 (1 H, dd, J=1.8 Hz, 7.5 Hz), 7.53 (1 H, s), 7.62 - 7.64 (2 H, m), 7.83 (1 H, d, J=8.4 Hz) . MS(ESI) :460 $[M+H]^+$.

Reference Example 22

2-[4-chloro-1-(3,4-difluorophenyl)butyl]-5-[3-methoxy-4-(2-methyl-1,3-oxazol-5-yl)phenyl]-1,3,4-oxadiazole

22a) 5-chloro-2-[2-(trifluoromethoxy)phenyl]pentanoic acid

To a solution of [2-(trifluoromethoxy)phenyl]acetic acid (2.52 g, 11.4 mmol) in THF (50 mL) was added dropwise n-butyllithium 1.6 M hexane solution (14.3 mL, 22.9 mmol) at - 78˚C, and the mixture was stirred under ice-cooling for 2 hr. 1-Bromo-3-chloropropane (12.0 mmol, 1.18 mL) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=20/80 - 50/50) to give the title compound as a colorless oil (2.71 g, 87%).
$^1$H NMR ($CDCl_3$) δ: 1. 58 - 2.01 (3 H, m), 2. 15 - 2.32 (1 H, m), 3.52 (2 H, t, J=6.4 Hz), 4.03 (1 H, t, J=7.5 Hz), 7.23 - 7.37 (3 H, m), 7.41 - 7.48 (1 H, m).

22b) 2-{4-chloro-1-[2-(trifluoromethoxy)phenyl]butyl}-5-[3-methoxy-4-(2-methyl-1,3-oxazol-5-yl)phenyl]-1,3,4-oxadiazole

To a mixture of 3-methoxy-4-(2-methyl-1,3-oxazol-5-yl)benzohydrazide hydrochloride (150 mg, 0.529 mmol), 5-chloro-2-[2-(trifluoromethoxy)phenyl]pentanoic acid (188 mg, 0.635 mmol) and triethylamine (0.0737 mL, 0.529 mmol) in DMF (2.6 mL) was added diethyl cyanophosphate (0.102 mL, 0.688 mmol), and the mixture was stirred at room temperature for 1 hr. Triethylamine (0.221 mL, 1.59 mmol) was added, and the mixture was stirred at room temperature for 16 hr. Water was added to the reaction mixture, and the mixture was extracted with ethyl acetate. The obtained extract was washed with water and saturated brine, and dried over anhydrous magnesium sulfate. The solvent was evaporated under reduced pressure. A mixture of the obtained residue, carbon tetrachloride (0.0909 mL, 0.945 mmol) and triphenylphosphine (496 mg, 1.89 mmol) in acetonitrile (4.7 mL) was stirred at 80˚C for 2 hr. The solvent was evaporated under reduced pressure, saturated aqueous sodium hydrogen carbonate solution was added to the residue, and the mixture was extracted with ethyl acetate. The obtained extract was washed with water and saturated brine, and dried over anhydrous magnesium sulfate. To the residue was added diethyl ether, the precipitate was filtered off, and the filtrate was evaporated under reduced pressure. The residue was purified by silica gel column chromatography (hexane/ethyl acetate=100/0 - 50/50) to give the title compound as a colorless amorphous solid (146 mg, 54%).
$^1$H NMR ($CDCl_3$) δ: 1.75 - 2.01 (2 H, m), 2.18 - 2.38 (1 H, m), 2.45 - 2.64 (4 H, m), 3.58 (2 H, t, J=6.4 Hz), 4.04 (3 H, s), 4.73 (1 H, t, J=7.7 Hz), 7.28 - 7.40 (3 H, m), 7.42 - 7.49 (1 H, m), 7.52 (1 H, s), 7.57 - 7.63 (2 H, m), 7.82 (1 H, d, J=8.3 Hz). MS(ESI):508 $[M+H]^+$.

Reference Example 23

2-{4-chloro-1-[4-fluoro-3-(trifluoromethoxy)phenyl]butyl}-5-[3-methoxy-4-(2-methyl-1,3-oxazol-5-yl)phenyl]-1,3,4-oxadiazole

23a) 5-chloro-2-[4-fluoro-3-(trifluoromethoxy)phenyl]pentanoic acid

To a solution of [4-fluoro-3-(trifluoromethoxy)phenyl]acetic acid (526 mg, 2.21 mmol) in THF (8.8 mL) was added dropwise n-butyllithium 1.6 M hexane solution (2.76 mL, 4.42 mmol) at -78˚C, and the mixture was stirred under ice-cooling for 2 hr. 1-Bromo-3-chloropropane (2.32 mmol, 0.228 mL) was added, and the mixture was stirred at room temperature for 16 hr. The reaction mixture was extracted with 1 M aqueous sodium hydroxide solution. The extract was acidified with 3 M hydrochloric acid, and extracted with ethyl acetate. The organic layer was washed with saturated brine, dried over anhydrous magnesium sulfate, and the solvent was evaporated under reduced pressure. The obtained residue was purified by silica gel column chromatography (hexane/ethyl acetate=20/80 - 50/50) to give the title compound as a colorless oil (0.515 g, 74%).
$^1$H NMR ($CDCl_3$) 5: 1.58 - 2.03 (3 H, m), 2.14 - 2.29 (1 H, m), 3.47 - 3.62 (3 H, m), 7.07 - 7.41 (3 H, m).

23b) N'-{6-chloro-2-[4-fluoro-3-(trifluoromethoxy)phenyl]hexanoyl}-3-methoxy-4-(2-methyl-1,3-oxazol-5-yl)benzohydrazide

To a solution of 3-methoxy-4-(2-methyl-1,3-oxazol-5-yl)benzohydrazide hydrochloride (0.15 g, 0.53 mmol), 5-chloro-2-[4-fluoro-3-(trifluoromethoxy)phenyl]pentanoic acid (0.20 g, 0.63 mmol) and triethylamine (74 μL, 0.53 mmol) in DMF (2.6 mL) was added dropwise DEPC (0.10 mL, 0.69 mmol), and the mixture was stirred at room temperature for 1 hr. Further, triethylamine (0.22 mL, 1.6 mmol) was added, and the mixture was stirred at room temperature for 12 hr. Water was added to the reaction mixture, and the mixture was extracted with ethyl acetate. The obtained extract was washed with saturated brine, and dried over anhydrous magnesium sulfate. The solvent was evaporated under reduced pressure, and the residue was purified by silica gel column chromatography (hexane/ethyl acetate=80/20) to give the title compound as a white solid (128 mg, 44%).
$^1$H NMR ($CDCl_3$) δ: 1.68 - 1.90 (2 H, m), 1.96 - 2.08 (1 H, m), 2.24 - 2.36 (1 H, m), 2.56 (3 H, s), 3.52 - 3.59 (2 H, m), 4.00 (3 H, s), 7.19 (1 H, dd, J=8.7 Hz, 8.7 Hz), 7.31 - 7.40 (3 H, m), 7.43 (1 H, s), 7.53 (1 H, s), 7.78 (1 H, d, J=7.8 Hz), 8.74 (1 H, br), 8.94 (1 H, br). MS(ESI) : 544 $[M+H]^+$.

23c) 2-{4-chloro-1-[4-fluoro-3-(trifluoromethoxy)phenyl]butyl}-5-[3-methoxy-4-(2-methyl-1,3-oxazol-5-yl)phenyl]-1,3,4-oxadiazole

To a solution of N'-{6-chloro-2-[4-fluoro-3-(trifluoromethoxy)phenyl]hexanoyl}-3-methoxy-4-(2-methyl-1,3-oxazol-5-yl)benzohydrazide (0.13 g, 0.24 mmol) and triphenylphosphine (0.82 g, 3.1 mmol) in acetonitrile (8.0 mL) was added carbon tetrachloride (0.15 mL, 1.6 mmol), and the mixture was heated under reflux for 2 hr. The solvent was evaporated under reduced pressure, saturated aqueous sodium hydrogen carbonate solution was added to the residue, and the mixture was extracted with ethyl acetate. The obtained extract was washed with saturated brine, and dried over anhydrous magnesium sulfate. The residue was purified by silica gel column chromatography (hexane/ethyl acetate=100/0 - 50/50) to give the title compound as a colorless oil (68 mg, 55%).
$^1$H NMR ($CDCl_3$) δ: 1.82 - 1.93 (2 H, m), 2.24 - 2.36 (1 H, m), 2. 44 - 2.54 (1 H, m), 2.57 (3 H, s), 3.60 (2 H, t, J=6.6 Hz), 4.06 (3 H, s), 4.32 (1 H, t, J=7.8 Hz), 7.17 - 7.23 (1 H, m), 7.30 - 7.40 (2 H, m), 7.54 (1 H, s), 7.61 (2 H, d, J=8.1 Hz), 7.84 (1 H, d, J=7.8 Hz). MS(ESI):526 $[M+H]^+$.

Formulation Example 1

| | | |
|---|---|---|
| (1) | compound of Example 1 | 10.0 g |
| (2) | lactose | 70.0 g |
| (3) | cornstarch | 50.0 g |
| (4) | soluble starch | 7.0 g |
| (5) | magnesium stearate | 3.0 g |

The compound of Example 1 (10.0 g) and magnesium stearate (3.0 g) are granulated with aqueous solution of soluble starch (70 ml, 7.0 g as soluble starch), dried, mixed with lactose (70.0 g) and cornstarch (50.0 g) (lactose, cornstarch, soluble starch and magnesium stearate are products on the Japanese Pharmacopoeia 14th ed.). The mixture is compressed to give a tablet.

Experimental Example

(1) Measurement of amyloid β production inhibition rate using primary nerve cell

The primary nerve cells were collected from the cerebral cortex of rat fetus (CLEA Japan, Inc.: SD rat, fetal life 17 days of age), and suspended in a neurobasal medium containing B27 supplement, L-glutamine, penicillin-streptomycin (manufactured by Invitrogen) at 500,000 cells/mL. Then, the suspension was seeded in poly L-lysine-coated 96 well plate (manufactured by SUMITOMO BAKELITE) by 100 μL, and cultured at 37°C, 5% $CO_2$ for 7 days. The medium was completely removed, and a new neurobasal medium was added at 75 μL/well. Thereto was added (75 μL/well) a neurobasal medium supplemented with a 2-fold measurement concentration of an evaluation target compound, and the cells were cultured for 3 days. The culture supernatant was collected from each well, diluted as appropriate, applied to sandwich ELISA between BNT77 antibody-BA27 antibody (for Aβ40) and sandwich ELISA between BNT77 antibody-BC05 antibody (for Aβ42), and the amounts of Aβ40 and Aβ42 were measured.
The amyloid β production inhibition rate (%) of the compound was calculated by the following formula.

(1-(amyloid β production amount with addition of compound)/(amyloid β production amount without addition of compound)) × 100

In addition, the cell toxicity of the evaluation target compound was measured by a method using ATP amount as an index (method 1) and a method using hyperventilation activity as an index (method 2), based on which no dependency of the amyloid β production inhibitory activity on cytotoxicity was confirmed.
(Method 1) A new neurobasal medium was added (75 μL/well) to the cells after recovery of the culture supernatant, and the cells were left standing for about 30 min to reach room temperature. Cell-Titer Glo Luminescent Cell Viability Assay (manufactured by Promega) was added at 75 μL/well, and the plate was shaken for 2 min and reacted for about 10 min. Luminescence intensity was measured, and cytotoxicity was quantified using the amount of ATP as an index.
(Method 2) A new neurobasal medium containing 10% of Cell Counting Kit-8 (manufactured by Dojindo) was added to the cells after recovery of the culture supernatant at 100 μL/well and the mixture was stirred for about 2 hr. The absorbance at 450 nm was measured and cytotoxicity was quantified with hyperventilation activity as an index.
The test results are shown in Table 5.

[Table 5]

| Ex. No. | Aβ42 production inhibitory activity $IC_{50}$ (nM) |
|---|---|
| 1 | 90 |
| 2 | 34 |
| 8 | 88 |
| 12 | 150 |
| 15 | 170 |
| 25 | 88 |
| 28 | 200 |
| 34 | 42 |

[Industrial Applicability]

Since the compound of the present invention or a prodrug thereof shows a superior amyloid β production inhibitory activity, it can provide a clinically useful prophylactic or therapeutic drug for diseases such as mild cognitive impairment, Alzheimer's disease and the like. In addition, since the compound of the present invention or a prodrug thereof is superior in efficacy, low toxicity, stability, in vivo kinetics and the like, it is useful as a medicament.

This application is based on a patent application No. 2009-165134 filed in Japan, the contents of which are incorporated in full herein. In addition, the patent documents and non-patent documents cited in the present specification are hereby incorporated in their entireties by reference, to the extent that they have been disclosed in the present specification.

## Claims

**1.** A compound represented by the formula (I):

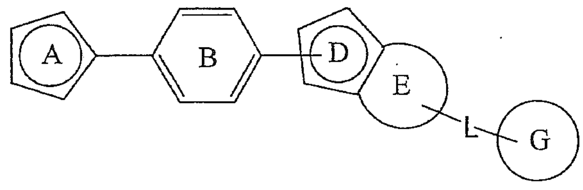

(I)

wherein

ring A is a imidazole ring, an oxazole ring or a triazole ring, each of which optionally has substituent(s),
ring B is a benzene ring, a pyridine ring or a pyrimidine ring, each of which optionally has substituent(s),
a group represented by a partial structural formula in the formula (I)

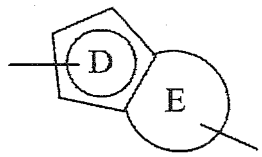

is a group represented by

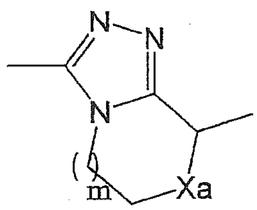

or

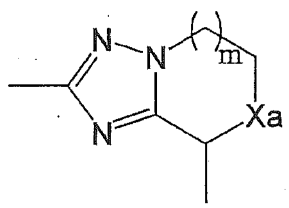

, each of which optionally has substituent(s),
wherein Xa is $-CH_2-$, -NH-, -O-, -S-, -SO- or $-SO_2-$ and m is 0, 1 or 2,
L is a bond, -O- or $-O-Y^1-$ wherein $Y^1$ is a $C_{1-6}$ alkylene group optionally having substituent(s), and
ring G is an aromatic hydrocarbon ring or an aromatic heterocycle, each of which optionally has substituent(s),

or a salt thereof.

**2.** The compound according to claim 1, wherein ring G is a benzene ring or a benzimidazole ring, each of which optionally has substituent(s), or a salt thereof.

**3.** The compound according to claim 1, wherein ring A is an imidazole ring, an oxazole ring or a triazole ring, each of which is optionally substituted by 1 to 3 $C_{1-6}$ alkyl groups, ring B is a benzene ring or a pyridine ring, each of which is optionally substituted by 1 to 3 substituents selected from a halogen atom, a cyano group and a $C_{1-6}$ alkoxy group,
Xa is $-CH_2-$,
m is 1 or 2,
L is a bond, -O- or $-O-y^1,-$ wherein $Y^{1'}$ is a $C_{1-6}$ alkylene group, and
ring G is a benzene ring or benzimidazole ring optionally substituted by 1 to 3 substituents selected from a halogen atom, a $C_{1-6}$ alkyl group optionally substituted by a halogen atom, a $C_{1-6}$ alkoxy group optionally substituted by a halogen atom, a $C_{2-6}$ alkenyl group, a $C_{1-6}$ alkylthio group, a $C_{1-6}$ alkylsulfonyl group and a morpholinyl group,
or a salt thereof.

**4.** The compound according to claim 3, wherein ring A is an imidazole ring, an oxazole ring or a triazole ring, each of which is substituted by 1 to 3 $C_{1-6}$ alkyl groups,
ring B is a benzene ring or a pyridine ring, each of which is substituted by 1 to 3 substituents selected from a halogen atom, a cyano group and a $C_{1-6}$ alkoxy group, and

ring G is a benzene ring or a benzimidazole ring, each of which is optionally substituted by 1 to 3 substituents selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group, or a salt thereof.

**5.** The compound according to claim 4, wherein ring B is (1) a benzene ring substituted by 1 to 3 substituents selected from a halogen atom, a cyano group and a $C_{1-6}$ alkoxy group, or (2) a pyridine ring substituted by 1 to 3 $C_{1-6}$ alkoxy groups, a group represented by a partial structural formula in the formula (I)

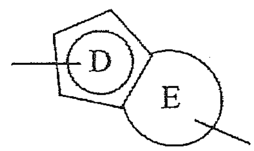

is a group represented by

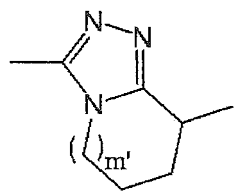

wherein m' is 1 or 2, or

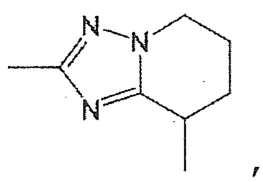

and
ring G is

(1)a benzene ring optionally substituted by 1 to 3 substituents selected from a halogen atom, a $C_{1-6}$ alkyl group substituted by a halogen atom, a $C_{1-6}$ alkoxy group substituted by a halogen atom, a $C_{2-6}$ alkenyl group, a $C_{1-6}$ alkylthio group, a $C_{1-6}$ alkylsulfonyl group and a morpholinyl group, or
(2) a benzimidazole ring substituted by 1 to 3 $C_{1-6}$ alkyl groups substituted by a halogen atom,

or a salt thereof.

**6.** The compound according to claim 1, wherein ring A is an oxazole ring substituted by 1 or 2 $C_{1-6}$ alkyl groups,
ring B is a benzene ring substituted by 1 to 3 $C_{1-6}$ alkoxy groups,
Xa is $-CH_2-$,
m is 1 or 2,
L is a bond or -O-, and
ring G is a benzene ring substituted by 1 to 3 substituents selected from a halogen atom and a $C_{1-6}$ alkyl group substituted by a halogen atom,
or a salt thereof.

**7.** The compound according to claim 6, wherein a group represented by a partial-structural formula in the formula (I)

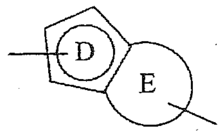

is a group represented by

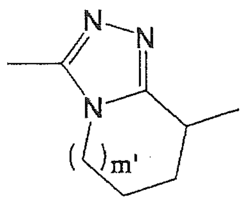

wherein m' is 1 or 2, or

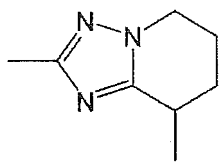

or a salt thereof.

8. The compound according to claim 1, wherein ring A is an oxazole ring substituted by 1 or 2 $C_{1-6}$ alkyl groups, ring B is a benzene ring substituted by 1 to 3 $C_{1-6}$ alkoxy groups, a group represented by a partial structural formula in the formula (I)

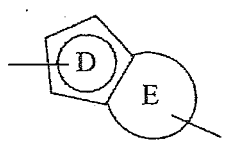

is a group represented by

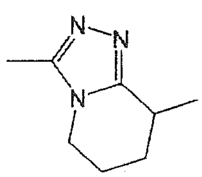

,

L is a bond, and
ring G is a benzene ring substituted by 1 to 3 substituents selected from a halogen atom and a $C_{1-6}$ alkyl group substituted by a halogen atom,

or a salt thereof.

9. 8-[4-fluoro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine or a salt thereof.

**10.** 8-[4-chloro-2-(trifluoromethyl)phenyl]-3-[3-methoxy-4-(2-methyl-1,3-oxazol-5-yl)phenyl]-5,6,7,8-tetrahydro[1,2,4]triazolo[4,3-a]pyridine or a salt thereof.

**11.** A prodrug of the compound according to claim 1 or a salt thereof.

**12.** A medicament comprising the compound according to claim 1 or a salt thereof or a prodrug thereof.

**13.** The medicament according to claim 12, which is a prophylactic or therapeutic drug for mild cognitive impairment or Alzheimer's disease.

**14.** A method of inhibiting amyloid β production, comprising administering an effective amount of the compound according to claim 1 or a salt thereof or a prodrug thereof to a mammal.

**15.** A method of preventing or treating mild cognitive impairment or Alzheimer's disease, comprising administering an effective amount of the compound according to claim 1 or a salt thereof or a prodrug thereof to a mammal.

**16.** Use of the compound according to claim 1 or a salt thereof or a prodrug thereof for the production of a drug for suppressing amyloid β production.

**17.** Use of the compound according to claim 1 or a salt thereof or a prodrug thereof for the production of a prophylactic or therapeutic drug for mild cognitive impairment or Alzheimer's disease.

**18.** The compound according to claim 1 or a salt thereof or a prodrug thereof for the suppression of amyloid β production.

**19.** The compound according to claim 1 or a salt thereof or a prodrug thereof for the prophylaxis or treatment of mild cognitive impairment or Alzheimer's disease.

**INTERNATIONAL SEARCH REPORT**

International application No. PCT/JP2010/061774

A. CLASSIFICATION OF SUBJECT MATTER
*C07D471/04*(2006.01)i, *A61K31/437*(2006.01)i, *A61K31/55*(2006.01)i, *A61P25/16*(2006.01)i, *A61P25/28*(2006.01)i, *A61P43/00*(2006.01)i, *C07D487/04* (2006.01)i

According to International Patent Classification (IPC) or to both national classification and IPC

B. FIELDS SEARCHED

Minimum documentation searched (classification system followed by classification symbols)
C07D471/04, A61K31/437, A61K31/55, A61P25/16, A61P25/28, A61P43/00, C07D487/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2009/073777 A1 (SCHERING CORP.), 11 June 2009 (11.06.2009), claim 1; page 70; page 97, compound B32; examples (Family: none) | 1-13,16-19 |
| Y | WO 2007/102580 A1 (Eisai R & D Management Co., Ltd.), 13 September 2007 (13.09.2007), claim 1; examples & US 2007/219181 A1 & EP 1992618 A1 | 1-13,16-19 |
| A | WO 2008/156580 A1 (MERCK & CO., INC.), 24 December 2008 (24.12.2008), entire text (Family: none) | 1-13,16-19 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
07 September, 2010 (07.09.10)

Date of mailing of the international search report
21 September, 2010 (21.09.10)

Name and mailing address of the ISA/
Japanese Patent Office

Authorized officer

Facsimile No.

Telephone No.

Form PCT/ISA/210 (second sheet) (July 2009)

**INTERNATIONAL SEARCH REPORT**

International application No.
PCT/JP2010/061774

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 14, 15
because they relate to subject matter not required to be searched by this Authority, namely:
Claims 14 and 15 involve methods for treatment of the human body or animal body by surgery or therapy and thus relate to a subject matter on which this International Searching Authority is not required to carry out a search under
(Continued to extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2010/061774

Continuation of Box No.II-1 of continuation of first sheet(2)

the provision of PCT Rule 67.1(iv) that PCT Rule 43bis.1(b) applies mutatis mutandis.

<Regarding Coverage of Search>

With respect to the "prodrug" in claims 11-13 and 16-19, it cannot be clearly specified what position of the compound is converted by what kind of binding group, and thus the description of the "prodrug" makes the inventions of claims 11-13 and 16-19 unclear.

This international search report therefore covers compounds represented by formula (I) set forth in claim 1 and salts of the compounds.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200160826 A
- WO 2004110350 A
- WO 2005115990 A
- WO 2007102580 A
- WO 2008097538 A
- WO 2009073777 A
- WO 2007081897 A
- WO 2007130824 A
- WO 2008078725 A
- WO 200877649 A
- JP 2009165134 A


### Non-patent literature cited in the description


- *Annual Reports in Medicinal Chemistry,* 2007, vol. 42, 27-47
- *Naunyn-Schmiedeberg's Arch. Pharmacol.,* 2008, vol. 377, 295-300
- *Neurotherapeutics,* 2008, vol. 5, 391-398
- *Expert Opinion on Therapeutic Patents,* 2008, vol. 18, 693-793
- Protective Groups in Organic Synthesis. Theodora W. Greene, Peter G. M. Wuts, Wiley-Interscience, 1999
- *Tetrahedron Letters,* 2003, vol. 44, 365
- *Tetrahedron,* 2002, vol. 58, 7663
- Shin Jikken Kagaku Koza. vol. 14, 15
- ORGANIC FUNCTIONAL GROUP PREPARATIONS. ACADEMIC PRESS, INC, 1989
- Comprehensive Organic Transformations. VCH Publishers Inc, 1989
- **Theodora W. Greene ; Peter G. M. Wuts.** Protective Groups in Organic Synthesis. Wiley-Interscience Inc, 1999
- *Bioorganic & Medicinal Chemistry Letters,* 2003, vol. 13, 2059
- ORGANIC FUNCTIONAL GROUP PREPARATIONS. ACADEMIC PRESS, INC.), 1989; Comprehensive Organic Transformations (VCH Publishers Inc, 1989
- *Indian Journal of Chemistry, Section B: Organic Chemistry Including Medicinal Chemistry,* 1982, vol. 21, 272
- *European journal of organic chemistry,* 2006, vol. 13, 2970
- *Synthetic communications,* 2006, vol. 36, 2927
- *Journal of organic chemistry,* 1979, vol. 44, 4160
- *Journal of the chemical society,* 1954, 4251
- Shin Jikken Kagaku Koza. vol. 14, 251-253
- Jikken Kagaku Kouza. vol. 19, 273-274
- Jikken Kagaku Koza. vol. 19, 273-274
- IYAKUHIN no KAIHATSU. HIROKAWA SHOTEN, vol. 7, 163-198